(19) Europäisches Patentamt
European Patent Office
Office européen des brevets

(11) EP 1 071 665 B1

(12) EUROPÄISCHE PATENTSCHRIFT

(45) Veröffentlichungstag und Bekanntmachung des Hinweises auf die Patenterteilung:
**01.10.2003 Patentblatt 2003/40**

(21) Anmeldenummer: **99920635.2**

(22) Anmeldetag: **10.04.1999**

(51) Int Cl.[7]: **C07D 209/34**, A61K 31/40, C07D 401/12, C07D 403/12, C07D 413/12

(86) Internationale Anmeldenummer:
**PCT/EP99/02436**

(87) Internationale Veröffentlichungsnummer:
**WO 99/052869 (21.10.1999 Gazette 1999/42)**

(54) **SUBSTITUIERTE INDOLINONE MIT INHIBIERENDE WIRKUNG AUF KINASEN UND CYCLIN/CDK-KOMPLEXE**

SUBSTITUTED INDOLINONES HAVING AN INHIBITING EFFECT ON KINASES AND CYCLINE/CDK COMPLEXES

INDOLINONES SUBSTITUEES A EFFET INHIBITEUR SUR DES KINASES ET DES COMPLEXES CYCLINE/CDK

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **15.04.1998 DE 19816624**

(43) Veröffentlichungstag der Anmeldung:
**31.01.2001 Patentblatt 2001/05**

(73) Patentinhaber: **Boehringer Ingelheim Pharma GmbH & Co.KG**
**55218 Ingelheim am Rhein (DE)**

(72) Erfinder:
- **HECKEL, Armin**
  **D-88400 Biberach (DE)**
- **WALTER, Rainer**
  **D-88400 Biberach (DE)**
- **GRELL, Wolfgang**
  **D-88400 Biberach (DE)**
- **VAN MEEL, Jacobus, C., A.**
  **D-88441 Mittelbiberach (DE)**
- **REDEMANN, Robert**
  **D-88400 Biberach (DE)**

(74) Vertreter: **Laudien, Dieter, Dr.**
**Boehringer Ingelheim GmbH,**
**B Patent**
**55216 Ingelheim am Rhein (DE)**

(56) Entgegenhaltungen:
**WO-A-96/22976** **WO-A-96/40116**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 071 665 B1

## Beschreibung

Die vorliegende Erfindung betrifft neue substituierte Indolinone der allgemeinen Formel

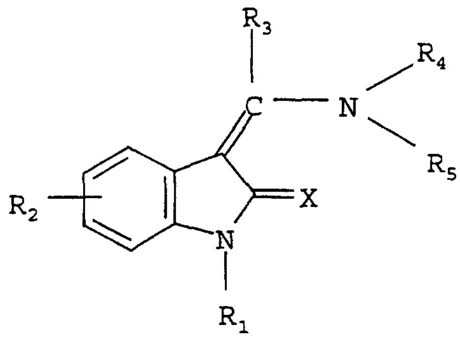

(I),

deren Isomere, deren Salze, insbesondere deren physiologisch verträgliche Salze, welche wertvolle Eigenschaften aufweisen.

Die Internationalen Anmeldungen WO 96/40116, WO 96/22976 und WO 99/15500 beschreiben Indolinon-Derivate mit inhibierender Wirkung auf Kinasen. Diese Dokumente stellen keine Verbindungen mit einer arylierten Methylen-Gruppe dar.

Die obigen Verbindungen der allgemeinen Formel I, in der $R_1$ ein Wasserstoffatom oder einen Prodrugrest darstellt, weisen wertvolle pharmakologische Eigenschaften auf, insbesondere eine inhibierende Wirkung auf verschiedene Kinasen, vor allem auf Komplexe von CDK's (CDK1, CDK2, CDK3, CDK4, CDK6, CDK7, CDK8 und CDK9) mit ihren spezifischen Cyclinen (A, B1, B2, C, D1, D2, D3, E, F, G1, G2, H, I und K) und auf virales Cyclin (siehe L. Mengtao in J. Virology 71(3), 1984-1991 (1997)), und die übrigen Verbindungen der obigen allgemeinen Formel I, in der $R_1$ kein Wasserstoffatom und keinen Prodrugrest darstellt, stellen wertvolle Zwischenprodukte zur Herstellung der vorstehend erwähnten Verbindungen dar.

Gegenstand der vorliegenden Erfindung sind somit die obigen Verbindungen der allgemeinen Formel I, wobei die Verbindungen, in denen $R_1$ ein Wasserstoffatom oder einen Prodrugrest darstellt, wertvolle pharmakologische Eigenschaften aufweisen, die die pharmakologisch wirksamen Verbindungen enthaltende Arzneimittel, deren Verwendung und Verfahren zu ihrer Herstellung.

In der obigen allgemeinen Formel I bedeuten

X ein Sauerstoff- oder Schwefelatom,

$R_1$ ein Wasserstoffatom, eine $C_{1-4}$-Alkoxy-carbonyl- oder $C_{2-4}$-Alkanoylgruppe,

$R_2$ eine Carboxy-, $C_{1-4}$-Alkoxy-carbonyl- oder Aminocarbonylgruppe, in der der Aminoteil durch eine oder zwei $C_{1-3}$-Alkylgruppen substituiert sein kann und die Substituenten gleich oder verschieden sein können,

$R_3$ eine Phenyl- oder Naphthylgruppe, die durch Fluor-, Chloroder Bromatome, durch $C_{1-3}$-Alkyl-, $C_{1-3}$-Alkoxy-, Cyano-, Trifluormethyl-, Nitro-, Amino-, $C_{1-3}$-Alkylamino-, Di- ($C_{1-3}$-alkyl)-amino-, $C_{2-4}$-Alkanoyl-amino-, N- ($C_{1-3}$-Alkyl)-$C_{2-4}$-alkanoylamino-, $C_{1-3}$-Alkylsulfonylamino-, Amino-$C_{1-3}$-alkyl-, $C_{1-3}$-Alkylamino-$C_{1-3}$-alkyl-, Di-($C_{1-3}$-Alkyl)-amino-$C_{1-3}$-alkyl-, N- ($C_{2-4}$-Alkanoyl)-amino-$C_{1-3}$-alkyl- oder N-($C_{2-4}$-Alkanoyl)-$C_{1-3}$-alkylamino-$C_{1-3}$-alkylgruppen substituiert und die Substituenten gleich oder verschieden sein können,

$R_4$ ein Wasserstoffatom oder eine $C_{1-3}$-Alkylgruppe und

$R_5$ ein Wasserstoffatom,

eine gegebenenfalls durch eine Phenyl-, Carboxy- oder $C_{1-3}$-Alkoxy-carbonylgruppe substituierte $C_{1-5}$-Alkylgruppe,

eine gegebenenfalls durch eine $C_{1-3}$-Alkylgruppe substitierte $C_{3-7}$-Cycloalkylgruppe,

eine gegebenenfalls durch eine $C_{1-3}$-Alkylgruppe substituierte Indanylgruppe,

eine 5-gliedrige Heteroarylgruppe, die eine gegebenenfalls durch eine $C_{1-3}$-Alkylgruppe substituierte Iminogruppe, ein Sauerstoff- oder Schwefelatom oder eine gegebenenfalls durch eine $C_{1-3}$-Alkylgruppe substituierte Iminogruppe und ein Sauerstoff-, Schwefel- oder Stickstoffatom oder zwei Stickstoffatome enthält oder eine 6-gliedrige Hereroarylgruppe, die 1 bis 3 Stickstoffatome enthält, wobei über zwei benachbarte Kohlenstoffatome oder über ein Kohlenstoffatom und eine benachbarte Iminogruppe der vorstehend erwähnten 5- und 6-gliedrigen Heteroarylgruppen zusätzlich eine 1,3-Butadienylenbrücke angefügt sein kann und das Kohlenstoffgerüst der vorstehend erwähnten mono- und bicyclischen Ringe durch Fluor-, Chlor-, Brom- oder Jodatome, durch $C_{1-5}$-Alkyl- oder Cyanogruppen mono- oder disubstituiert und die Substituenten gleich oder verschieden sein können,

eine über ein Kohlenstoffatom verknüpfte Pyrrolidinyl- oder Piperidinylgruppe, die jeweils am Stickstoffatom durch eine $C_{1-3}$-Alkylgruppe substituiert sein kann,

eine gegebenenfalls durch Fluor-, Chlor-, Brom- oder Jodatome, durch $C_{1-5}$-Alkyl-, $C_{1-3}$-Alkoxy-, Carboxy-, $C_{1-3}$-Alkoxycarbonyl-, Aminosulfonyl-, Nitro- oder Cyanogruppen disubstituierte Phenylgruppe, wobei die Substituenten gleich oder verschieden sein können,

eine Phenyl-, Pyridyl-, Pyrimidyl- oder Thienylgruppe, die jeweils

durch eine Trifluormethoxygruppe, durch ein ein Fluor-, Chlor-, Brom- oder Jodatom,

durch eine $C_{1-3}$-Alkoxygruppe, die in 2- oder 3-Stellung durch eine Amino-, $C_{1-3}$-Alkylamino-, Di- ($C_{1-3}$-Alkyl) -amino-, Phenyl-$C_{1-3}$-alkylamino-, N- ($C_{1-3}$-Alkyl) -phenyl-$C_{1-3}$-alkylamino-, Pyrrolidino- oder Piperidinogruppe substituiert sein kann,

durch eine Phenyl-$C_{1-3}$-alkylamino-$C_{1-3}$-alkylgruppe, die im Phenylkern durch eine Trifluormethylgruppe, durch Fluor-, Chlor-, Brom- oder Jodatome, durch $C_{1-5}$-Alkyl- oder $C_{1-3}$-Alkoxygruppen mono- oder disubstituiert sein kann, wobei die Substituenten gleich oder verschieden sein können, und zusätzlich am Aminstickstoffatom durch eine $C_{1-3}$-Alkylgruppe, in der die Wasserstoffatome ab Position 2 ganz oder teilweise durch Fluoratome ersetzt sein können,

durch eine $C_{1-5}$-Alkyl-, Phenyl-, Imidazolyl-, $C_{3-7}$-Cycloalkyl-, $C_{1-3}$-Alkoxy-$C_{1-3}$-alkoxy-, Phenyl-$C_{1-3}$-alkoxy-, Carboxy-$C_{1-3}$-alkyl-, $C_{1-3}$-Alkoxycarbonyl-$C_{1-3}$-alkyl-, Carboxy-, $C_{1-3}$-Alkoxycarbonyl-, Aminocarbonyl-, $C_{1-3}$-Alkylaminocarbonyl-, Di-($C_{1-3}$-Alkyl)-aminocarbonyl-, Phenyl-$C_{1-3}$-alkylaminocarbonyl-, N- ($C_{1-3}$-Alkyl)-phenyl-$C_{1-3}$-alkylaminocarbonyl-, Piperazinocarbonyl-, N-($C_{1-3}$-Alkyl)-piperazinocarbonyl-, Nitro-, Amino-, $C_{1-3}$-Alkylamino-, Di- ($C_{1-3}$-alkyl) -amino-, Pyrrolidino-, Piperidino-, Morpholino-, $C_{2-4}$-Alkanoylamino-, N-($C_{1-3}$-Alkyl)-$C_{2-4}$-alkanoylamino-, Benzoylamino- oder N-($C_{1-3}$-Alkyl)-benzoylaminogruppe,

durch eine N-($C_{1-3}$-Alkyl)-$C_{2-4}$-alkanoylaminogruppe, die im Alkylteil zusätzlich durch eine Carboxy- oder $C_{1-3}$-Alkoxycarbonylgruppe substituiert ist,

durch eine $C_{1-3}$-Alkylaminocarbonyl- oder Di-($C_{1-3}$-Alkyl)-aminocarbonylgruppe, in denen ein Alkylteil zusätzlich durch eine Di-($C_{1-3}$-Alkyl)-aminogruppe substituiert ist, oder

durch eine N-($C_{1-3}$-Alkyl)-$C_{1-3}$-alkylsulfonylamino- oder N-($C_{1-3}$-Alkyl)-phenylsulfonylaminogruppe, in denen der Alkylteil zusätzlich durch eine Cyano-, Carboxy-, $C_{1-3}$-Alkoxycarbonyl-, $C_{1-3}$-Alkylamino-, Di- ($C_{1-3}$-Alkyl) -amino-, Aminocarbonyl-, $C_{1-3}$-Alkylaminocarbonyl-, Di-($C_{1-3}$-Alkyl)-aminocarbonyl-, Piperidinocarbonyl- oder 2-[Di-($C_{1-3}$-Alkylamino)]-ethylaminocarbonylgruppe substituiert sein kann, substituiert sind,

eine durch eine $C_{1-3}$-Alkylgruppe substituierte Phenyl- oder Thienylgruppe, in der der Alkylteil durch eine Hydroxy-, $C_{1-3}$-Alkoxy-, Carboxy-, $C_{1-3}$-Alkoxy-carbonyl-, Amino-, $C_{1-5}$-Alkylamino-, Di- ($C_{1-5}$-Alkyl) -amino-, $C_{2-4}$-Alkanoylamino-, N-($C_{1-3}$-Alkyl)-$C_{2-4}$-alkanoylamino-, Pyrrolidino-, Dehydropyrrolidino-, Piperidino-, Dehydropiperidino-, 3-Hydroxypiperidino-, 4-Hydroxypiperidino-, Hexamethylenimino-, Morpholino-, Thiomorpholino-, Piperazino-, 4-($C_{1-3}$-Alkyl)-piperazino-, 4-Phenyl-piperazino-, 4-($C_{2-4}$-Alkanoyl)-piperazino-, 4-Benzoyl-piperazino- oder Imidazolylgruppe substituiert ist,

wobei die vorstehend erwähnten gesättigten Cycloalkyleniminoringe, $C_{1-5}$-Alkylamino- oder Di- ($C_{1-5}$-Alkyl)-aminogruppen zusätzlich durch eine oder zwei $C_{1-5}$-Alkylgruppen, durch eine $C_{3-7}$-Cycloalkyl-, Hydroxy-, $C_{1-3}$-Alkoxy-, Carboxy-, $C_{1-3}$-Alkoxycarbonyl-, Aminocarbonyl-, $C_{1-3}$-Alkylaminocarbonyl- oder Di-($C_{1-3}$-Alkyl)-aminocarbonylgruppe, durch eine im Phenylkern gegebenenfalls durch Fluor-, Chlor-, Brom- oder Jodatome, durch $C_{1-3}$-Alkyl- oder Cyanogruppen mono- oder disubstituierte Phenyl-$C_{1-3}$-alkyl- oder Phenylgruppe, wobei die Substituenten gleich oder verschieden sein können, substituiert sein können

oder eine zu dem Stickstoffatom benachbarte Methylengruppe in den vorstehend erwähnten Cycloalkyleniminoringe durch eine Carbonyl- oder Sulfonylgruppe ersetzt sein kann, und die vorstehend erwähnten monosubstituierten Phenylgruppen zusätzlich durch ein Fluor-, Chlor- oder Bromatom, eine Methyl-, Amino-, $C_{1-3}$-Alkylamino- oder Di-($C_{1-3}$-Alkyl)-aminogruppe substituiert sein können, oder

an einen der vorstehend erwähnten unsubstituierten Cycloalkyleniminoringe über zwei benachbarte Kohlenstoffatome ein gegebenenfalls durch eine oder zwei $C_{1-3}$-Alkoxygruppen substituierter Phenylring ankondensiert sein kann.

Die bei der Definition der vorstehend erwähnten Resten erwähnten Carboxygruppen können außerdem durch eine in-vivo in eine Carboxygruppe überführbare Gruppe ersetzt sein sowie

die bei der Definition der vorstehend erwähnten Resten erwähnten Amino- und Iminogruppen können außerdem durch einen in vivo abspaltbaren Rest substituiert sein.

Desweiteren schließen die bei der Definition der vorstehend erwähnten gesättigten Alkyl- und Alkoxyteile, die mehr als 2 Kohlenstoffatome enthalten, auch deren verzweigte Isomere wie beispielsweise die Isopropyl-, tert.Butyl-, Isobutylgruppe etc. ein.

Bevorzugte Verbindungen der allgemeinen Formel I sind diejenigen, in denen

X ein Sauerstoff- oder Schwefelatom,

$R_1$ ein Wasserstoffatom, eine $C_{1-4}$-Alkoxy-carbonyl- oder $C_{2-4}$-Alkanoylgruppe,

$R_2$ eine Carboxy-, $C_{1-4}$-Alkoxy-carbonyl- oder Aminocarbonylgruppe, in der der Aminoteil durch eine oder zwei $C_{1-3}$-Alkylgruppen substituiert sein kann und die Substituenten gleich oder verschieden sein können,

$R_3$ eine Phenyl- oder Naphthylgruppe, die durch Fluor-, Chloroder Bromatome, durch $C_{1-3}$-Alkyl-, $C_{1-3}$-Alkoxy-, Cyano-,

Trifluormethyl-, Nitro-, Amino-, $C_{1-3}$-Alkylamino-, Di-($C_{1-3}$-alkyl)-amino-, $C_{2-4}$-Alkanoyl-amino-, N-($C_{1-3}$-Alkyl)-$C_{2-4}$-alkanoylamino-, $C_{1-3}$-Alkylsulfonylamino-, Amino-$C_{1-3}$-alkyl-, $C_{1-3}$-Alkylamino-$C_{1-3}$-alkyl-, Di- ($C_{1-3}$-Alkyl)-amino-$C_{1-3}$-alkyl-, N- ($C_{2-4}$-Alkanoyl)-amino-$C_{1-3}$-alkyl- oder N- ($C_{2-4}$-Alkanoyl) -$C_{1-3}$-alkylamino-$C_{1-3}$-alkylgruppen substituiert und die Substituenten gleich oder verschieden sein können,

$R_4$ ein Wasserstoffatom oder eine $C_{1-3}$-Alkylgruppe und

$R_5$ ein Wasserstoffatom,

eine gegebenenfalls durch eine Phenyl-, Carboxy- oder $C_{1-3}$-Alkoxy-carbonylgruppe substituierte $C_{1-5}$-Alkylgruppe,

eine gegebenenfalls durch eine $C_{1-3}$-Alkylgruppe substitierte $C_{3-7}$-Cycloalkylgruppe,

eine gegebenenfalls durch eine $C_{1-3}$-Alkylgruppe substituierte Indanylgruppe,

eine 5-gliedrige Heteroarylgruppe, die eine gegebenenfalls durch eine $C_{1-3}$-Alkylgruppe substituierte Iminogruppe, ein Sauerstoff- oder Schwefelatom oder eine gegebenenfalls durch eine $C_{1-3}$-Alkylgruppe substituierte Iminogruppe und ein Sauerstoff-, Schwefel- oder Stickstoffatom oder zwei Stickstoffatome enthält oder eine 6-gliedrige Hereroarylgruppe, die 1 bis 3 Stickstoffatome enthält, wobei über zwei benachbarte Kohlenstoffatome oder über ein Kohlenstoffatom und eine benachbarte Iminogruppe der vorstehend erwähnten 5- und 6-gliedrigen Heteroarylgruppen zusätzlich eine 1,3-Butadienylenbrücke angefügt sein kann und das Kohlenstoffgerüst der vorstehend erwähnten mono- und bicyclischen Ringe durch Fluor-, Chlor-, Brom- oder Jodatome, durch $C_{1-5}$-Alkyl- oder Cyanogruppen mono- oder disubstituiert und die Substituenten gleich oder verschieden sein können,

eine über ein Kohlenstoffatom verknüpfte Pyrrolidinyl- oder Piperidinylgruppe, die jeweils am Stickstoffatom durch eine $C_{1-3}$-Alkylgruppe substituiert sein kann,

eine gegebenenfalls durch Fluor-, Chlor-, Brom- oder Jodatome, durch $C_{1-5}$-Alkyl- oder Cyanogruppen mono- oder disubstituiert Phenylgruppe, wobei die Substituenten gleich oder verschieden sein können,

eine Phenyl-, Pyridyl-, Pyrimidyl- oder Thienylgruppe, die jeweils durch eine $C_{3-7}$-Cycloalkyl-, $C_{1-3}$-Alkoxy-, Phenyl-$C_{1-3}$-alkoxy-, Carboxy-$C_{1-3}$-alkyl-, $C_{1-3}$-Alkoxy-carbonyl-$C_{1-3}$-alkyl-, Carboxy-, $C_{1-3}$-Alkoxycarbonyl-, Aminocarbonyl-, $C_{1-3}$-Alkylaminocarbonyl-, Di-($C_{1-3}$-Alkyl)-aminocarbonyl-, Nitro-, Amino-, $C_{1-3}$-Alkylamino-, Di-($C_{1-3}$-alkyl)-amino-, $C_{2-4}$-Alkanoyl-amino-, N- ($C_{1-3}$-Alkyl)-$C_{2-4}$-alkanoylamino- oder N- ($C_{1-3}$-Alkyl) -$C_{2-4}$-alkanoylaminogruppe, durch eine $C_{1-3}$-Alkylaminocarbonylgruppe, in der der Alkylteil zusätzlich durch eine Di-($C_{1-3}$-Alkyl)-aminogruppe substituiert ist, oder durch eine N-($C_{1-3}$-Alkyl)-$C_{1-3}$-alkylsulfonylaminogruppe, in der der Alkylteil zusätzlich durch eine Cyano-, Carboxy-, $C_{1-3}$-Alkoxycarbonyl-, $C_{1-3}$-Alkylaminooder Di-($C_{1-3}$-Alkyl)-aminogruppe substituiert sein kann, substituiert sind,

eine durch eine $C_{1-3}$-Alkylgruppe substituierte Phenyl- oder Thienylgruppe, in der der Alkylteil durch eine Hydroxy-, $C_{1-3}$-Alkoxy-, Carboxy-, $C_{1-3}$-Alkoxy-carbonyl-, Amino-, $C_{1-5}$-Alkylamino-, Di- ($C_{1-5}$-Alkyl)-amino-, $C_{2-4}$-Alkanoylamino-, N- ($C_{1-3}$-Alkyl)-$C_{2-4}$-alkanoylamino-, Pyrrolidino-, Piperidino-, Hexamethylenimino-, Morpholino-, Piperazino-, 4-($C_{1-3}$-Alkyl)-piperazino-, 4-($C_{2-4}$-Alkanoyl)-piperazino-, 4-Benzoyl-piperazino- oder Imidazolylgruppe substituiert ist, wobei die vorstehend erwähnten Cycloalkyleniminoringe, $C_{1-5}$-Alkylamino- oder Di-($C_{1-5}$-Alkyl)-aminogruppen zusätzlich durch eine $C_{1-5}$-Alkyl-, $C_{3-7}$-Cycloalkyl-, Hydroxy-, $C_{1-3}$-Alkoxy-, Carboxy-, $C_{1-3}$-Alkoxycarbonyl-, Aminocarbonyl-, $C_{1-3}$-Alkylaminocarbonyl- oder Di-($C_{1-3}$-Alkyl)-aminocarbonylgruppe, durch eine im Phenylkern gegebenenfalls durch Fluor-, Chlor-, Brom- oder Jodatome, durch $C_{1-3}$-Alkyl- oder Cyanogruppen mono- oder disubstituierte Phenyl-$C_{1-3}$-alkyl- oder Phenylgruppe, wobei die Substituenten gleich oder verschieden sein können, substituiert sein können oder eine zu dem Stickstoffatom benachbarte Methylengruppe in den vorstehend erwähnten Cycloalkyleniminoringe durch eine Carbonyl- oder Sulfonylgruppe ersetzt sein kann, und die vorstehend erwähnten monosustituierten Phenylgruppe zusätzlich durch ein Fluor-, Chlor- oder Bromatom oder durch eine Methylgruppe substituiert sein können, bedeuten,

insbsondere diejenigen Verbindungen der allgemeinen Formel I, in denen

X ein Sauerstoffatom,

$R_1$ ein Wasserstoffatom oder eine $C_{1-4}$-Alkoxy-carbonylgruppe,

$R_2$ eine Carboxy-, $C_{1-4}$-Alkoxy-carbonyl- oder Aminocarbonylgruppe, in der der Aminoteil durch eine oder zwei $C_{1-3}$-Alkylgruppen substituiert sein kann und die Substituenten gleich oder verschieden sein können,

$R_3$ eine gegebenenfalls durch ein Fluor-, Chlor- oder Bromatom, durch eine Methyl-, Cyano- oder Aminomethylgruppe substituierte Phenylgruppe,

$R_4$ ein Wasserstofatom oder eine Methylgruppe und

$R_5$ ein Wasserstofatom,

eine gegebenenfalls durch eine Carboxy- oder $C_{1-3}$-Alkoxy-carbonylgruppe substituierte $C_{1-5}$-Alkylgruppe oder eine Benzylgruppe,

eine gegebenenfalls durch eine Methylgruppe substitierte $C_{3-7}$-Cycloalkylgruppe,

eine gegebenenfalls durch eine Methylgruppe substituierte Indanyl-, Pyridyl-, Oxazolyl-, Thiazolyl- oder Imidazolylgruppe, an die jeweils zusätzlich über zwei benachbarte Kohlenstoffatome ein Phenylring ankondensiert sein kann,

eine gegebenenfalls durch ein Fluor-, Chlor- oder Bromatom, durch eine Methoxy-, Carboxy-, $C_{1-3}$-Alkyloxycarbonyl-, Nitrooder Aminosulfonylgruppe substituierte Methylphenylgruppe oder eine Dimethoxyphenylgrruppe,

eine über ein Kohlenstoffatom verknüpfte Pyrrolidinyl- oder Piperidinylgruppe, die jeweils am Stickstoffatom durch eine

$C_{1-3}$-Alkylgruppe substituiert sind,
eine Phenylgruppe, die

durch eine Trifluormethoxygruppe, durch ein ein Fluor-, Chlor-, Brom- oder Jodatom,

durch eine $C_{1-3}$-Alkoxygruppe, die in 2- oder 3-Stellung durch eine Amino-, $C_{1-3}$-Alkylamino-, Di- ($C_{1-3}$-Alkyl) -amino-, Phenyl-$C_{1-3}$-alkylamino-, N- ($C_{1-3}$-Alkyl)-phenyl-$C_{1-3}$-alkylamino-, Pyrrolidino- oder Piperidinogruppe substituiert sein kann,

durch eine Phenyl-$C_{1-3}$-alkylamino-$C_{1-3}$-alkylgruppe, die im Phenylkern durch ein Fluor-, Chlor-, Brom- oder Jodatom, durch eine $C_{1-5}$-Alkyl-, $C_{1-3}$-Alkoxy- oder Trifluormethylgruppe und zusätzlich am Aminstickstoffatom durch eine $C_{1-3}$-Alkylgruppe, in der die Wasserstoffatome ab Position 2 ganz oder teilweise durch Fluoratome ersetzt sein können,

durch eine $C_{1-5}$-Alkyl-, Phenyl-, Imidazolyl-, $C_{3-7}$-Cycloalkyl-, $C_{1-3}$-Alkoxy-$C_{1-3}$-alkoxy-, Phenyl-$C_{1-3}$-alkoxy-, Carboxy-$C_{1-3}$-alkyl-, $C_{1-3}$-Alkoxycarbonyl-$C_{1-3}$-alkyl-, Carboxy-, $C_{1-3}$-Alkoxycarbonyl-, Aminocarbonyl-, $C_{1-3}$-Alkylaminocarbonyl-, Di-($C_{1-3}$-Alkyl)-aminocarbonyl-, Phenyl-$C_{1-3}$-alkylaminocarbonyl-, N-($C_{1-3}$-Alkyl)-phenyl-$C_{1-3}$-alkylaminocarbonyl-, Piperazinocarbonyl-, N-($C_{1-3}$-Alkyl)-piperazinocarbonyl-, Nitro-, Amino-, $C_{1-3}$-Alkylamino-, Di- ($C_{1-3}$-alkyl)-amino-, Pyrrolidino-, Piperidino-, Morpholino-, $C_{2-4}$-Alkanoylamino-, N-($C_{1-3}$-Alkyl)-$C_{2-4}$-alkanoylamino-, Benzoylamino- oder N- ($C_{1-3}$-Alkyl) -benzoylaminogruppe,

durch eine N-($C_{1-3}$-Alkyl)-$C_{2-4}$-alkanoylaminogruppe, die im Alkylteil zusätzlich durch eine Carboxy- oder $C_{1-3}$-Alkoxycarbonylgruppe substituiert ist,

durch eine $C_{1-3}$-Alkylaminocarbonyl- oder Di-($C_{1-3}$-Alkyl)-aminocarbonylgruppe, in denen ein Alkylteil zusätzlich durch eine Di-($C_{1-3}$-Alkyl)-aminogruppe substituiert ist, oder

durch eine N- ($C_{1-3}$-Alkyl)-$C_{1-3}$-alkylsulfonylamino- oder N-($C_{1-3}$-Alkyl)-phenylsulfonylaminogruppe, in denen der Alkylteil zusätzlich durch eine Cyano-, Carboxy-, $C_{1-3}$-Alkoxycarbonyl-, $C_{1-3}$-Alkylamino-, Di- ($C_{1-3}$-Alkyl)-amino-, Aminocarbonyl-, $C_{1-3}$-Alkylaminocarbonyl-, Di-($C_{1-3}$-Alkyl)-aminocarbonyl-, Piperidinocarbonyl- oder 2-[Di-($C_{1-3}$-Alkylamino)]-ethylaminocarbonylgruppe substituiert sein kann, substituiert ist,

eine gegebenenfalls durch eine $C_{1-3}$-Alkylgruppe substituierte Phenylgruppe, in der der Alkylteil durch eine Hydroxy-, $C_{1-3}$-Alkoxy-, Carboxy-, $C_{1-3}$-Alkoxy-carbonyl-, Amino-, $C_{1-5}$-Alkylamino-, Di-($C_{1-5}$-Alkyl) -amino-, $C_{2-4}$-Alkanoylamino-, N-($C_{1-3}$-Alkyl)-$C_{2-4}$-alkanoylamino-, Pyrrolidino-, Dehydropyrrolidino-, Piperidino-, Dehydropiperidino-, 3-Hydroxypiperidino-, 4-Hydroxypiperidino-, Hexamethylenimino-, Morpholino-, Thiomorpholino-, Piperazino-, 4-($C_{1-3}$-Alkyl)-piperazino-, 4-Phenyl-piperazino-, 4-($C_{2-4}$-Alkanoyl)-piperazino-, 4-Benzoyl-piperazino- oder Imidazolylgruppe substituiert ist,

wobei die vorstehend erwähnten gesättigten Cycloalkyleniminoringe, $C_{1-5}$-Alkylamino- oder Di-($C_{1-5}$-Alkyl)-aminogruppen zusätzlich durch eine oder zwei $C_{1-5}$-Alkylgruppen, durch eine $C_{3-7}$-Cycloalkyl-, Hydroxy-, $C_{1-3}$-Alkoxy-, Carboxy-, $C_{1-3}$-Alkoxycarbonyl-, Aminocarbonyl-, $C_{1-3}$-Alkylaminocarbonyl- oder Di-($C_{1-3}$-Alkyl)-aminocarbonylgruppe, durch eine im Phenylkern gegebenenfalls durch Fluor-, Chlor-, Brom- oder Jodatome, durch $C_{1-3}$-Alkyl- oder Cyanogruppen mono- oder disubstituierte Phenyl-$C_{1-3}$-alkyl- oder Phenylgruppe, wobei die Substituenten gleich oder verschieden sein können, substituiert sein können

oder eine zu dem Stickstoffatom benachbarte Methylengruppe in den vorstehend erwähnten Cycloalkyleniminoringe durch eine Carbonyl- oder Sulfonylgruppe ersetzt sein kann, und die vorstehend erwähnten monosubstituierten Phenylgruppen zusätzlich durch ein Fluor-, Chlor- oder Bromatom, eine Methyl-, Amino-, $C_{1-3}$-Alkylamino- oder Di-($C_{1-3}$-Alkyl)-aminogruppe substituiert sein können, oder

an einen der vorstehend erwähnten unsubstituierten Cycloalkyleniminoringe über zwei benachbarte Kohlenstoffatome ein gegebenenfalls durch eine oder zwei $C_{1-3}$-Alkoxygruppen substituierter Phenylring ankondensiert sein kann,

bedeuten, deren Isomere und deren Salze.

Besonders bevorzugte Verbindungen der obigen allgemeinen Formel I sind diejenigen, in denen
X ein Sauerstoffatom,
$R_1$ ein Wasserstoffatom,
$R_2$ eine Carboxy-, $C_{1-4}$-Alkoxycarbonyl- oder Aminocarbonylgruppe, in der der Aminoteil durch eine oder zwei $C_{1-3}$-Alkylgruppen substituiert sein kann und die Substituenten gleich oder verschieden sein können,
$R_3$ eine gegebenenfalls durch eine Methylgruppe substituierte Phenylgruppe,
$R_4$ ein Wasserstoffatom oder eine Methylgruppe und
$R_5$ ein Wasserstoffatom,
eine $C_{1-3}$-Alkylgruppe, eine Benylgruppe oder eine durch eine Carboxy- oder $C_{1-3}$-Alkoxycarbonylgruppe substituierte Methyloder Ethylgruppe,
eine gegebenenfalls durch eine Methylgruppe substitierte $C_{3-7}$-Cycloalkylgruppe,
eine gegebenenfalls durch eine Methylgruppe substituierte Indanyl-, Pyridyl-, Oxazolyl-, Thiazolyl- oder Imidazolylgruppe, an die jeweils zusätzlich über zwei benachbarte Kohlenstoffatome ein Phenylring ankondensiert sein kann,
eine gegebenenfalls durch ein Fluor-, Chlor- oder Bromatom, durch eine Methoxy-, Carboxy-, $C_{1-3}$-Alkyloxycarbonyl-,

Nitrooder Aminosulfonylgruppe substituierte Methylphenylgruppe oder eine Dimethoxyphenylgrruppe,
eine 3-Pyrrolidinyl- oder 4-Piperidinylgruppe, die jeweils am Stickstoffatom durch eine $C_{1-3}$-Alkylgruppe substituiert sind,
eine Phenylgruppe, die

durch eine Trifluormethoxy-, Benzyloxy-, Cyano- oder Nitrogruppe, durch ein ein Fluor-, Chlor- oder Bromatom,

durch eine $C_{1-3}$-Alkoxygruppe, wobei die Ethoxy- und n-Propoxygruppe jeweils endständig durch eine Dimethylamino-, Diethylamino-, N-Ethyl-methylamino-, N-Benzyl-methylamino- oder Piperidinogruppe substituiert sein kann,

durch eine Phenyl-$C_{1-3}$-alkylamino-$C_{1-3}$-alkylgruppe, die im Phenylkern durch ein Fluor-, Chlor-, Brom- oder Jodatom, durch eine Methyl-, Methoxy- oder Trifluormethylgruppe und zusätzlich am Aminstickstoffatom durch eine $C_{1-5}$-Alkyl- oder 2,2,2-Trifluorethylgruppe substituiert sein kann,

durch eine $C_{1-4}$-Alkyl-, Phenyl-, Imidazolyl-, Cyclohexyl-, Methoxymethyl-, Carboxymethyl-, $C_{1-3}$-Alkoxycarbonylmethyl-, Carboxy-, $C_{1-3}$-Alkoxycarbonyl-, Aminocarbonyl-, $C_{1-3}$-Alkylaminocarbonyl-, Di-($C_{1-3}$-Alkyl)-aminocarbonyl-, Phenyl-$C_{1-3}$-alkylaminocarbonyl-, N-($C_{1-3}$-Alkyl)-phenyl-$C_{1-3}$-alkylaminocarbonyl-, Piperazinocarbonyl-, N-($C_{1-3}$-Alkyl)-piperazinocarbonyl-, Amino-, $C_{1-3}$-Alkylamino-, Di-($C_{1-3}$-alkyl)-amino-, Pyrrolidino-, Piperidino-, Morpholino-, $C_{2-4}$-Alkanoylamino-, N-($C_{1-3}$-Alkyl)-$C_{2-4}$-alkanoylamino-, Benzoylamino- oder N-($C_{1-3}$-Alkyl)-benzoylaminogruppe,

durch eine N-($C_{1-3}$-Alkyl)-$C_{2-4}$-alkanoylaminogruppe, die im Alkylteil zusätzlich durch eine Carboxy- oder $C_{1-3}$-Alkoxycarbonylgruppe substituiert ist, durch eine $C_{1-3}$-Alkylaminocarbonyl- oder Di- ($C_{1-3}$-Alkyl) -aminocarbonylgruppe, in denen ein Alkylteil zusätzlich durch eine Di-($C_{1-3}$-Alkyl)-aminogruppe substituiert ist, oder

durch eine N-($C_{1-3}$-Alkyl)-$C_{1-3}$-alkylsulfonylamino- oder N-($C_{1-3}$-Alkyl)-phenylsulfonylaminogruppe, in denen der Alkylteil zusätzlich durch eine Cyano-, Carboxy-, $C_{1-3}$-Alkoxycarbonyl-, $C_{1-3}$-Alkylamino-, Di- ($C_{1-3}$-Alkyl)-amino-, Aminocarbonyl-, $C_{1-3}$-Alkylaminocarbonyl-, Di- ($C_{1-3}$-Alkyl)-aminocarbonyl-, Piperidinocarbonyl- oder 2-[Di-($C_{1-3}$-Alkylamino)]-ethylaminocarbonylgruppe substituiert sein kann, substituiert ist,

eine gegebenenfalls durch eine $C_{1-3}$-Alkylgruppe substituierte Phenylgruppe, in der die Alkylgruppe durch eine Hydroxy-, $C_{1-3}$-Alkoxy-, Carboxy-, $C_{1-3}$-Alkoxycarbonyl-, Amino-, $C_{1-5}$-Alkylamino-, Di- ($C_{1-5}$-Alkyl)-amino-, $C_{2-4}$-Alkanoylamino-, N-($C_{1-3}$-Alkyl)-$C_{2-4}$-alkanoylamino-, Pyrrolidino-, Dehydropyrrolidino-, Piperidino-, Dehydropiperidino-, 4-Hydroxypiperidino-, Hexamethylenimino-, Morpholino-, Thiomorpholino-, Piperazino-, 4-($C_{1-3}$-Alkyl)-piperazino-, 4-Phenyl-piperazino-, 4-($C_{2-4}$-Alkanoyl)-piperazino-, 4-Benzoyl-piperazino- oder Imidazolylgruppe substituiert ist,

wobei die vorstehend erwähnten gesättigten Cycloalkyleniminoringe zusätzlich durch eine Phenylgruppe oder durch eine oder zwei Methylgruppen,

die vorstehend erwähnten $C_{1-5}$-Alkylamino- und Di-($C_{1-5}$-Alkyl)-aminogruppen zusätzlich durch eine oder zwei $C_{1-3}$-Alkylgruppen, durch eine Cyclohexyl-, Hydroxy-, $C_{1-3}$-Alkoxy-, Carboxy-, $C_{1-3}$-Alkoxycarbonyl-, Aminocarbonyl-, $C_{1-3}$-Alkylaminocarbonyloder Di-($C_{1-3}$-Alkyl)-aminocarbonylgruppe, durch eine im Phenylkern gegebenenfalls durch ein Fluor-, Chlor-, Brom- oder Jodatom, durch eine Methyl- oder Cyanogruppe substituierte Phenyl-$C_{1-3}$-alkyl- oder Phenylgruppe substituiert sein kann,

oder eine zu dem Stickstoffatom benachbarte Methylengruppe in den vorstehend erwähnten Cycloalkyleniminoringe durch eine Carbonyl- oder Sulfonylgruppe ersetzt sein kann, und die vorstehend erwähnten monosubstituierten Phenylgruppen zusätzlich durch ein Fluor-, Chlor- oder Bromatom, eine Methyl-, Amino-, $C_{1-3}$-Alkylamino- oder Di-($C_{1-3}$-Alkyl)-aminogruppe substituiert sein können, oder

an einen der vorstehend erwähnten unsubstituierten Cycloalkyleniminoringe über zwei benachbarte Kohlenstoffatome ein gegebenenfalls durch eine oder zwei $C_{1-3}$-Alkoxygruppen substituierter Phenylring ankondensiert sein kann,

bedeuten, deren Isomere und deren Salze.

Ganz besonders bevorzugte Verbindungen der obigen allgemeinen Formel I sind diejenigen, in denen
X ein Sauerstoffatom,
$R_1$ ein Wasserstoffatom,
$R_2$ eine Carboxy- oder Aminocarbonylgruppe, in der der Aminoteil durch eine oder zwei $C_{1-3}$-Alkylgruppen substituiert sein kann und die Substituenten gleich oder verschieden sein können,
$R_3$ eine gegebenenfalls durch eine Methylgruppe substituierte Phenylgruppe,
$R_4$ ein Wasserstoffatom und
$R_5$ ein Wasserstoffatom,
eine 3-Pyrrolidinyl- oder 4-Piperidinylgruppe, die jeweils am Stickstoffatom durch eine $C_{1-3}$-Alkylgruppe substituiert sind,
eine Phenylgruppe, die

durch eine $C_{1-3}$-Alkoxygruppe, wobei die Ethoxy- und n-Propoxygruppe jeweils endständig durch eine Dimethylamino-, Diethylamino-, N-Ethyl-methylamino-, N-Benzyl-methylamino- oder Piperidinogruppe substituiert sein kann,

durch eine Phenyl-$C_{1-3}$-alkylamino-$C_{1-3}$-alkylgruppe, die im Phenylkern durch ein Fluor-, Chlor-, Brom- oder Jodatom, durch eine Methyl-, Methoxy- oder Trifluormethylgruppe und zusätzlich am Aminstickstoffatom durch eine $C_{1-5}$-Alkyl- oder 2,2,2-Trifluorethylgruppe substituiert sein kann, substituiert ist,

eine gegebenenfalls durch eine $C_{1-3}$-Alkylgruppe substituierte Phenylgruppe, in der die Alkylgruppe durch eine Hydroxy-, $C_{1-3}$-Alkoxy-, Carboxy-, $C_{1-3}$-Alkoxycarbonyl-, Amino-, $C_{1-5}$-Alkylamino-, Di- ($C_{1-5}$-Alkyl)-amino-, $C_{2-4}$-Alkanoylamino-, N-($C_{1-3}$-Alkyl)-$C_{2-4}$-alkanoylamino-, Pyrrolidino-, Dehydropyrrolidino-, Piperidino-, Dehydropiperidino-, 4-Hydroxypiperidino-, Hexamethylenimino-, Morpholino-, Thiomorpholino-, Piperazino-, 4-($C_{1-3}$-Alkyl)-piperazino-, 4-Phenyl-piperazino-, 4-($C_{2-4}$-Alkanoyl)-piperazino-, 4-Benzoyl-piperazino- oder Imidazolylgruppe substituiert ist,

wobei die vorstehend erwähnten gesättigten Cycloalkyleniminoringe zusätzlich durch eine Phenylgruppe oder durch eine oder zwei Methylgruppen,

die vorstehend erwähnten $C_{1-5}$-Alkylamino- und Di-($C_{1-5}$-Alkyl)-aminogruppen zusätzlich durch eine oder zwei $C_{1-3}$-Alkylgruppen, durch eine Cyclohexyl-, Hydroxy-, $C_{1-3}$-Alkoxy-, Carboxy-, $C_{1-3}$-Alkoxycarbonyl-, Aminocarbonyl-, $C_{1-3}$-Alkylaminocarbonyloder Di-($C_{1-3}$-Alkyl)-aminocarbonylgruppe, durch eine im Phenylkern gegebenenfalls durch ein Fluor-, Chlor-, Brom- oder Jodatom, durch eine Methyl- oder Cyanogruppe substituierte Phenyl-$C_{1-3}$-alkyl- oder Phenylgruppe substituiert sein kann,

oder eine zu dem Stickstoffatom benachbarte Methylengruppe in den vorstehend erwähnten Cycloalkyleniminoringe durch eine Carbonyl- oder Sulfonylgruppe ersetzt sein kann, und die vorstehend erwähnten monosubstituierten Phenylgruppen zusätzlich durch ein Fluor-, Chlor- oder Bromatom, eine Methyl-, Amino-, $C_{1-3}$-Alkylamino- oder Di-($C_{1-3}$-Alkyl)-aminogruppe substituiert sein können, oder

an einen der vorstehend erwähnten unsubstituierten Cycloalkyleniminoringe über zwei benachbarte Kohlenstoffatome ein gegebenenfalls durch eine oder zwei $C_{1-3}$-Alkoxygruppen substituierter Phenylring ankondensiert sein kann,

bedeuten, deren Isomere und deren Salze.

Besonders bevorzugt sind die vorstehend erwähnten Verbindungen, in denen der Rest $R_2$ in 5-Stellung steht, insbsondere die folgenden Verbindungen:

(a) 3-Z-[1-(4-Aminomethyl-phenylamino)-1-phenyl-methylen]-5-amido-2-indolinon,

(b) 3-Z-[1-Phenylamino)-1-phenyl-methylen]-5-amido-2-indolinon,

(c) 3-Z-[1-(4-Brom-phenylamino)-1-phenyl-methylen]-5-amido-2-indolinon,

(d) 3-Z-[1-(4-Dimethylamino-methyl)-phenylamino)-1-phenyl-methylen] -5-amido-2-indolinon,

(e) 3-Z-[1-(4-Pyrrolidinomethyl-phenylamino)-1-phenyl-methylen] -5-amido-2-indolinon,

(f) 3-Z-[1-(4-Piperidinomethyl-phenylamino)-1-phenyl-methylen]-5-amido-2-indolinon,

(g) 3-Z-[1-(4-Hexamethyleniminomethyl-phenylamino)-1-phenylmethylen] -5-amido-2-indolinon,

(h) 3-Z-[1-(4-(4-Benzyl-piperidino)-methyl)-phenylamino)-1-phenyl-methylen]-5-amido-2-indolinon,

(i) 3-Z-[1-(4-(N-Butyl-aminomethyl)-phenylamino)-1-phenyl-methylen] -5-amido-2-indolinon,

(j) 3-Z-[1-(4-(N-(Phenyl-methyl)-aminomethyl)-phenylamino)-1-phenyl-methylen]-5-amido-2-indolinon,

(k) 3-Z-[1-(4-(N-Methyl-N-benzyl-amino-methyl)-phenylamino)-1-phenyl-methylen]-5-amido-2-indolinon,

(1) 3-Z-[1-(4-Piperidino-methyl-phenylamino)-1-phenyl-methylen]-5-dimethylcarbamoyl-2-indolinon,

(m) 3-Z-[1-(4-Piperidino-methyl-phenylamino)-1-phenyl-methylen]-5-diethylcarbamoyl-2-indolinon,

(n) 3-Z-[1-(4-(3-Diethylamino-propoxy)-phenylamino)-1-phenylmethylen]-5-amido-2-indolinon

und deren Salze.

Erfindungsgemäß erhält man die neuen Verbindungen beispielsweise nach folgenden im Prinzip literaturbekannten Verfahren:

a. Umsetzung einer Verbindung der allgemeinen Formel

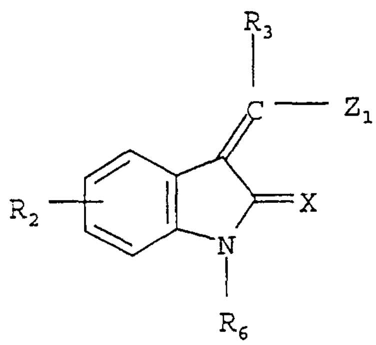

(II),

in der
X, $R_2$ und $R_3$ wie eingangs erwähnt definiert sind,
$R_6$ ein Wasserstoffatom, eine Schutzgruppe für das Stickstoffatom der Lactamgruppe oder eine Bindung an eine Festphase und
$Z_1$ ein Halogenatom, eine Hydroxy-, Alkoxy- oder Aralkoxygruppe, z.B. ein Chlor- oder Bromatom, eine Methoxy-, Ethoxy- oder Benzyloxygruppe, bedeuten,
mit einem Amin der allgemeinen Formel

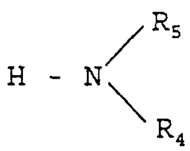

(III),

in der
$R_4$ und $R_5$ wie eingangs erwähnt definiert sind,
und erforderlichenfalls anschließende Abspaltung einer verwendeten Schutzgruppe für das Stickstoffatom der Lactamgruppe oder von einer Festphase.

Als Schutzgruppe für das Stickstoffatom der Lactamgruppe kommt beispielsweise eine Acetyl-, Benzoyl-, Ethoxycarbonyl-, tert.-Butyloxycarbonyl- oder Benzyloxycarbonylgruppe und
als Festphase ein Rink- oder Sieber-Harz Betracht.

Die Umsetzung wird zweckmäßigerweise in einem Lösungsmittel wie Dimethylformamid, Toluol, Acetonitril, Tetrahydrofuran, Dimethylsulfoxid, Methylenchlorid oder deren Gemischen gegebenenalls in Gegenwart einer inerten Base wie Triethylamin, N-Ethyl-diisopropylamin oder Natriumhydrogencarbonat bei Temperaturen zwischen 20 und 175°C durchgeführt, wobei eine verwendete Schutzgruppe infolge Umamidierung gleichzeitig abgespalten werden kann.

Bedeutet $Z_1$ in einer Verbindung der allgemeinen Formel II ein Halogenatom, dann wird die Umsetzung vorzugsweise in Gegenwart einer inerten Base bei Temperaturen zwischen 20 und 120°C, durchgeführt.

Bedeutet $Z_1$ in einer Verbindung der allgemeinen Formel II eine Hydroxy-, Alkoxy- oder Aralkoxygruppe, dann wird die Umsetzung vorzugsweise bei Temperaturen zwischen 20 und 200°C, durchgeführt.

Die gegebenenfalls erforderliche anschließende Abspaltung einer verwendeten Schutzgruppe wird zweckmäßigerweise entweder hydrolytisch in einem wäßrigen oder alkoholischen Lösungsmittel, z.B. in Methanol/Wasser, Ethanol/Wasser, Isopropanol/Wasser, Tetrahydrofuran/Wasser, Dioxan/Wasser, Dimethylformamid/Wasser, Methanol oder Ethanol in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C,
oder vorteilhafterweise durch Umamidierung mit einer primären oder sekundären organischen Base wie Ammoniak, Methylamin, Butylamin, Dimethylamin oder Piperidin in einem Lösungsmittel wie Methanol, Ethanol, Dimethylformamid und deren Gemischen oder in einem Überschuß des eingesetzten Amins bei Temperaturen zwischen 0 und 100°C, vorzugsweise bei Temperaturen zwischen 10 und 50°C, durchgeführt.

Die Abspaltung von einer verwendeten Festphase erfolgt vorzugsweise mittels Trifluoressigsäure und Wasser in Gegenwart von einem Dialkylsulfid wie Dimethylsulfid bei Temperaturen zwischen 0 und 35°C, vorzugsweise bei Raumtemperatur.

b. Zur Herstellung einer Verbindung der allgemeinen Formel I, die eine Aminomethylgruppe enthält und X ein Sauerstoffatom darstellt:

Reduktion einer Verbindung der allgemeinen Formel

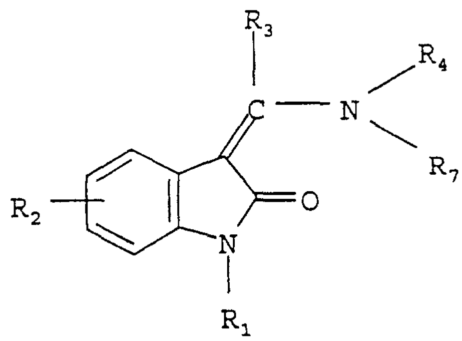

(IV),

in der
$R_1$ bis $R_4$ wie eingangs erwähnt definiert sind und
$R_7$ mit der Maßgabe die für $R_5$ eingangs erwähnten Bedeutungen aufweist, daß $R_5$ eine Cyanogruppe enthält.

Die Reduktion wird vorzugsweise mittels katalytischer Hydrierung mit Wasserstoff in Gegenwart eines Katalysators wie Palladium/Kohle oder Platin in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Erhält man erfindungsgemäß eine Verbindung der allgemeinen Formel I, die eine Alkoxycarbonylgruppe enthält, so kann diese mittels Hydrolyse in eine entsprechende Carboxyverbindung übergeführt werden, oder
eine Verbindung der allgemeinen Formel I, die eine Amino- oder Alkylaminogruppe enthält, so kann diese mittels Alkylierung oder reduktiver Alkylierung in eine entsprechende Alkylaminooder Dialkylaminoverbindung übergeführt werden, oder
eine Verbindung der allgemeinen Formel I, die eine Amino- oder Alkylaminogruppe enthält, so kann diese mittels Acylierung in eine entsprechende Acylverbindung übergeführt werden, oder
eine Verbindung der allgemeinen Formel I, die eine Carboxygruppe enthält, so kann diese mittels Veresterung oder Amidierung in eine entsprechende Ester- oder Aminocarbonylverbindung übergeführt werden.

Die anschließende Hydrolyse erfolgt vorzugsweise in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die anschließende reduktive Alkylierung wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100°C, vorzugsweise bei Temperaturen zwischen 20 und 80°C, durchgeführt.

Die anschließende Alkylierung wird mit eimem Alkylierungsmittel wie eimem Alkylhalogenid oder Dialkylsulfat wie Methyljodid, Dimethylsulfat oder Propylbromid vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart einer anorganischen oder einer tertiären organischen Base wie Triethylamin, N-Ethyl-diisopropylamin oder Dimethylaminopyridin, vorzugsweise bei Temperaturen zwischen 20°C und der Siedetemperatur des verwendeten Lösungsmittel, durchgeführt.

Die anschließende Acylierung wird vorzugsweise in einem Lösungsmittel wie Methylenchlorid, Diethylether, Tetrahydrofuran, Toluol, Dioxan, Acetonitril, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt. Hierbei wird die Acylierung mit einer entsprechenden Säure vorzugsweise in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylamino-pyridin, N-Methyl-morpholin oder Triethylamin zweckmäßigerweise bei Temperaturen zwischen 0 und 150°C, vorzugsweise bei Temperaturen zwischen 0 und 100°C, und die Acylierung mit einer entsprechenden reaktionsfähigen Verbindung wie deren Anhydrid, Ester, Imidazolide oder Halogenide gegebenenfalls in Gegenwart einer tertiären organischen Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.

Die anschließende Veresterung oder Amidierung wird zweckmäßigerweise durch Umsetzung eines reaktionsfähigen entsprechenden Carbonsäurederivates mit einem entsprechenden Alkohol oder Amin wie vorstehend beschrieben durchgeführt.

Bei den vorstehend beschriebenen Umsetzungen können gegebenenfalls vorhandene reaktive Gruppen wie Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Carboxylgruppe die Trimethylsilyl-, Methyl-, Ethyl-, tert.Butyl-, Benzyl- oder Tetrahydropyranylgruppe und als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzyl-gruppe und für die Aminogruppe zusätzlich die Phthalyl-gruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan, Essigester oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können erhaltene chirale Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen Gemisches diastereomerer Salze oder Derivate, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure, Dibenzoylweinsäure, Di-o-Tolylweinsäure, Apfelsäure, Mandelsäure, Camphersulfonsäure, Glutaminsäure, N-Acetylglutaminsäure, Asparaginsäure, N-Acetyl-asparaginsäure oder Chinasäure. Als optisch aktiver Alkohol kommt beispielsweise (+)- oder (-)-Men-thol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure, Maleinsäure oder Methansulfonsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsprodukte verwendeten Verbindungen der allgemeinen Formeln I bis VIII sind teilweise literaturbekannt oder man erhält diese nach literaturbekannten Verfahren oder werden in den Beispielen beschrieben.

Wie bereits eingangs erwähnt, weisen die neuen Verbindungen der allgemeinen Formel I, in der $R_1$ ein Wasserstofatom oder einen Prodrugrest darstellt, wertvolle pharmakologische Eigenschaften auf, insbesondere inhibierende Wirkungen auf verschiedene Kinasen und Cyclin/CDK-Komplexe, auf die Proliferation kultivierter humaner Tumor-Zellen sowie nach oraler Gabe auf das Wachstum von Tumoren in Nacktmäusen, die mit humanen Tumorzellen infiziert worden waren.

Beispielsweise wurden die in Tabelle 1 aufgeführten Verbindungen auf ihre biologischen Eigenschaften wie folgt geprüft:

Test 1

Inhibierung von Cyclin/CDK-Enzym, -Aktivität in vitro

High Five$^{TM}$ Insekten-Zellen (BTI-TN-5B1-4), die mit einem hohen Titer an rekombinantem Baculovirus infiziert waren, wurden für die Produktion von aktiven humanen Cyclin/CDK Holoenzymen benutzt. Durch die Verwendung eines Baculovirus-Vektors, der zwei Promoter enthielt (polyhedrin enhancer promoter, P10-enhancer promoter), wurden GST-tagged Cycline (z.B. Cyclin D1 oder Cyclin D3) mit der entsprechenden His6-tagged CDK-Untereinheit (z.B. für CDK4 oder CDK6) in derselben Zelle exprimiert. Das aktive Holoenzym wurde durch Affinitäts-Chromatographie an Glutathion-Sepharose isoliert. Rekombinantes GST-tagged pRB (aa 379-928) wurde in E. coli produziert und durch Affinitäts-Chromatographie an Glutathion-Sepharose gereinigt.

Die Substrate, die für die Kinase-Assays verwendet wurden, hingen von den spezifischen Kinasen ab. Histone H1 (Sigma) wurde verwendet als Substrat für Cyclin E/CDK2, Cyclin A/CDK2, Cyclin B/CDK1 und für v-Cyclin/CDK6. GST-tagged pRB (aa 379-928) wurde verwendet als Substrat für Cyclin D1/CDK4, Cyclin D3/CDK4, Cyclin D1/CDK6 und für Cyclin D3/CDK6.

Lysate der mit rekombinanten Baculovirus-infizierten Insekten-Zellen oder auch rekombinante Kinasen (erhalten aus den Lysaten durch Reinigung) wurden zusammen mit radioaktiv markiertem ATP in Gegenwart eines geeigneten Substrates mit verschiedenen Konzentrationen des Inhibitors in einer 1%igen DMSO-Lösung (Dimethylsulfoxid) 45 Minuten lang bei 30°C inkubiert. Die Substrat Proteine mit assoziierter Radioaktivität wurden mit 5%iger TCA (Trichloressigsäure) in hydrophoben PVDF multi-well Mikrotiter Platten (Millipore) oder mit 0.5%iger Phosphorsäure-Lösung auf Whatman P81 Filtern ausgefällt. Nach Zugabe von Scintillations-Flüssigkeit wurde die Radioaktivität in einem Wallace 1450 Microbeta Flüssig-Scintillations-Zähler gemessen. Pro Konzentration der Substanz wurden Doppel-Messungen durchgeführt; $IC_{50}$-Werte für die Enzym-Inhibition wurden berechnet.

Test 2

Inhibierung der Profileration von kultivierten humanen Tumorzellen

Zellen der Leiomyosarcoma Tumorzell-Linie SK-UT-1B (erhalten von der American Type Culture Collection (ATCC)) wurden in Minimum Essential Medium mit nicht essentiellen Aminosäuren (Gibco), ergänzt mit Natrium-Pyruvat (1 mMol), Glutamin (2 mMol) und 10% fötalem Rinderserum (Gibco) kultiviert und in der log-Wachstumsphase geerntet. Anschließend wurden die SK-UT-1B-Zellen in Cytostar® multi-well Platten (Amersham) mit einer Dichte von 4000 cells per well eingebracht und über Nacht in einem Inkubator inkubiert. Verschiedene Konzentrationen der Verbindungen (gelöst in DMSO; Endkonzentration: <1%) wurden zu den Zellen zugegeben. Nach 48 Stunden Inkubation wurde $^{14}C$-Thymidin (Amersham) zu jedem well zugesetzt, und es wurde weitere 24 Stunden inkubiert. Die Menge an $^{14}C$-Thymidin, die in Gegenwart des Inhibitors in die Tumorzellen eingebaut wurde und die die Zahl der Zellen in

der S-Phase repräsentiert, wurde in einem Wallace 1450 Microbeta Flüssig Scintillations Zähler gemessen. $IC_{50}$-Werte für die Inhibierung der Proliferation (= Inhibierung von eingebautem $^{14}C$-Thymidin) wurden - unter Korrektur für die Hintergrundstrahlung - berechnet. Alle Messungen wurden zweifach ausgeführt.

Test 3

In vivo Effekte an Tumor-tragenden Nacktmäusen

$10^6$ Zellen [SK-UT-1B, oder non-small cell Lungen-Tumor NCI-H460 (erhalten von ATCC)] in einem Volumen von 0.1 ml wurden in männliche und/oder weibliche Nacktmäuse (NMRI nu/nu; 25-35 g; N = 10-20) subkutan injiziert; alternativ wurden kleine Stückchen von SK-UT-1B- oder NCI-H460-Zellklumpen subkutan implantiert. Eine bis drei Wochen nach Injektion bzw. Implantation wurde ein Kinase-Inhibitor täglich für die Dauer von 2 bis 4 Wochen oral (per Schlundsonde) appliziert. Die Tumor-Größe wurde dreimal pro Woche mit einer digitalen Schieblehre gemessen. Der Effekt eines Kinase-Hemmers auf das Tumor-Wachstum wurde als Prozentinhibierung im Vergleich zu einer mit Placebo behandelten Kontroll-Gruppe bestimmt.

Die nachfolgende Tabelle enthält die gefundenen Ergebnisse des in vitro-Tests 2:

| Verbindung (Beispiel Nr.) | Hemmung der SKUT-1B-Proliferation $IC_{50}$ [µM] |
|---|---|
| 4( 2) | 0.17 |
| 4( 14) | 0.18 |
| 4( 62) | 0.05 |
| 4( 53) | 0.01 |
| 4( 54) | 0.03 |
| 4( 60) | 0.03 |
| 4(120) | 0.04 |
| 4(122) | 0.04 |
| 4( 94) | 0.03 |
| 3( 3) | 0.01 |
| 3( 7) | 0.01 |
| 4(129) | 0.04 |

Auf Grund ihrer biologischen Eigenschaften eignen sich die neuen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören (ohne Anspruch auf Vollständigkeit): Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphoma und solide Tumore; Haut-Erkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung.

Die neuen Verbindungen können zur Kurz- oder Langzeitbehandlung der vorstehend erwähnten Krankheiten auch gegebenenfalls in Kombination mit anderen "State-of-art" Verbindungen wie anderen Cytostatika verwendet werden.

Die zur Erzielung einer entsprechenden Wirkung erforderliche Dosierung beträgt zweckmäßigerweise bei intravenöser Gabe 0,1 bis 30 mg/kg, vorzugsweise 0,3 bis 10 mg/kg, und bei oraler Gabe 0,1 bis 100 mg/kg, vorzugsweise 0,3 bis 30 mg/kg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Ge mischen, in übliche galenische Zubereitungen wie Tabletten, Dragées, Kapseln, Pulver, Suspensionen, Zäpfchen oder als Lösungen für Injektionen oder Infusionen einarbeiten.

Die nachfolgenden Beispiele sollen die Erfindung näher erläutern:

Beispiel I

1-Acetyl-2-indolinon-5-carbonsäuremethylester

10,5 g 2-Indolinon-5-carbonsäuremethylester (Hergestellt analog Ogawa et al. Chem.Pharm.Bull 36, 2253-2258 (1988)) werden in 30 ml Acetanhydrid 4 Stunden bei 140°C gerührt. Anschließend läßt man abkühlen, gießt auf Eiswasser und saugt den Niederschlag ab. Das Produkt wird nochmals mit Wasser gewaschen, dann in Methylenchlorid aufgenommen, über Natriumsulfat getrocknet und eingeengt.
Ausbeute: 11 g (86 % der Theorie),
$R_f$-Wert: 0,63 (Kieselgel; Methylenchlorid/Methanol = 50:1)

Beispiel II

1-Acetyl-3-(1-ethoxy-1-phenyl-methylen)-2-indolinon-5-carbonsäuremethylester

11 g 1-Acetyl-2-indolinon-5-carbonsäuremethylester werden in 110 ml Acetanhydrid und 30 ml Orthobenzoesäuretriethylester 2 Stunden bei 100°C gerührt. Anschließend wird einrotiert, der Rückstand mit Ether gewaschen und abgesaugt.
Ausbeute: 11,5 g (67 % der Theorie),
$R_f$-Wert: 0,55 (Kieselgel, Methylenchlorid/Petrolether/Essigester = 4:5:1)

Beispiel III

28,0 g Rink-Harz (MBHA-Harz, Firma Novobiochem) läßt man in 330 ml Dimethylformamid quellen. Anschließend gibt man 330 ml 30 % Piperidin in Dimethylformamid zu und schüttelt 7 Minuten, um die FMOC-Schutzgruppe abzuspalten. Dann wird das Harz mehrmals mit Dimethylformamid gewaschen. Schließlich gibt man 7,3 g 2-Indolinon-5-carbonsäure, 5,6 g Hydroxybenzotriazol, 13,3 g O-(Benzotriazol-1-yl)-N,N,N',N'-tetramethyl-uronium-tetrafluorborat und 5,7 ml N-Ethyl-diisopropylamin in 300 ml Dimethylformamid zu und schüttelt 1 Stunde. Nun wird die Lösung abgesaugt und das Harz fünfmal mit 300 ml Dimethylformamid und dreimal mit 300 ml Methylenchlorid gewaschen. Zum Trocknen wird Stickstoff durch das Harz geblasen.
Ausbeute: 28 g beladenes Harz

Beispiel IV

5 g des gemäß Beispiel III hergesetellten belegten Harzes werden mit 15 ml Acetanhydrid bei 80°C 1 Stunde gerührt. Dann gibt man 15 ml Orthobenzoesäuretriethylester zu und schüttelt weitere 3 Stunde bei 110°C. Danach wird das Harz abgesaugt und mit Dimethylformamid, Methanol und schließlich mit Methylenchlorid gewaschen.
Ausbeute: 7 g feuchtes Harz

Beispiel V

4-(Ethylamino-methyl)-nitrobenzol

6 g 4-Nitrobenzylbromid werden in 25 ml Ethanol gelöst, mit 25 ml 10%iger ethanolischer Ethylaminlösung versetzt und 2 Stunden am Rückfluß erhitzt. Dann wird die Lösung einrotiert, der Rückstand mit Methylenchlorid aufgenommen und mit verdünnter Natronlauge gewaschen. Schließlich wird die organische Phase eingeengt.
Ausbeute: 2.3 g (46 % der Theorie),
$R_f$-Wert: 0,2 (Kieselgel; Methylenchlorid/Methanol = 9:1)

Analog werden hergestellt:

4-[N-(4-Chlorphenyl-methyl)-amino-methyl]-nitrobenzol

4-(N-Cyclohexyl-amino-methyl)-nitrobenzol

4-(N-Isopropyl-amino-methyl)-nitrobenzol

4-(N-Butyl-amino-methyl)-nitrobenzol

4-(N-Methoxycarbonyl-methyl-amino-methyl)-nitrobenzol

4-(N-(Phenyl-methyl)-amino-methyl)-nitrobenzol

4-(Pyrrolidino-methyl)-nitrobenzol

4-(Morpholino-methyl)-nitrobenzol

4-(Piperidino-methyl)-nitrobenzol

4-(Hexamethylenimino)-nitrobenzol

4-(4-Hydroxy-piperidino-methyl)-nitrobenzol

4-(4-Methyl-piperidino-methyl)-nitrobenzol

4-(4-Ethyl-piperidino-methyl)-nitrobenzol

4-(4-Isopropyl-piperidino-methyl)-nitrobenzol

4-(4-Phenyl-piperidino-methyl)-nitrobenzol

4-(4-Benzyl-piperidino-methyl)-nitrobenzol

4-(4-Ethoxycarbonyl-piperidino-methyl)-nitrobenzol.

4-(Dimethylamino-methyl)-nitrobenzol

4-(Dipropylamino-methyl)-nitrobenzol

4-(4-tert.Butyloxycarbonyl-piperazino-methyl)-nitrobenzol

3-(Dimethylamino-methyl)-nitrobenzol

4-(2-Diethylamino-ethyl)-nitrobenzol

4-(2-Morpholinyl-ethyl)-nitrobenzol

4-(2-Pyrrolidinyl-ethyl)-nitrobenzol

4-(2-Piperidinyl-ethyl)-nitrobenzol

4-(N-Ethyl-N-benzyl-amino-methyl)-nitrobenzol

4-(N-Propyl-N-benzyl-amino-methyl)-nitrobenzol

4-[N-Methyl-N-(4-chlorphenylmethyl)-amino-methyl])-nitrobenzol

4-[N-Methyl-N-(4-bromphenylmethyl)-amino-methyl]-nitrobenzol

4-[N-Methyl-N-(3-chlorphenylmethyl)-amino-methyl]-nitrobenzol

4-[N-Methyl-N-(3,4-dimethoxyphenylmethyl)-amino-methyl]-nitrobenzol

4-[N-Methyl-N-(4-methoxyphenylmethyl)-amino-methyl]-nitrobenzol

4-[N-2,2,2-Trifluorethyl-N-(phenylmethyl)-amino-methyl]-nitrobenzol

4-[N-2,2,2-Trifluorethyl-N-(4-chlorphenylmethyl)-amino-methyl]-nitrobenzol

Beispiel VI

4-(N-Ethyl-N-tert.butoxycarbonyl-amino-methyl)-nitrobenzol

2,2 g 4-(Ethylamino-methyl)-nitrobenzol werden in 50 ml Essigester gelöst und mit 2,6 g Di-tert-butyl-dicarbonat 30 Minuten bei Raumtemperatur gerührt. Anschließend wird die Lösung mit Wasser gewaschen und eingeengt.
Ausbeute: 3,4 g,
$R_f$-Wert: 0,9 (Kieselgel, Methylenchlorid/Methanol = 9:1)
Analog werden hergestellt:

4-[N-(4-Chlorphenyl-methyl)-N-tert.butoxycarbonyl-amino-methyl)-nitrobenzol

4-(N-Cyclohexyl-N-tert.butoxycarbonyl-amino-methyl)-nitrobenzol

4-(N-Isopropyl-N-tert.butoxycarbonyl-amino-methyl)-nitrobenzol

4-(N-Butyl-N-tert.butoxycarbonyl-amino-methyl)-nitrobenzol

4-(N-Methoxycarbonyl-methyl-N-tert.butoxycarbonyl-amino-methyl)-nitrobenzol

4-(N-(Phenyl-methyl)-N-tert.butoxycarbonyl-amino-methyl)-nitrobenzol

Beispiel VII

4-(N-Ethyl-N-tert.butoxycarbonyl-amino-methyl)-anilin

6,4 g 4-(N-Ethyl-N-tert.butoxycarbonyl-amino-methyl)-nitrobenzol werden in 60 ml Methanol gelöst und mit 1,5 g Raney-Nickel bei Raumtemperatur und 3 bar hydriert. Anschließend wird der Katalysator abfiltriert und die Lösung eingeengt.
Ausbeute: 4,78 g,
$R_f$-Wert: 0,7 (Kieselgel, Methylenchlorid/Methanol 50:1)
Analog werden hergestellt:

4-[N-(4-Chlorphenyl-methyl)-N-tert.butoxycarbonyl-amino-methyl] -anilin

4-(N-Cyclohexyl-N-tert.butoxycarbonyl-amino-methyl)-anilin

4-(N-Isopropyl-N-tert.butoxycarbonyl-amino-methyl)-anilin

4-(N-Butyl-N-tert.butoxycarbonyl-amino-methyl)-anilin

4-(N-Methoxycarbonyl-methyl-N-tert.butoxycarbonyl-amino-methyl)-anilin

4-(N-(Phenyl-methyl)-N-tert.butoxycarbonyl-amino-methyl)-anilin

4-(Pyrrolidino-methyl)-anilin

4-(Morpholino-methyl)-anilin

4-(Piperidino-methyl)-anilin

4-(Hexamethylenimino-methyl)-anilin

4-(4-Hydroxy-piperidino-methyl)-anilin

4-(4-Methyl-piperidino-methyl)-anilin

4-(4-Ethyl-piperidino-methyl)-anilin

4-(4-Isopropyl-piperidino-methyl)-anilin

4-(4-Phenyl-piperidino-methyl)-anilin

4-(4-Benzyl-piperidino-methyl) -anilin

4-(4-Ethoxycarbonyl-piperidino-methyl)-anilin

4-(2-Morpholinyl-ethyl)-anilin

4-(2-Pyrrolidinyl-ethyl)-anilin

4-(2-Piperidinyl-ethyl)-anilin

4-(N-Ethyl-N-benzyl-amino-methyl)-anilin

4-(N-Propyl-N-benzyl-amino-methyl)-anilin

4-[N-Methyl-N-(4-chlorphenylmethyl)-amino-methyl]-anilin

4-[N-Methyl-N-(4-bromphenylmethyl)-amino-methyl]-anilin

4-[N-Methyl-N-(3-chlorphenylmethyl)-amino-methyl]-anilin

4-[N-Methyl-N-(3,4-dimethoxyphenylmethyl)-amino-methyl]-anilin

4-[N-Methyl-N-(4-methoxyphenylmethyl)-amino-methyl]-anilin

4-[N-2,2,2-Trifluorethyl-N-(phenylmethyl)-amino-methyl]-anilin

4-[N-2,2,2-Trifluorethyl-N-(4-chlorphenylmethyl)-amino-methyl]-anilin

Herstellung der Endprodukte:

Beispiel 1

3-Z-[1-(1-Methyl-piperidin-4-yl-amino)-1-phenyl-methylen]-2-indolinon-5-carbonsäuremethylester

11,5 g 1-Acetyl-3-(1-ethoxy-1-phenyl-methylen)-2-indolinon-5-carbonsäuremethylester werden in 115 ml Methylenchlorid gelöst und mit 10,8 g 4-Amino-N-methylpiperidin 5 Stunden bei Raumtemperatur gerührt. Anschließend gibt man 20 ml methanolischen Ammoniak zu und läßt über Nacht stehen. Die Lösung wird eingedampft und der Rückstand mit Ether gewaschen.
Ausbeute: 11,9 g (97 % der Theorie),
$R_f$-Wert: 0,20 (Kieselgel; Methylenchlorid/Methanol = 9:1) $C_{23}H_{25}N_3O_3$
Massenspektrum: m/z = 391 ($M^+$)

Analog werden hergestellt:

(1) 3-Z-[1-(4-(Piperidino-methyl)-phenylamino)-1-phenyl-methylen]-2-indolinon-5-carbonsäuremethylester
$R_f$-Wert: 0,4 (Kieselgel, Methylenchlorid/Methanol = 9:1)
$C_{29}H_{29}N_3O_3$
Massenspektrum: m/z = 467 ($M^+$)

(2) 3-Z-[1-(4-(N-Phenylmethyl-N-methylamino-methyl)-phenylamino)-1-phenyl-methylen]-2-indolinon-5-carbonsäuremethylester

$C_{32}H_{29}N_3O_3$
Massenspektrum: m/z = 503 ($M^+$)

(3) 3-Z-[1-(4-(Dimethylamino-methyl)-phenylamino)-1-phenyl-methylen] -2-indolinon-5-carbonsäuremethylester
$C_{26}H_{25}N_3O_3$
Massenspektrum: m/z = 427 ($M^+$)

(4) 3-Z-[1-(3-(Dimethylamino-methyl)-phenylamino)-1-phenylmethylen]-2-indolinon-5-carbonsäuremethylester
$C_{26}H_{25}N_3O_3$
Massenspektrum: m/z = 427 ($M^+$)

(5) 3-Z-[1-(4-Chlor-phenylamino)-1-phenyl-methylen]-2-indolinon-5-carbonsäuremethylester

(6) 3-Z-(1-Phenylamino-1-phenyl-methylen)-2-indolinon-5-carbonsäuremethylester

Beispiel 2

3-Z-[1-(1-Methyl-piperidin-4-yl-amino)-1-phenyl-methylen]-2-indolinon-5-carbonsäure

11,9 g 3-Z-[1-(1-Methyl-piperidin-4-yl-amino)-1-phenyl-methylen]-2-indolinon-5-carbonsäuremethylester werden in 300 ml Methanol und 150 ml 1N Natronlauge 4 Stunden am Rückfluß erhitzt. Anschließend neutralisiert man mit 150 ml 1N Salzsäure und engt zur Trockene ein. Der Rückstand wird mehrmals mit Wasser gewaschen und getrocknet.
Ausbeute: 86 % der Theorie,
$R_f$-Wert: 0,17 (Kieselgel; Methylenchlorid/Methanol = 4:1)
$C_{22}H_{23}N_3O_3$
Massenspektrum: m/z = 377 ($M^+$)
Analog werden hergestellt:

(1) 3-Z-[1-(4-(Piperidino-methyl)-phenylamino)-1-phenyl-methylen]-2-indolinon-5-carbonsäure
$R_f$-Wert: 0,15 (Kieselgel, Methylenchlorid/Methanol = 9:1)
$C_{28}H_{27}N_3O_3$
Massenspektrum: m/z = 453 ($M^+$)

(2) 3-Z-[1-(4-(N-Phenylmethyl-N-methylamino-methyl)-phenylamino) -1-phenyl-methylen)-2-indolinon-5-carbonsäure
$C_{31}H_{27}N_3O_3$
Massenspektrum: m/z = 489 ($M^+$)

(3) 3-Z-[1-(4-(Dimethylamino-methyl)-phenylamino)-1-phenyl-methylen]-2-indolinon-5-carbonsäure
$C_{25}H_{23}N_3O_3$
Massenspektrum: m/z = 413 ($M^+$)

(4) 3-Z-[1-(3-(Dimethylamino-methyl)-phenylamino)-1-phenyl-methylen)-2-indolinon-5-carbonsäure
$C_{25}H_{23}N_3O_3$
Massenspektrum: m/z = 413 ($M^+$)

(5) 3-Z-[1-(4-Chlor-phenylamino)-1-phenyl-methylen]-2-indolinon-5-carbonsäure

(6) 3-Z-[1-Phenylamino-1-phenyl-methylen)-2-indolinon-5-carbonsäure

Beispiel 3

3-Z-[1-(1-Methyl-piperidin-4-yl-amino)-1-phenyl-methylen]-5-dimethylcarbamoyl-2-indolinon

2 g 3-Z-[1-(1-Methyl-piperidin-4-yl-amino)-1-phenyl-methylen]-2-indolinon-5-carbonsäure werden mit 5 ml Thionylchlorid 2 Stunden am Rückfluß erhitzt. Anschließend wird einrotiert und der Rückstand mit Ether gewaschen. 0,5 g dieses Säurechlorides werden ohne weitere Reinigung in 5 ml Methylenchlorid aufgenommen und mit 0,5 ml

Dimethylamin in 5 ml Methylenchlorid versetzt und über Nacht bei Raumtemperatur gerührt. Das Produkt wird über eine Kieselgelsäule mit Methylenchlorid/Methanol/Ammoniak (4:1:0.1) chromatographiert.
Ausbeute: 50 % der Theorie,
$R_f$-Wert: 0,14 (Kieselgel: Methylenchlorid/Methanol = 9:1)
$C_{24}H_{28}N_4O_2$
Massenspektrum: m/z = 404 ($M^+$)

Analog werden folgende Verbindungen hergestellt:

(1) 3-Z-[1-(1-Methyl-piperidin-4-yl-amino)-1-phenyl-methylen]-5-methylcarbamoyl-2-indolinon
Ausbeute: 49 % der Theorie,
$R_f$-Wert: 0,19 (Kieselgel; Methylenchlorid/Methanol = 4:1)
$C_{23}H_{26}N_4O_2$
Massenspektrum: m/z = 390 ($M^+$)

(2) 3-Z-[1-(1-Methyl-piperidin-4-yl-amino)-1-phenyl-methylen]-5-carbamoyl-2-indolinon
Ausbeute: 58 % der Theorie,
$R_f$-Wert: 0,15 (Kieselgel; Methylenchlorid/Methanol = 4:1)
$C_{22}H_{24}N_4O_2$
Massenspektrum: m/z = 376 ($M^+$)

(3) 3-Z-[1-(4-Piperidino-methyl-phenylamino)-1-phenyl-methylen] -5-dimethylcarbamoyl-2-indolinon
Hergestellt aus 3-Z-[1-(4-Piperidino-methyl-phenylamino)-1-phenyl-methylen]-2-indolinon-5-carbonsäure und Dimethylamin oder es werden 0,64 g Z-[1-(4-Piperidino-methyl-phenylamino)-1-phenyl-methylen] -2-indolinon-5-carbonsäure, 0.34 g Dimethylaminhydrochlorid, 0.9 g O-Benzotriazol-1-yl-N,N,N',N'-tetramethyluronium-tetrafluoroborat), 0.4 g 1-Hydroxy-1H-benztriazol und 2,9 g Diisopropylethylamin werden in 20 ml Dimethylformamid 20 Stunden bei Raumtemperatur gerührt. Anschließend wird eingeengt und der Rückstand in Wasser suspendiert. Der Niederschlag wird abgesaugt.
Ausbeute: 600 mg (88% der Theorie),
$R_f$-Wert: 0,2 (Kieselgel, Methylenchlorid/Ethanol = 9:1)
$C_{30}H_{32}N_4O_2$
Massenspektrum: m/z = 481 $(M+H)^+$

(4) 3-Z-[1-(4-Piperidino-methyl-phenylamino)-1-phenyl-methylen]-5-methylcarbamoyl-2-indolinon
Hergestellt aus 3-Z-[1-(4-Piperidino-methyl-phenylamino)-1-phenyl-methylen]-2-indolinon-5-carbonsäure und Methylamin analog Beispiel 3(3).
$R_f$-Wert: 0,2 (Kieselgel, Methylenchlorid/Ethanol = 9:1)
$C_{29}H_{30}N_4O_2$
Massenspektrum: m/z = 467 $(M+H)^+$

(5) 3-Z-[1-(4-Piperidino-methyl-phenylamino)-1-phenyl-methylen]-5-methylethylcarbamoyl-2-indolinon
Hergestellt aus 3-Z-[1-(4-Piperidino-methyl-phenylamino)-1-phenyl-methylen]-2-indolinon-5-carbonsäure und Methylethylamin analog Beispiel 3(3).
$R_f$-Wert: 0,55 (Kieselgel, Methylenchlorid/Ethanol = 9:1)
$C_{31}H_{34}N_4O_2$
Massenspektrum: m/z = 495 $(M+H)^+$

(6) 3-Z-[1-(4-Piperidino-methyl-phenylamino)-1-phenyl-methylen]-5-propylcarbamoyl-2-indolinon
Hergestellt aus 3-Z-[1-(4-Piperidino-methyl-phenylamino)-1-phenyl-methylen]-2-indolinon-5-carbonsäure und Propylamin analog Beispiel 3(3).
$R_f$-Wert: 0,31 (Kieselgel, Methylenchlorid/Ethanol = 9:1)
$C_{31}H_{34}N_4O_2$
Massenspektrum: m/z = 495 $(M+H)^+$

(7) 3-Z-[1-(4-Piperidino-methyl-phenylamino)-1-phenyl-methylen]-5-diethylcarbamoyl-2-indolinon
Hergestellt aus 3-Z-[1-(4-Piperidino-methyl-phenylamino)-1-phenyl-methylen]-2-indolinon-5-carbonsäure und Diethylamin anlog Beispiel 3(3).
$R_f$-Wert: 0,55 (Kieselgel, Methylenchlorid/Ethanol = 9:1)
$C_{32}H_{36}N_4O_2$

Massenspektrum: m/z = 509 $(M+H)^+$

(8) 3-Z-[1-(4-(N-Phenylmethyl-N-methyl-aminomethyl)-phenylamino)-1-phenyl-methylen]-5-methylcarbamoyl-2-indolinon

(9) 3-Z-[1-(4-(N-Phenylmethyl-N-methyl-aminomethyl)-phenylamino)-1-phenyl-methylen]-5-dimethylcarbamoyl-2-indolinon

(10) 3-Z-[1-(4-(N-Phenylmethyl-N-methyl-aminomethyl)-phenylamino)-1-phenyl-methylen]-5-diethylcarbamoyl-2-indolinon

(11) 3-Z-[1-(4-(N-Phenylmethyl-N-methyl-aminomethyl)-phenylamino)-1-phenyl-methylen]-5-propylcarbamoyl-2-indolinon

(12) 3-Z-[1-(4-(N-Phenylmethyl-N-methyl-aminomethyl)-phenylamino)-1-phenyl-methylen]-5-dipropylcarbamoyl-2-indolinon

(13) 3-Z-[1-(4-(Dimethylamino-methyl)-phenylamino)-1-phenylmethylen]-5-methylcarbamoyl-2-indolinon

(14) 3-Z-[1-(4-(Dimethylamino-methyl)-phenylamino)-1-phenyl-methylen]-5-dimethylcarbamoyl-2-indolinon

(15) 3-2-[1-(4-(Dimethylamino-methyl)-phenylamino-1-phenyl-methylen]-5-diethylcarbamoyl-2-indolinon

(16) 3-Z-[1-(4-(Dimethylamino-methyl)-phenylamino)-1-phenyl-methylen]-5-propylcarbamoyl-2-indolinon

(17) 3-Z-[1-(4-(Dimethylamino-methyl)-phenylamino)-1-phenyl-methylen]-5-dipropylcarbamoyl-2-indolinon

(18) 3-Z-[1-(3-(Dimethylamino-methyl)-phenylamino)-1-phenyl-methylen]-5-methylcarbamoyl-2-indolinon

(19) 3-Z-[1-(3-(Dimethylamino-methyl)-phenylamino)-1-phenyl-methylen]-5-dimethylcarbamoyl-2-indolinon

(20) 3-Z-[1-(3-(Dimethylamino-methyl)-phenylamino)-1-phenyl-methylen]-5-diethylcarbamoyl-2-indolinon

(21) 3-Z-[1-(3-(Dimethylamino-methyl)-phenylamino)-1-phenyl-methylen]-5-propylcarbamoyl-2-indolinon

(22) 3-Z-[1-(3-(Dimethylamino-methyl)-phenylamino)-1-phenyl-methylen]-5-dipropylcarbamoyl-2-indolinon

(23) 3-Z-[1-(4-Chlor-phenylamino)-1-phenyl-methylen]-5-methylcarbamoyl-2-indolinon

(24) 3-Z-[1-(4-Chlor-phenylamino)-1-phenyl-methylen]-5-dimethylcarbamoyl-2-indolinon

(25) 3-Z-[1-(4-Chlor-phenylamino-1-phenyl-methylen]-5-diethylcarbamoyl-2-indolinon

(26) 3-Z-[1-(4-Chlor-phenylamino)-1-phenyl-methylen]-5-propylcarbamoyl-2-indolinon

(27) 3-Z-[1-(4-Chlor-phenylamino)-1-phenyl-methylen]-5-dipropylcarbamoyl-2-indolinon

(28) 3-Z-(1-Phenylamino-1-phenyl-methylen)-5-methylcarbamoyl-2-indolinon

(29) 3-Z-(1-Phenylamino-1-phenyl-methylen)-5-dimethylcarbamoyl-2-indolinon

(30) 3-Z-(1-Phenylamino-1-phenyl-methylen)-5-diethylcarbamoyl-2-indolinon

(31) 3-Z-(1-Phenylamino-1-phenyl-methylen)-5-propylcarbamoyl-2-indolinon

(32) 3-Z-(1-Phenylamino-1-phenyl-methylen)-5-dipropylcarbamoyl-2-indolinon

Beispiel 4

3-Z-[1-(4-Amino-phenylamino)-1-phenyl-methylen]-5-amido-2-indolinon

800 mg gemäß Beispiel IV hergestelltes Harz werden in 4 ml Methylenchlorid suspendiert und mit 0,8 g 1,4-Phenylendiamin 16 Stunden bei Raumtemperatur geschüttelt. Es wird abfiltriert und das Harz mehrmals mit Methylenchlorid, Methanol und Dimethylformamid gewaschen. Anschließend gibt man für 2 Stunden 3 ml methanolischen Ammoniak zu, um die Acetylgruppe zu entfernen. Schließlich gibt man nach weiterem Waschen 4 ml 10%ige Trifluoressigsäure in Methylenchlorid während 90 Minuten zu, trennt das Harz ab und engt die Lösung ein. Der Rückstand wird mit wenig 1N Natronlauge aufgenommen und mit Methylenchlorid extrahiert. Die organische Phase wird über Natriumsulfat getrocknet und einrotiert.
Ausbeute: 45 mg (30 % der Theorie über alle Stufen),
$R_f$-Wert: 0,26 (Kieselgel; Methylenchlorid/Methanol = 9:1)
$C_{22}H_{18}N_4O_2$
Massenspektrum: m/z = 370 ($M^+$)

Analog werden folgende Verbindungen hergestellt:

(1) 3-Z-[1-(3-Amino-phenylamino)-1-phenyl-methylen]-5-amido-2-indolinon
Ausbeute: 24 % der Theorie,
$R_f$-Wert: 0,44 (Kieselgel; Methylenchlorid/Methanol = 9:1)
$C_{22}H_{18}N_4O_2$
Massenspektrum: m/z = 370 ($M^+$)

(2) 3-Z-(1-Phenylamino-1-phenyl-methylen)-5-amido-2-indolinon Ausbeute: 27 % der Theorie,
$R_f$-Wert: 0,53 (Kieselgel; Methylenchlorid/Methanol 9:1)
$C_{22}H_{17}N_3O_2$
Massenspektrum: m/z = 355 ($M^+$)

(3) 3-Z-[1-(4-Acetylamino-phenylamino) -1-phenyl-methylen]-5-amido-2-indolinon
Ausbeute: 28 % der Theorie,
$R_f$-Wert: 0,35 (Kieselgel; Methylenchlorid/Methanol = 9:1)
$C_{24}H_{20}N_4O_3$
Massenspektrum: m/z = 412 ($M^+$)

(4) 3-Z-[1-(4-Acetyl-N-methyl-amino-phenylamino)-1-phenylmethylen] -5-amido-2-indolinon
Ausbeute: 15 % der Theorie,
$R_f$-Wert: 0,36 (Kieselgel; Methylenchlorid/Methanol = 9:1)
$C_{25}H_{22}N_4O_3$
Massenspektrum: m/z = 426 ($M^+$)

(5) 3-Z-[1-(4-(2-Amino-ethyl)-phenylamino)-1-phenyl-methylen]-5-amido-2-indolinon
Ausbeute: 30 % der Theorie,
$R_f$-Wert: 0,04 (Kieselgel; Methylenchlorid/Methanol = 9:1)
$C_{24}H_{22}N_4O_2$
Massenspektrum: m/z = 398 ($M^+$)

(6) 3-Z-[1-(4-Methoxy-phenylamino)-1-phenyl-methylen]-5-amido-2-indolinon
Ausbeute: 32 % der Theorie,
$R_f$-Wert: 0,48 (Kieselgel; Methylenchlorid/Methanol = 9:1)
$C_{23}H_{19}N_3O_3$
Massenspektrum: m/z = 385 ($M^+$)

(7) 3-Z-[1-(4-Biphenylamino)-1-phenyl-methylen]-5-amido-2-indolinon
Ausbeute: 22 % der Theorie,
$R_f$-Wert: 0,51 (Kieselgel; Methylenchlorid/Methanol = 9:1)
$C_{28}H_{21}N_3O_2$
Massenspektrum: m/z = 431 ($M^+$)

(8) 3-Z-[1-(3-Pyridylamino)-1-phenyl-methylen]-5-amido-2-indolinon
Ausbeute: 35 % der Theorie,
$R_f$-Wert: 0,41 (Kieselgel; Methylenchlorid/Methanol = 9:1)
$C_{21}H_{16}N_4O_2$
Massenspektrum: m/z = 356 ($M^+$)

(9) 3-Z-[1-(4-Dimethylamino-phenylamino)-1-phenyl-methylen]-5-amido-2-indolinon
Ausbeute: 19 % der Theorie,
$R_f$-Wert: 0,49 (Kieselgel; Methylenchlorid/Methanol = 9:1)
$C_{24}H_{22}N_4O_2$
Massenspektrum: m/z = 398 ($M^+$)

(10) 3-Z-[1-(4-Morpholino-phenylamino)-1-phenyl-methylen]-5-amido-2-indolinon
Ausbeute: 42 % der Theorie,
$R_f$-Wert: 0,48 (Kieselgel; Methylenchlorid/Methanol = 9:1)
$C_{26}H_{24}N_4O_3$
Massenspektrum: m/z = 440 ($M^+$)

(11) 3-Z-[1-(4-tert.Butyl-phenylamino)-1-phenyl-methylen]-5-amido-2-indolinon
Ausbeute: 32 % der Theorie,
$R_f$-Wert: 0,48 (Kieselgel; Methylenchlorid/Methanol = 9:1)
$C_{26}H_{25}N_3O_2$
Massenspektrum: m/z = 411 ($M^+$)

(12) 3-Z-[1-(2-Amino-phenylamino)-1-phenyl-methylen]-5-amido-2-indolinon
Ausbeute: 28 % der Theorie,
$R_f$-Wert: 0,52 (Kieselgel; Methylenchlorid/Methanol = 9:1)
$C_{22}H_{18}N_4O_2$
Massenspektrum: m/z = 370 ($M^+$)

(13) 3-Z-[1-(4-Benzyloxy-phenylamino)-1-phenyl-methylen]-5-amido-2-indolinon
Ausbeute: 40 % der Theorie,
$R_f$-Wert: 0,4 (Kieselgel; Methylenchlorid/Methanol = 9:1)
$C_{29}H_{23}N_3O_3$
Massenspektrum: m/z = 461 ($M^+$)

(14) 3-Z-[1-(4-Brom-phenylamino)-1-phenyl-methylen]-5-amido-2-indolinon
Ausbeute: 35 % der Theorie,
$R_f$-Wert: 0,46 (Kieselgel; Methylenchlorid/Methanol = 9:1) $C_{22}H_{16}BrN_3O_2$
Massenspektrum: m/z = 433/435 ($M^+$)

(15) 3-Z-[1-(4-Methoxycarbonyl-phenylamino)-1-phenyl-methylen]-5-amido-2-indolinon
Ausbeute: 34 % der Theorie,
$R_f$-Wert: 0,36 (Kieselgel; Methylenchlorid/Methanol = 9:1)
$C_{24}H_{19}N_3O_4$
Massenspektrum: m/z = 413 ($M^+$)

(16) 3-Z-[1-(3-Amido-phenylamino)-1-phenyl-methylen]-5-amido-2-indolinon
Ausbeute: 32 % der Theorie,
$R_f$-Wert: 0,32 (Kieselgel; Methylenchlorid/Methanol = 9:1)
$C_{23}H_{18}N_4O_3$
Massenspektrum: m/z = 398 ($M^+$)

(17) 3-Z-[1-(3-Methyl-phenylamino)-1-phenyl-methylen]-5-amido-2-indolinon
Ausbeute: 12 % der Theorie,
$R_f$-Wert: 0,5 (Kieselgel; Methylenchlorid/Methanol = 9:1)
$C_{23}H_{19}N_3O_2$
Massenspektrum: m/z = 369 ($M^+$)

(18) 3-Z-[1-(2-Methyl-phenylamino)-1-phenyl-methylen]-5-amido-2-indolinon
Ausbeute: 21 % der Theorie,
$R_f$-Wert: 0,5 (Kieselgel; Methylenchlorid/Methanol = 9:1)
$C_{23}H_{19}N_3O_2$
Massenspektrum: m/z = 369 ($M^+$)

(19) 3-Z-[1-(3-Methoxy-phenylamino)-1-phenyl-methylen]-5-amido-2-indolinon
$R_f$-Wert: 0,49 (Kieselgel; Methylenchlorid/Methanol = 9:1)
$C_{23}H_{19}N_3O_3$
Massenspektrum: m/z = 385 ($M^+$)

(20) 3-Z-[1-(3-Ethoxycarbonyl-phenylamino)-1-phenyl-methylen]-5-amido-2-indolinon
$R_f$-Wert: 0,48 (Kieselgel; Methylenchlorid/Methanol = 9:1)
$C_{25}H_{21}N_3O_4$
Massenspektrum: m/z = 427 ($M^+$)

(21) 3-Z- [1-(3-Nitro-phenylamino)-1-phenyl-methylen]-5-amido-2-indolinon
Ausbeute: 32 % der Theorie,
$R_f$-Wert: 0,56 (Kieselgel; Methylenchlorid/Methanol = 9:1)
$C_{22}H_{16}N_4O_4$
Massenspektrum: m/z = 400 ($M^+$)

(22) 3-Z-[1-(4-Amido-phenylamino)-1-phenyl-methylen]-5-amido-2-indolinon
Ausbeute: 26 % der Theorie,
$R_f$-Wert: 0,47 (Kieselgel; Methylenchlorid/Methanol = 9:1)
$C_{23}H_{18}N_4O_3$
Massenspektrum: m/z = 398 ($M^+$)

(23) 3-Z-[1-(4-Pyridylamino)-1-phenyl-methylen]-5-amido-2-indolinon
Ausbeute: 15 % der Theorie,
$R_f$-Wert: 0,42 (Kieselgel; Methylenchlorid/Methanol = 9:1)
$C_{21}H_{16}N_4O_2$
Massenspektrum: m/z = 356 ($M^+$)

(24) 3-Z-[1-(4-Methyl-phenylamino)-1-phenyl-methylen]-5-amido-2-indolinon
Ausbeute: 45 % der Theorie,
$R_f$-Wert: 0,54 (Kieselgel; Methylenchlorid/Methanol = 9:1)
$C_{23}H_{19}N_3O_2$
Massenspektrum: m/z = 369 ($M^+$)

(25) 3-Z-[1-(4-Ethoxy-phenylamino)-1-phenyl-methylen]-5-amido-2-indolinon
Ausbeute: 40 % der Theorie,
$R_f$-Wert: 0,51 (Kieselgel; Methylenchlorid/Methanol = 9:1)
$C_{24}H_{21}N_3O_3$
Massenspektrum: m/z = 399 ($M^+$)

(26) 3-Z-[1-(3-Brom-phenylamino)-1-phenyl-methylen]-5-amido-2-indolinon
Ausbeute: 41 % der Theorie,
$R_f$-Wert: 0,53 (Kieselgel; Methylenchlorid/Methanol = 9:1) $C_{22}H_{16}BrN_3O_2$
Massenspektrum: m/z = 433/435 ($M^+$)

(27) 3-Z-[1-(4-Chlor-phenylamino)-1-phenyl-methylen]-5-amido-2-indolinon
Ausbeute: 50 % der Theorie,
$R_f$-Wert: 0,49 (Kieselgel; Methylenchlorid/Methanol = 9:1) $C_{22}H_{16}ClN_3O_2$
Massenspektrum: m/z = 389/391 ($M^+$)

(28) 3-Z-[1-(4-Isopropyl-phenylamino)-1-phenyl-methylen]-5-amido-2-indolinon
Ausbeute: 48 % der Theorie,

$R_f$-Wert: 0,65 (Kieselgel; Methylenchlorid/Methanol = 9:1)
$C_{25}H_{23}N_3O_2$
Massenspektrum: m/z = 397 ($M^+$)

(29) 3-Z-[1-(2-Fluorenylamino)-1-phenyl-methylen]-5-amido-2-indolinon
Ausbeute: 43 % der Theorie,
$R_f$-Wert: 0,58 (Kieselgel; Methylenchlorid/Methanol = 9:1)
$C_{29}H_{21}N_3O_2$
Massenspektrum: m/z = 443 ($M^+$)

(30) 3-Z-[1-(4-(2-Hydroxyethyl)-phenylamino)-1-phenyl-methylen]-5-amido-2-indolinon
Ausbeute: 22 % der Theorie,
$R_f$-Wert: 0,37 (Kieselgel; Methylenchlorid/Methanol = 9:1)
$C_{24}H_{21}N_3O_3$
Massenspektrum: m/z = 398 (M-H)

(31) 3-Z-[1-(4-(4-Imidazolyl)-phenylamino)-1-phenyl-methylen]-5-amido-2-indolinon
Ausbeute: 23 % der Theorie,
$R_f$-Wert: 0,5 (Kieselgel; Methylenchlorid/Methanol = 9:1)
$C_{25}H_{19}N_5O_2$
Massenspektrum: m/z = 421 ($M^+$)

(32) 3-Z-[1-(4-Ethoxycarbonylmethyl-phenylamino)-1-phenyl-methylen]-5-amido-2-indolinon
$C_{26}H_{23}N_3O_4$
Massenspektrum: m/z = 442 $(M+H)^+$

(33) 3-Z-[1-(4-Brom-3-methyl-phenylamino)-1-phenyl-methylen]-5-amido-2-indolinon
$C_{23}H_{18}BrN_3O_2$
Massenspektrum: m/z = 447/449 ($M^+$)

(34) 3-Z- [1- (4-Cyclohexyl-phenylamino) -1-phenyl-methylen]-5-amido-2-indolinon
$C_{28}H_{27}N_3O_2$
Massenspektrum: m/z = 437 ($M^+$)

(35) 3-Z-[1-(4-Brom-2-methyl-phenylamino)-1-phenyl-methylen]-5-amido-2-indolinon
$C_{23}H_{18}BrN_3O_2$
Massenspektrum: m/z = 447/449 (M+)

(36) 3-Z-[1-Amino-1-phenyl-methylen]-5-amido-2-indolinon
$R_f$-Wert: 0,3 (Kieselgel; Methylenchlorid/Methanol = 9:1)
$C_{16}H_{13}N_3O_2$
Massenspektrum: m/z = 279 ($M^+$)

(37) 3-Z-[1-Cyclohexylamino-1-phenyl-methylen]-5-amido-2-indolinon
$R_f$-Wert: 0,55 (Kieselgel; Methylenchlorid/Methanol = 9:1)
$C_{22}H_{23}N_3O_2$
Massenspektrum: m/z = 361 ($M^+$)

(38) 3-Z-[1-Cyclopentylamino-1-phenyl-methylen]-5-amido-2-indolinon
$R_f$-Wert: 0,53 (Kieselgel; Methylenchlorid/Methanol = 9:1)
$C_{21}H_{21}N_3O_2$
Massenspektrum: m/z = 347 ($M^+$)

(39) 3-Z-[1-Methylamino-1-phenyl-methylen]-5-amido-2-indolinon $R_f$-Wert: 0,5 (Kieselgel; Methylenchlorid/Methanol = 9:1)
$C_{17}H_{15}N_3O_2$
Massenspektrum: m/z = 293 ($M^+$)

(40) 3-Z-[1-Ethylamino-1-phenyl-methylen]-5-amido-2-indolinon $R_f$-Wert: 0,52 (Kieselgel; Methylenchlorid/Methanol = 9:1)
$C_{18}H_{17}N_3O_2$
Massenspektrum: m/z = 307 ($M^+$)

(41) 3-Z-[1-Isopropylamino-1-phenyl-methylen]-5-amido-2-indolinon
$R_f$-Wert: 0,44 (Kieselgel; Methylenchlorid/Methanol = 9:1)
$C_{19}H_{19}N_3O_2$
Massenspektrum: m/z = 321 (M+)

(42) 3-Z-[1-Dimethylamino-1-phenyl-methylen]-5-amido-2-indolinon
$R_f$-Wert: 0,39 (Kieselgel; Methylenchlorid/Methanol = 9:1)
$C_{18}H_{17}N_3O_2$
Massenspektrum: m/z = 307 ($M^+$)

(43) 3-Z-[1-Cyclopropylamino-1-phenyl-methylen]-5-amido-2-indolinon
$R_f$-Wert: 0,47 (Kieselgel; Methylenchlorid/Methanol = 9:1)
$C_{19}H_{17}N_3O_2$
Massenspektrum: m/z = 319 ($M^+$)

(44) 3-Z-[1-Cycloheptylamino-1-phenyl-methylen]-5-amido-2-indolinon
$R_f$-Wert: 0,58 (Kieselgel; Methylenchlorid/Methanol = 9:1)
$C_{23}H_{25}N_3O_2$
Massenspektrum: m/z = 375 ($M^+$)

(45) 3-Z-[1-Cyclobutylamino-1-phenyl-methylen]-5-amido-2-indolinon
$R_f$-Wert: 0,49 (Kieselgel; Methylenchlorid/Methanol = 9:1)
$C_{20}H_{19}N_3O_2$
Massenspektrum: m/z = 333 ($M^+$)

(46) 3-Z-[1-(4-Methylcyclohexylamino)-1-phenyl-methylen]-5-amido-2-indolinon
$R_f$-Wert: 0,67 (Kieselgel; Methylenchlorid/Methanol = 9:1)
$C_{23}H_{25}N_3O_2$
Massenspektrum: m/z = 375 ($M^+$)

(47) 3-Z-[1-(1-(R,S)-Indanylamino)-1-phenyl-methylen]-5-amido-2-indolinon
$R_f$-Wert: 0,59 (Kieselgel; Methylenchlorid/Methanol = 9:1)
$C_{25}H_{21}N_3O_2$
Massenspektrum: m/z = 395 ($M^+$)

(48) 3-Z-[1-(Methoxycarbonylmethylamino)-1-phenyl-methylen]-5-amido-2-indolinon
$R_f$-Wert: 0,46 (Kieselgel; Methylenchlorid/Methanol = 9:1)
$C_{19}H_{17}N_3O_4$
Massenspektrum: m/z = 351 ($M^+$)

(49) 3-Z-[1-((2-Methoxycarbonyl-ethyl)-amino)-1-phenyl-methylen] -5-amido-2-indolinon
$R_f$-Wert: 0,45 (Kieselgel; Methylenchlorid/Methanol = 9:1)
$C_{20}H_{19}N_3O_4$
Massenspektrum: m/z = 365 ($M^+$)

(50) 3-Z-[1-(4-Aminomethyl-phenylamino)-1-phenyl-methylen]-5-amido-2-indolinon
Ausbeute: 32 % der Theorie,
$R_f$-Wert: 0,46 (Kieselgel; Methylenchlorid/Methanol = 9:1)
$C_{23}H_{20}N_4O_2$
Massenspektrum: m/z = 384 ($M^+$)

(51) 3-Z-[1-(4-Pyrrolidinomethyl-phenylamino)-1-phenyl-methylen] -5-amido-2-indolinon-trifluoracetat
Ausbeute: 60 % der Theorie,

$R_f$-Wert: 0,07 (Kieselgel; Methylenchlorid/Methanol = 9:1)
$C_{27}H_{26}N_4O_2$
Massenspektrum: m/z = 438 ($M^+$)

(52) 3-Z-[1-(4-Morpholinomethyl-phenylamino)-1-phenyl-methylen] -5-amido-2-indolinon
Ausbeute: 65 % der Theorie,
$R_f$-Wert: 0,46 (Kieselgel; Methylenchlorid/Methanol = 9:1)
$C_{27}H_{26}N_4O_3$
Massenspektrum: m/z = 454 ($M^+$)

(53) 3-Z-[1-(4-Piperidinomethyl-phenylamino)-1-phenyl-methylen] -5-amido-2-indolinon-trifluoracetat
Ausbeute: 60 % der Theorie,
$R_f$-Wert: 0,08 (Kieselgel; Methylenchlorid/Methanol = 9:1)
$C_{28}H_{28}N_4O_2$
Massenspektrum: m/z = 452 ($M^+$)

(54) 3-Z-[1-(4-Hexamethyleniminomethyl-phenylamino)-1-phenylmethylen]-5-amido-2-indolinon-trifluoracetat
$C_{29}H_{30}N_4O_2$
Massenspektrum: m/z = 466 ($M^+$)

(55) 3-Z-[1-(4-(4-Hydoxy-piperidinomethyl)-phenylamino)-1-phenyl-methylen]-5-amido-2-indolinon
$C_{28}H_{28}N_4O_3$
Massenspektrum: m/z = 468 ($M^+$)

(56) 3-Z-[1-(4-(4-Methyl-piperidinomethyl)-phenylamino)-1-phenyl-methylen]-5-amido-2-indolinon
$C_{29}H_{30}N_4O_2$
Massenspektrum: m/z = 466 ($M^+$)

(57) 3-Z-[1-(4-(4-Ethyl-piperidinomethyl)-phenylamino)-1-phenyl-methylen]-5-amido-2-indolinon
$C_{30}H_{32}N_4O_2$
Massenspektrum: m/z = 480 ($M^+$)

(58) 3-Z-[1-(4-(4-Isopropyl-piperidinomethyl)-phenylamino)-1-phenyl-methylen]-5-amido-2-indolinon
$C_{31}H_{34}N_4O_2$
Massenspektrum: m/z = 494 ($M^+$)

(59) 3-Z-[1-(4-(4-Phenyl-piperidinomethyl)-phenylamino)-1-phenyl-methylen]-5-amido-2-indolinon
$C_{34}H_{32}N_4O_2$
Massenspektrum: m/z = 528 ($M^+$)

(60) 3-Z-[1-(4-(4-Benzyl-piperidinomethyl)-phenylamino)-1-phenyl-methylen]-5-amido-2-indolinon
$C_{35}H_{34}N_4O_2$
Massenspektrum: m/z = 542 ($M^+$)

(61) 3-Z-[1-(4-(4-Ethoxycarbonyl-piperidinomethyl)-phenylamino)-1-phenyl-methylen]-5-amido-2-indolinon
$C_{31}H_{32}N_4O_4$
Massenspektrum: m/z = 524 ($M^+$)

(62) 3-Z-[1-(4-Dimethylaminomethyl-phenylamino)-1-phenyl-methylen]-5-amido-2-indolinon
$C_{25}H_{24}N_4O_2$
Massenspektrum: m/z = 412 ($M^+$)

(63) 3-Z-[1-(4-Dipropylaminomethyl-phenylamino)-1-phenyl-methylen]-5-amido-2-indolinon
$C_{29}H_{32}N_4O_2$
Massenspektrum: m/z = 468 ($M^+$)

(64) 3-Z-[1-(4-Piperazinylmethyl-phenylamino)-1-phenyl-methylen] -5-amido-2-indolinon
$C_{27}H_{27}N_5O_2$

Massenspektrum: m/z = 453 ($M^+$)

(65) 3-Z-[1-(3-Dimethylaminomethyl-phenylamino)-1-phenyl-methylen]-5-amido-2-indolinon
$C_{25}H_{24}N_4O_2$
Massenspektrum: m/z = 412 ($M^+$)

(66) 3-Z-[1-(4-(2-Diethylamino-ethyl)-phenylamino)-1-phenyl-methylen]-5-amido-2-indolinon
$C_{28}H_{30}N_4O_2$
Massenspektrum: m/z = 454 ($M^+$)

(67) 3-Z-[1-(4-(2-Morpholino-ethyl)-phenylamino)-1-phenyl-methylen]-5-amido-2-indolinon
$C_{28}H_{28}N_4O_3$
Massenspektrum: m/z = 468 ($M^+$)

(68) 3-Z-[1-(4-(2-Pyrrolidinyl-ethyl)-phenylamino)-1-phenyl-methylen]-5-amido-2-indolinon
$C_{28}H_{28}N_4O_2$
Massenspektrum: m/z = 452 ($M^+$)

(69) 3-Z-[1-(4-(2-Piperidinyl-ethyl)-phenylamino)-1-phenyl-methylen]-5-amido-2-indolinon
$C_{29}H_{30}N_4O_2$
Massenspektrum: m/z = 466 ($M^+$)

(70) 3-Z-[1-(2-Thiazolylamino)-1-phenyl-methylen]-5-amido-2-indolinon
Ausbeute: 30 % der Theorie,
$R_f$-Wert: 0,48 (Kieselgel; Methylenchlorid/Methanol = 9:1)
$C_{19}H_{14}N_4O_2S$
Massenspektrum: m/z = 362 ($M^+$)

(71) 3-Z-[1-(Benzimidazol-2-ylamino)-1-phenyl-methylen]-5-amido-2-indolinon
Ausbeute: 29 % der Theorie,
$R_f$-Wert: 0,44 (Kieselgel; Methylenchlorid/Methanol = 9:1)
$C_{23}H_{17}N_5O_2$
Massenspektrum: m/z = 395 ($M^+$)

(72) 3-Z-[1-(5-Methyl-isoxazol-3-yl-amino)-1-phenyl-methylen]-5-amido-2-indolinon
Ausbeute: 39 % der Theorie,
$R_f$-Wert: 0,43 (Kieselgel; Methylenchlorid/Methanol = 9:1)
$C_{21}H_{18}N_4O_3$
Massenspektrum: m/z = 374 ($M^+$)

(73) 3-Z-[1-Benzylamino-1-phenyl-methylen]-5-amido-2-indolinon $R_f$-Wert: 0,63 (Kieselgel; Methylenchlorid/Methanol = 9:1)
$C_{23}H_{19}N_3O_2$
Massenspektrum: m/z = 369 ($M^+$)

(74) 3-Z-[1-(4-(1-Imidazolyl-methyl)-phenylamino)-1-phenylmethylen]-5-amido-2-indolinon
$R_f$-Wert: 0,45 (Kieselgel; Methylenchlorid/Methanol = 9:1)
$C_{26}H_{21}N_5O_2$
Massenspektrum: m/z = 436 $(M+H)^+$

(75) 3-Z-[1-(4-((2-Diethylamino-ethyl)-aminocarbonyl)-phenylamino)-1-phenyl-methylen]-5-amido-2-indolinon-trifluoracetat Ausbeute: 27 % der Theorie,
$R_f$-Wert: 0,05 (Kieselgel; Methylenchlorid/Methanol = 9:1)
$C_{29}H_{31}N_5O_3$
Massenspektrum: m/z = 497 ($M^+$)

(76) 3-Z-[1-(4-Acetylaminomethyl-phenylamino)-1-phenyl-methylen] -5-amido-2-indolinon
$R_f$-Wert: 0,4 (Kieselgel; Methylenchlorid/Methanol = 9:1)

$C_{25}H_{22}N_4O_3$
Massenspektrum: m/z = 426 ($M^+$)

(77) 3-Z-[1-(4-((2-Dimethylaminoethyl)-N-methansulfonyl-amino)-phenylamino)-1-phenyl-methylen]-5-amido-2-indolinon
$R_f$-Wert: 0,1 (Kieselgel; Methylenchlorid/Methanol = 9:1)
$C_{27}H_{29}N_5O_4S$
Massenspektrum: m/z = 519 ($M^+$)

(78) 3-Z-[1-(4-(N-(Ethoxycarbonylmethyl)-N-methansulfonyl-amino)-phenylamino)-1-phenyl-methylen]-5-amido-2-indolinon
$R_f$-Wert: 0,57 (Kieselgel; Methylenchlorid/Methanol = 9:1)
$C_{27}H_{26}N_4O_6$
Massenspektrum: m/z = 534 ($M^+$)

(79) 3-Z-[1-(4-(N-(Cyanomethyl)-N-methansulfonyl-amino)-phenylamino)-1-phenyl-methylen]-5-amido-2-indolinon
$R_f$-Wert: 0,49 (Kieselgel; Methylenchlorid/Methanol = 9:1)
$C_{25}H_{21}N_5O_4S$
Massenspektrum: m/z = 487 ($M^+$)

(80) 3-Z-[1-(4-(N-Methyl-N-methansulfonyl-amino)-phenylamino)-1-phenyl-methylen]-5-amido-2-indolinon
$R_f$-Wert: 0,46 (Kieselgel; Methylenchlorid/Methanol = 9:1)
$C_{24}H_{22}N_4O_4S$
Massenspektrum: m/z = 462 ($M^+$)

(81) 3-Z-[1-(4-(2-Oxo-pyrrolidin-1-yl-methyl)-phenylamino)-1-phenyl-methylen]-5-amido-2-indolinon
$C_{27}H_{24}N_4O_3$
Massenspektrum: m/z = 452 ($M^+$)

(82) 3-Z-[1-(4-(2-Oxo-piperidin-1-yl-methyl)-phenylamino)-1-phenyl-methylen]-5-amido-2-indolinon
$C_{28}H_{26}N_4O_3$
Massenspektrum: m/z = 466 ($M^+$)

(83) 3-Z-[1-(4-(4-Cyclohexyl-piperidino-methyl)-phenylamino)-1-phenyl-methylen]-5-amido-2-indolinon-trifluoracetat
$C_{34}H_{38}N_4O_2$
Massenspektrum: m/z = 534 ($M^+$)

(84) 3-Z-[1-(4-(2,6-Dimethyl-piperidino-methyl)-phenylamino)-1-phenyl-methylen]-5-amido-2-indolinon-trifluoracetat
$C_{30}H_{32}N_4O_2$
Massenspektrum: m/z: 480 ($M^+$)

(85) 3-Z-[1-(4-(4-Phenyl-4-hydroxy-piperidino-methyl)-phenylamino)-1-phenyl-methylen]-5-amido-2-indolinon-trifluoracetat
$C_{34}H_{32}N_4O_3$
Massenspektrum: m/z = 545 ($M^+$)
$R_f$-Wert: 0,66 (Kieselgel, Methylenchlorid/Methanol = 4:1)

(86) 3-Z-[1-(4-(2-Methoxycarbonyl-pyrrolidino-methyl)-phenylamino)-1-phenyl-methylen]-5-amido-2-indolinon-trifluoracetat
$C_{29}H_{28}N_4O_4$
Massenspektrum: m/z = 497 $(M+H)^+$
$R_f$-Wert: 0,65 (Kieselgel, Methylenchlorid/Methanol = 4:1)

(87) 3-Z-[1-(4-(1-Oxo-thiomorpholin-4-ylmethyl)-phenylamino)-1-phenyl-methylen]-5-amido-2-indolinon-trifluoracetat

$C_{27}H_{26}N_4O_3S$
Massenspektrum: m/z = 487 $(M+H)^+$
$R_f$-Wert: 0,68 (Kieselgel, Methylenchlorid/Methanol = 4:1)

(88) 3-Z-[1-(4-( 3,6-Dihydro-2H-pyridin-1-ylmethyl)-phenylamino)-1-phenyl-methylen]-5-amido-2-indolinon-trifluoracetat
$C_{28}H_{26}N_4O_2$
Massenspektrum: m/z = 451 $(M+H)^+$

(89) 3-Z-[1-(4-(2,5-Dihydro-pyrrol-1-ylmethyl)-phenylamino)-1-phenyl-methylen]-5-amido-2-indolinon-trifluoracetat
$C_{27}H_{24}N_4O_2$
Massenspektrum: m/z = 437 $(M+H)^+$
$R_f$-Wert: 0,49 (Kieselgel, Methylenchlorid/Methanol = 4:1)

(90) 3-Z-[1-(4-(Thiomorpholin-4-ylmethyl)-phenylamino)-1-phenyl-methylen]-5-amido-2-indolinon-trifluoracetat
$C_{27}H_{26}N_4O_2S$
Massenspektrum: m/z = 471 $(M+H)^+$
$R_f$-Wert: 0,78 (Kieselgel, Methylenchlorid/Methanol = 4:1)

(91) 3-Z-[1-(4-(6,7-Dimethoxy-tetrahydroisochinolin-2-ylmethyl)-phenylamino)-1-phenyl-methylen] -5-amido-2-indolinontrifluoracetat
$C_{34}H_{32}N_4O_4$
Massenspektrum: m/z = 561 $(M+H)^+$
$R_f$-Wert: 0,8 (Kieselgel, Methylenchlorid/Methanol = 4:1)

(92) 3-Z-[1-(4-(4-Phenyl-piperazin-1-ylmethyl))-phenylamino)-1-phenyl-methylen]-5-amido-2-indolinon-trifluoracetat
$C_{33}H_{31}N_5O_2$
Massenspektrum: m/z = 530 $(M+H)^+$
$R_f$-Wert: 0,78 (Kieselgel, Methylenchlorid/Methanol = 4:1)

(93) 3-Z-[1-(4-(3,5-Dimethyl-piperidino-methyl)-phenylamino)-1-phenyl-methylen]-5-amido-2-indolinon-trifluoracetat
$C_{30}H_{32}N_4O_2$
Massenspektrum: m/z = 480 (M+)
$R_f$-Wert: 0,54 (Kieselgel, Methylenchlorid/Methanol = 4:1)

(94) 3-Z-[1-(4-(N-Methyl-N-benzyl-amino-methyl)-phenylamino)-1-phenyl-methylen]-5-amido-2-indolinon-trifluoracetat
$C_{31}H_{29}N_4O_4$
Massenspektrum: m/z = 488 $(M^+)$

(95) 3-Z-[1-(3,4-Dimethoxy-phenylamino)-1-phenyl-methylen]-5-amido-2-indolinon
$C_{24}H_{21}N_3O_4$
Massenspektrum: m/z = 415 $(M^+)$
$R_f$-Wert: 0,5 (Kieselgel, Methylenchlorid/Methanol = 9:1)

(96) 3-Z-[1-(4-Trifluormethoxy-phenylamino)-1-phenyl-methylen]-5-amido-2-indolinon
$C_{23}H_{16}F_3N_3O_3$
Massenspektrum: m/z = 439 $(M^+)$
$R_f$-Wert: 0,5 (Kieselgel, Methylenchlorid/Methanol = 9:1)

(97) 3-Z-[1-(3-Ethoxycarbonyl-phenylamino)-1-phenyl-methylen]-5-amido-2-indolinon
$C_{25}H_{21}N_3O_4$
Massenspektrum: m/z = 427 $(M^+)$
$R_f$-Wert: 0,52 (Kieselgel, Methylenchlorid/Methanol = 9:1)

(98) 3-Z-[1-(3-Carboxy-phenylamino)-1-phenyl-methylen]-5-amido-2-indolinon
$C_{23}H_{17}N_3O_4$
Massenspektrum: m/z = 399 ($M^+$)
Rf-Wert: 0,14 (Kieselgel, Methylenchlorid/Methanol = 9:1)

(99) 3-Z-[1-(3-Diethylcarbamoyl-phenylamino)-1-phenyl-methylen]-5-amido-2-indolinon
$C_{27}H_{26}N_4O_3$
Massenspektrum: m/z = 454 ($M^+$)
$R_f$-Wert: 0,48 (Kieselgel, Methylenchlorid/Methanol = 9:1)

(100) 3-Z-[1-(3-Ethylcarbamoyl-phenylamino)-1-phenyl-methylen]-5-amido-2-indolinon
$C_{25}H_{22}N_4O_3$
Massenspektrum: m/z = 426 ($M^+$)
$R_f$-Wert: 0,42 (Kieselgel, Methylenchlorid/Methanol = 9:1)

(101) 3-Z-[1-(3-Trifluormethoxy-phenylamino)-1-phenyl-methylen] -5-amido-2-indolinon
$C_{23}H_{16}F_3N_3O_3$
Massenspektrum: m/z = 439 ($M^+$)
$R_f$-Wert: 0,5 (Kieselgel, Methylenchlorid/Methanol = 9:1)

(102) 3-Z-[1-(3-Ethoxy-phenylamino)-1-phenyl-methylen]-5-amido-2-indolinon
$C_{24}H_{21}N_3O_3$
Massenspektrum: m/z = 399 ($M^+$)
$R_f$-Wert: 0,49 (Kieselgel, Methylenchlorid/Methanol = 9:1)

(103) 3-Z-[1-(4-Methoxymethyl-phenylamino)-1-phenyl-methylen]-5-amido-2-indolinon
$C_{24}H_{21}N_3O_3$
Massenspektrum: m/z = 399 ($M^+$)
$R_f$-Wert: 0,4 (Kieselgel, Methylenchlorid/Methanol = 4:1)

(104) 3-Z-[1-(4-Ethyl-phenylamino)-1-phenyl-methylen]-5-amido-2-indolinon
$C_{24}H_{21}N_3O_2$
Massenspektrum: m/z = 383 ($M^+$)
$R_f$-Wert: 0,52 (Kieselgel, Methylenchlorid/Methanol = 4:1)

(105) 3-Z- [1-(4-Methyl-3-nitro-phenylamino)-1-phenyl-methylen]-5-amido-2-indolinon
$C_{23}H_{18}N_4O_4$
Massenspektrum: m/z = 414 ($M^+$)

(106) 3-Z-[1-(4-Methyl-3-methoxy-phenylamino)-1-phenyl-methylen] -5-amido-2-indolinon
$C_{24}H_{21}N_3O_3$
Massenspektrum: m/z = 399 ($M^+$)

(107) 3-Z-[1-(4-(4-Aminophenyl-methyl)-phenylamino)-1-phenylmethylen]-5-amido-2-indolinon-trifluoracetat
$C_{29}H_{24}N_4O_2$
Massenspektrum: m/z = 460 ($M^+$)

(108) 3-Z- [1-(4-Methoxycarbonyl-3-methyl-phenylamino) -1-phenylmethylen]-5-amido-2-indolinon
$C_{25}H_{21}N_3O_4$
Massenspektrum: m/z = 427 ($M^+$)
$R_f$-Wert: 0,56 (Kieselgel, Methylenchlorid/Methanol = 4:1)

(109) 3-Z-[1-(4-Cyanophenylamino)-1-phenyl-methylen]-5-amido-2-indolinon
$C_{23}H_{16}N_4O_2$
Massenspektrum: m/z = 380 ($M^+$)
$R_f$-Wert: 0,65 (Kieselgel, Methylenchlorid/Methanol = 9:1)

(110) 3-Z-[1-(5-Methyl-pyridin-2-yl-amino) -1-phenyl-methylen]-5-amido-2-indolinon

$R_f$-Wert: 0,6 (Kieselgel, Methylenchlorid/Methanol = 9:1)
$C_{22}H_{18}N_4O_2$
Massenspektrum: m/z = 370 ($M^+$)

(111) 3-Z-[1-(5-Brom-pyridin-2-yl-amino)-1-phenyl-methylen]-5-amido-2-indolinon
$R_f$-Wert: 0,65 (Kieselgel, Methylenchlorid/Methanol = 9:1)
$C_{21}H_{15}BrN_4O_2$
Massenspektrum: m/z = 434/436 ($M^+$)

(112) 3-Z-[1-(2-Chlor-pyridin-5-yl-amino)-1-phenyl-methylen]-5-amido-2-indolinon
$R_f$-Wert: 0,49 (Kieselgel, Methylenchlorid/Methanol = 9:1)
$C_{21}H_{15}ClN_4O_2$
Massenspektrum: m/z = 390/392 ($M^+$)

(113) 3-Z-[1-(3-Cyanophenylamino)-1-phenyl-methylen]-5-amido-2-indolinon
$C_{23}H_{16}N_4O_2$
Massenspektrum: m/z = 380 ($M^+$)
$R_f$-Wert: 0,57 (Kieselgel, Methylenchlorid/Methanol = 9:1)

(114) 3-Z-[1-(4-(N-Phenyl-amino-methyl)-phenylamino)-1-phenylmethylen] -5-amido-2-indolinon
$C_{29}H_{24}N_4O_2$
Massenspektrum: m/z = 460 ($M^+$)
$R_f$-Wert: 0,74 (Kieselgel, Methylenchlorid/Methanol = 9:1)

(115) 3-Z-[1-(4-(N-Methyl-N-phenyl-aminomethyl)-phenylamino)-1-phenyl-methylen]-5-amido-2-indolinon
$C_{30}H_{26}N_4O_2$
Massenspektrum: m/z = 474 ($M^+$)
$R_f$-Wert: 0,75 (Kieselgel, Methylenchlorid/Methanol = 9:1)

(116) 3-Z-[1-(4-(N-Ethyl-aminomethyl)-phenylamino)-1-phenylmethylen] -5-amido-2-indolinon-trifluoracetat
$C_{25}H_{24}N_4O_2$
Massenspektrum: m/z = 412 ($M^+$)

(117) 3-Z-[1-(4-(N-(4-Chlorphenyl-methyl)-aminomethyl)-phenylamino)-1-phenyl-methylen]-5-amido-2-indolinon-trifluoracetat
$C_{30}H_{25}ClN_4O_2$
Massenspektrum: m/z = 508/510 ($M^+$)

(118) 3-Z-[1-(4-(N-Cyclohexyl-aminomethyl)-phenylamino)-1-phenyl-methylen]-5-amido-2-indolinon-trifluoracetat
$C_{29}H_{30}N_4O_2$
Massenspektrum: m/z = 466 ($M^+$)

(119) 3-Z-[1-(4-(N-Isopropyl-aminomethyl)-phenylamino)-1-phenyl-methylen]-5-amido-2-indolinon-trifluoracetat
$C_{26}H_{26}N_4O_2$
Massenspektrum: m/z = 426 ($M^+$)

(120) 3-2-[1-(4-(N-Butyl-aminomethyl)-phenylamino)-1-phenylmethylen]-5-amido-2-indolinon-trifluoracetat
$C_{27}H_{28}N_4O_2$
Massenspektrum: m/z = 440 ($M^+$)

(121) 3-Z-[1-(4-(N-Methoxycarbonyl-methylamino-methyl)-phenylamino)-1-phenyl-methylen]-5-amido-2-indolinon-trifluoracetat
$C_{26}H_{24}N_4O_4$
Massenspektrum: m/z = 456 ($M^+$)

(122) 3-Z-[1-(4-(N-(Phenyl-methyl)-aminomethyl)-phenylamino)-1-phenyl-methylen]-5-amido-2-indolinon-trifluoracetat
$C_{30}H_{26}N_4O_2$

Massenspektrum: m/z = 464 ($M^+$)

(123) 3-Z-[1-(4-(N-Acetyl-N-ethoxycarbonylmethyl-amino)-phenylamino)-1-phenyl-methylen]-5-amido-2-indolinon
$C_{28}H_{26}N_4O_5$
Massenspektrum: m/z = 498 ($M^+$)

(124) 3-Z-[1-(4-Methyl-3-sulfamoyl-phenylamino)-1-phenyl-methylen]-5-amido-2-indolinon
$C_{23}H_{20}N_4O_4S$
Massenspektrum: m/z = 448 ($M^+$)

(125) 3-Z-[1-(4-(N-Methansulfonyl-N-(methylcarbamoylmethyl)-amino)-phenylamino)-1-phenyl-methylen]-5-amido-2-indolinon
$C_{26}H_{25}N_5O_5S$
Massenspektrum: m/z = 519 ($M^+$)

(126) 3-Z-[1-(4-(N-Methansulfonyl-N-(piperidin-carbonyl-methyl)amino]-phenylamino)-1-phenyl-methylen]-5-amido-2-indolinon
$C_{30}H_{31}N_5O_5S$
Massenspektrum: m/z = 573 ($M^+$)

(127) 3-Z-[1-(4-Carboxy-phenylamino)-1-phenyl-methylen]-5-amido-2-indolinon
$C_{23}H_{17}N_3O_4$
Massenspektrum: m/z = 398 ($M-H^+$)

(128) 3-Z-[1-(4-Carboxy-3-methyl-phenylamino)-1-phenyl-methylen]-5-amido-2-indolinon
$C_{24}H_{19}N_3O_4$
Massenspektrum: m/z = 412 ($M-H^+$)

(129) 3-Z-[1-(4-(3-Diethylamino-propoxy)-phenylamino)-1-phenylmethylen] -5-amido-2-indolinon-trifluoracetat
$C_{29}H_{32}N_4O_3$
Massenspektrum: m/z = 484 ($M^+$)

(130) 3-Z-[1-(4-(2-Piperidino-ethoxy)-phenylamino)-1-phenyl-methylen]-5-amido-2-indolinon-trifluoracetat
$C_{29}H_{30}N_4O_3$
Massenspektrum: m/z = 483 $(M+H)^+$

(131) 3-Z-[1-(4-(3-Piperidino-propoxy)-phenylamino)-1-phenylmethylen]-5-amido-2-indolinon-trifluoracetat
$C_{30}H_{32}N_4O_3$
Massenspektrum: m/z = 496 ($M^+$)

(132) 3-Z-[1-(4-(3-Dimethylamino-propoxy)-phenylamino)-1-phenyl-methylen]-5-amido-2-indolinon-trifluoracetat
$C_{27}H_{28}N_4O_3$
Massenspektrum: m/z = 457 $(M+H)^+$

(133) 3-Z-[1-(4-(3-N-Methyl-N-benzylamino-propoxy)-phenylamino)-1-phenyl-methylen]-5-amido-2-indolinon-trifluoracetat
$C_{33}H_{32}N_4O_3$
Massenspektrum: m/z = 533 $(M+H)^+$

(134) 3-Z-[1-(4-(2-Dimethylamino-ethoxy)-phenylamino)-1-phenylmethylen]-5-amido-2-indolinon-trifluoracetat
$C_{26}H_{26}N_4O_3$
Massenspektrum: m/z = 443 $(M+H)^+$

(135) 3-Z- [1-(4- (N-Ethyl-N-benzyl-aminomethyl)-phenylamino)-1-phenyl-methylen]-5-amido-2-indolinon

(136) 3-Z-[1-(4-(N-Propyl-N-benzyl-aminomethyl)-phenylamino)-1-phenyl-methylen]-5-amido-2-indolinon

(137) 3-Z-[1-(4-(N-Methyl-N-(4-chlorphenyl-methyl)-aminomethyl)-phenylamino)-1-phenyl-methylen]-5-amido-2-indolinon

(138) 3-Z-[1-(4-(N-Methyl-N-(4-bromphenyl-methyl)-amino-methyl)-phenylamino)-1-phenyl-methylen]-5-amido-2-indolinon

(139) 3-Z-[1-(4-(N-Methyl-N-(3-chlorphenyl-methyl)-amino-methyl)-phenylamino)-1-phenyl-methylen]-5-amido-2-indolinon

(140) 3-Z-[1-(4-(N-Methyl-N-(3,4-dimethoxyphenyl-methyl)-aminomethyl)-phenylamino)-1-phenyl-methylen]-5-amido-2-indolinon

(141) 3-Z-[1-(4-(N-Methyl-N-(4-methoxyphenyl-methyl)-aminomethyl)-phenylamino)-1-phenyl-methylen]-5-amido-2-indolinon

(142) 3-Z-[1-(4-(N-Trifluorethyl-N-(phenyl-methyl)-amino-methyl)-phenylamino)-1-phenyl-methylen]-5-amido-2-indolinon

(143) 3-Z-[1-(4-(N-Trifluorethyl-N-(4-chlorphenyl-methyl)-aminomethyl)-phenylamino)-1-phenyl-methylen]-5-amido-2-indolinon

Beispiel 5

3-Z-[1-(4-(4-Acetyl-piperazinylmethyl)-phenylamino)-1-phenylmethylen]-5-amido-2-indolinon

25 mg 3-Z-[1-(4-(Piperazinylmethyl)-phenylamino)-1-phenyl-methylen]-5-amido-2-indolinon und 0,02 g Triethylamin werden in 10 ml Methylenchlorid gelöst und mit 5 mg Acetylchlorid versetzt und die Lösung 16 Stunden bei Raumtemperatur gerührt. Anschließend wird mit Wasser gewaschen und dann die organische Phase einrotiert.
Ausbeute: 15 mg g (68 % der Theorie),
$C_{29}H_{29}N_5O_3$
Massenspektrum: m/z = 495 ($M^+$)
Analog wird folgende Verbindung hergestellt:

(1) 3-Z-[1-(4-(4-Benzoyl-piperazinylmethyl)-phenylamino)-1-phenyl-methylen] -5-amido-2-indolinon
Hergestellt aus 3-Z-[1-(4-(Piperazinyl-methyl-phenylamino)-1-phenyl-methylen]-5-amido-2-indolinon und Benzoylchlorid. Ausbeute: 91 % der Theorie,
$C_{34}H_{31}N_5O_3$
Massenspektrum: m/z = 557 ($M^+$)

Beispiel 6

3-Z-[1-(4-Diethylcarbamoyl-phenylamino-1-phenyl-methylen]-5-amido-2-indolinon

7 g Harz aus Stufe IV werden analog Beispiel 4 mit 4-Aminobenzoesäureethylester umgesetzt. Das feuchte beladene Harz wird in 30 ml Dioxan und 30 ml Methanol suspendiert und mit 25 ml 1 N Natronlauge 40 Stunden gerührt. Anschließend wird mit verdünnter Salzsäure neutralisiert und mit Methylenchlorid, Methanol und Dimethylformamid gewaschen. Anschließend werden 300 mg des Harzes in 3 ml Dimethylformamid suspendiert, mit 0,2 ml Diethylamin, 0,5 g O-(Benzotriazol-1-yl)-N,N,N',N'-tetramethyluronium-tetrafluorborat und 0,8 ml Ethyldiisopropylamin 60 Stunden bei Raumtemperatur stehen gelassen. Schließlich spaltet man das Produkt wie in Beispiel 4 beschrieben vom Harz.
Ausbeute: 29 mg,
$R_f$-Wert: 0,46 (Kieselgel, Methylenchlorid/Methanol = 9:1)
$C_{27}H_{26}N_4O_3$
Massenspektrum: m/z = 454 ($M^+$)
Analog werden hergestellt:

(1) 3-Z-[1-(4-(Piperidinocarbonyl)-phenylamino)-1-phenylmethylen]-5-amido-2-indolinon
$R_f$-Wert: 0,43 (Kieselgel, Methylenchlorid/Methanol = 9:1)
$C_{28}H_{26}N_4O_3$

Massenspektrum: m/z = 466 ($M^+$)

(2) 3-Z-[1-(4-(4-Methylpiperazinocarbonyl)-phenylamino)-1-phenyl-methylen]-5-amido-2-indolinon-trifluoracetat
$R_f$-Wert: 0,84 (Kieselgel, Methylenchlorid/Methanol = 4:1)
$C_{28}H_{27}N_5O_3$
Massenspektrum: m/z = 481 ($M^+$)

(3) 3-Z-[1-(4- (N- (2-Dimethylamino-ethyl) -N-methyl-carbamoyl)-phenylamino)-1-phenyl-methylen]-5-amido-2-indolinon-trifluoracetat
$R_f$-Wert: 0,25 (Kieselgel, Methylenchlorid/Methanol = 9:1)
$C_{28}H_{29}N_5O_3$
Massenspektrum: m/z = 484 $(M+H)^+$

(4) 3-Z-[1-(4-(N-Methoxycarbonylmethyl-carbamoyl)-phenylamino)-1-phenyl-methylen]-5-amido-2-indolinon
Rf-Wert: 0,4 (Kieselgel, Methylenchlorid/Methanol = 9:1)
$C_{26}H_{22}N_4O_5$
Massenspektrum: m/z = 470 ($M^+$)

(5) 3-Z-[1-(4-Benzylcarbamoyl-phenylamino)-1-phenyl-methylen]-5-amido-2-indolinon
$R_f$-Wert: 0,48 (Kieselgel, Methylenchlorid/Methanol = 9:1)
$C_{30}H_{24}N_4O_3$
Massenspektrum: m/z = 488 ($M^+$)

Beispiel 7

3-Z-[1-(4-(N-Methyl-benzoylamino)-phenylamino)-1-phenyl-methylen]-5-amido-2-indolinon

4,5 g Harz aus Stufe IV werden analog Beispiel 4 mit 3,4 g 4-(9H-Fluoren-9-ylmethoxycarbonyl)-methyl-amino)-anilin in Dimethylformamid umgesetzt. Anschließend wird die 9H-Fluorenschutzgruppe mit 4 ml 30%igem Piperidin in Dimethylformamid abgespalten und das Harz mehrfach gewaschen. Anschließend werden 400 mg des Harzes in 4 ml Dimethylformamid und 0,3 ml Triethylamin suspendiert und mit 0,3 ml Benzoylchlorid eine Stunde bei Raumtemperatur umgesetzt. Schließlich spaltet man das Produkt wie in Beispiel 4 beschrieben vom Harz.
Ausbeute: 33 mg.
$R_f$-Wert: 0,45 (Kieselgel, Methylenchlorid/Methanol = 9:1)
$C_{30}H_{24}N_4O_3$
Massenspektrum: m/z = 488 ($M^+$)
Analog werden hergestellt:

(1) 3-Z-[1-(4-(N-Methyl-propionylamino)-phenylamino)-1-phenylmethylen] -5-amido-2-indolinon
$R_f$-Wert: 0,42 (Kieselgel, Methylenchlorid/Methanol = 9:1)
$C_{26}H_{24}N_4O_3$
Massenspektrum: m/z = 440 ($M^+$)

(2) 3-Z-[1-(4-(N-Methyl-butyrylamino)-phenylamino)-1-phenyl-methylen]-5-amido-2-indolinon
$R_f$-Wert: 0,44 (Kieselgel, Methylenchlorid/Methanol = 9:1)
$C_{27}H_{26}N_4O_3$
Massenspektrum: m/z = 453 ($M-H^+$)

(3) 3-Z-[1-(4-(N-Methyl-ethylsulfonylamino)-phenylamino)-1-phenyl-methylen]-5-amido-2-indolinon
$R_f$-Wert: 0,42 (Kieselgel, Methylenchlorid/Methanol = 9:1)
$C_{25}H_{24}N_4O_4S$
Massenspektrum: m/z = 475 ($M-H^+$)

(4) 3-Z-[1-(4-(N-Methyl-propylsulfonylamino)-phenylamino)-1-phenyl-methylen]-5-amido-2-indolinon
$R_f$-Wert: 0,44 (Kieselgel, Methylenchlorid/Methanol = 9:1)
$C_{25}H_{26}N_4O_4S$
Massenspektrum: m/z = 491 $(M+H)^+$

(5) 3-Z-[1-(4-(N-Methyl-phenylsulfonylamino)-phenylamino)-1-phenyl-methylen]-5-amido-2-indolinon
$R_f$-Wert: 0,53 (Kieselgel, Methylenchlorid/Methanol = 9:1)
$C_{29}H_{24}N_4O_4S$
Massenspektrum: m/z = 524 ($M^+$)

Beispiel 8

| Trockenampulle mit 75 mg Wirkstoff pro 10 ml | |
|---|---|
| Zusammensetzung: | |
| Wirkstoff | 75,0 mg |
| Mannitol | 50,0 mg |
| Wasser für Injektionszwecke | ad 10,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 9

| Trockenampulle mit 35 mg Wirkstoff pro 2 ml | |
|---|---|
| Zusammensetzung: | |
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 10

| Tablette mit 50 mg Wirkstoff | |
|---|---|
| Zusammensetzung: | |
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreBt, biplan mit beidseitiger Facette und einseitiger Teilkerbe.

Durchmesser der Tabletten: 9 mm.

Beispiel 11

| Tablette mit 350 mg Wirkstoff | |
|---|---|
| Zusammensetzung: | |
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 12 mm.

Beispiel 12

| Kapseln mit 50 mg Wirkstoff | |
|---|---|
| Zusammensetzung: | |
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

Beispiel 13

| Kapseln mit 350 mg Wirkstoff | |
|---|---|
| Zusammensetzung: | |
| (1) Wirkstoff | 350,0 mg |
| (2) Maisstärke getrocknet | 46,0 mg |
| (3) Milchzucker pulverisiert | 30,0 mg |
| (4) Magnesiumstearat | 4, 0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Gr6Be 0 abgefüllt.

Beispiel 14

| Suppositorien mit 100 mg Wirkstoff | | |
|---|---|---|
| 1 | Zäpfchen enthält: | |
| | Wirkstoff | 100,0 mg |
| | Polyethylenglykol (M.G. 1500) | 600,0 mg |
| | Polyethylenglykol (M.G. 6000) | 460,0 mg |
| | Polyethylensorbitanmonostearat | 840,0 mg |
| | | 2 000,0 mg |

Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. Substituierte Indolinone der allgemeinen Formel

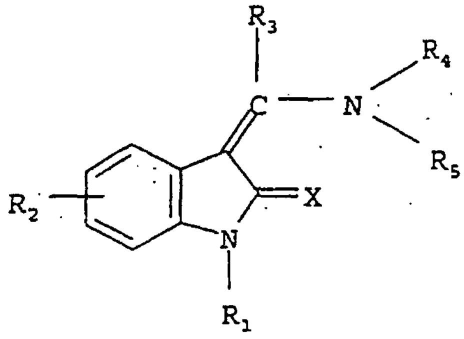

(I),

in der
X ein Sauerstoff- oder Schwefelatom,
$R_1$ ein Wasserstoffatom, eine $C_{1-4}$-Alkoxy-carbonyl- oder $C_{2-4}$-Alkanoylgruppe,
$R_2$ eine Carboxy-, $C_{1-4}$-Alkoxy-carbonyl- oder Aminocarbonylgruppe, in der der Aminoteil durch eine'oder zwei $C_{1-3}$-Alkylgruppen substituiert sein kann und die Substituenten gleich oder verschieden sein können,
$R_3$ eine Phenyl- oder Naphthylgruppe, die durch Fluor-, Chloroder Bromatome, durch $C_{1-3}$-Alkyl-, $C_{1-3}$-Alkoxy-, Cyano-, Trifluormethyl-, Nitro-, Amino-, $C_{1-3}$-Alkylamino-, Di-($C_{1-3}$-alkyl)-amino-, $C_{2-4}$-Alkanoyl-amino-, N-($C_{1-3}$-Alkyl)-$C_{2-4}$-alkanoylamino-, $C_{1-3}$-Alkylsulfonylamino-, Amino-$C_{1-3}$-alkyl-, $C_{1-3}$-Alkylamino-$C_{1-3}$-alkyl-, Di- ($C_{1-3}$-Alkyl)-amino-$C_{1-3}$-alkyl-, N-($C_{2-4}$-Alkanoyl)-amino-$C_{1-3}$-alkyl- oder N- ($C_{2-4}$-Alkanoyl)-$C_{1-3}$-alkylamino-$C_{1-3}$-alkylgruppen substituiert und die Substituenten gleich oder verschieden sein können,
$R_4$ ein Wasserstoffatom oder eine $C_{1-3}$-Alkylgruppe und
$R_5$ ein Wasserstoffatom,
eine gegebenenfalls durch eine Phenyl-, Carboxy- oder $C_{1-3}$-Alkoxy-carbonylgruppe substituierte $C_{1-5}$-Alkylgruppe,

eine gegebenenfalls durch eine $C_{1-3}$-Alkylgruppe substitierte $C_{3-7}$-Cycloalkylgruppe,
eine gegebenenfalls durch eine $C_{1-3}$-Alkylgruppe substituierte Indanylgruppe,
eine 5-gliedrige Heteroarylgruppe, die eine gegebenenfalls durch eine $C_{1-3}$-Alkylgruppe substituierte Iminogruppe, ein Sauerstoff- oder Schwefelatom oder eine gegebenenfalls durch eine $C_{1-3}$-Alkylgruppe substituierte Iminogruppe und ein Sauerstoff-, Schwefel- oder Stickstoffatom oder zwei Stickstoffatome enthält oder eine 6-gliedrige Hereroarylgruppe, die 1 bis 3 Stickstoffatome enthält, wobei über zwei benachbarte Kohlenstoffatome oder über ein Kohlenstoffatom und eine benachbarte Iminogruppe der vorstehend erwähnten 5- und 6-gliedrigen Heteroarylgruppen zusätzlich eine 1,3-Butadienylenbrücke angefügt sein kann und das Kohlenstoffgerüst der vorstehend erwähnten mono- und bicyclischen Ringe durch Fluor-, Chlor-, Brom- oder Jodatome, durch $C_{1-5}$-Alkyl- oder Cyanogruppen mono- oder disubstituiert und die Substituenten gleich oder verschieden sein können,
eine über ein Kohlenstoffatom verknüpfte Pyrrolidinyl- oder Piperidinylgruppe, die jeweils am Stickstoffatom durch eine $C_{1-3}$-Alkylgruppe substituiert sein kann,
eine gegebenenfalls durch Fluor-, Chlor-, Brom- oder Jodatome, durch $C_{1-5}$-Alkyl-, $C_{1-3}$-Alkoxy-, Carboxy-, $C_{1-3}$-Alkoxycarbonyl-, Aminosulfonyl-, Nitro- oder Cyanogruppen disubstituierte Phenylgruppe, wobei die Substituenten gleich oder verschieden sein können,
eine Phenyl-, Pyridyl-, Pyrimidyl- oder Thienylgruppe, die jeweils

durch eine Trifluormethoxygruppe, durch ein ein Fluor-, Chlor-, Brom- oder Jodatom,

durch eine $C_{1-3}$-Alkoxygruppe, die in 2- oder 3-Stellung durch eine Amino-, $C_{1-3}$-Alkylamino-, Di-($C_{1-3}$-Alkyl)-amino-, Phenyl-$C_{1-3}$-alkylamino-, N- ($C_{1-3}$-Alkyl)-phenyl-$C_{1-3}$-alkylamino-, Pyrrolidino- oder Piperidinogruppe substituiert sein kann,

durch eine Phenyl-$C_{1-3}$-alkylamino-$C_{1-3}$-alkylgruppe, die im Phenylkern durch eine Trifluormethylgruppe, durch Fluor-, Chlor-, Brom- oder Jodatome, durch $C_{1-5}$-Alkyl- oder $C_{1-3}$-Alkoxygruppen mono- oder disubstituiert sein kann, wobei die Substituenten gleich oder verschieden sein können, und zusätzlich am Aminstickstoffatom durch eine $C_{1-3}$-Alkylgruppe, in der die Wasserstoffatome ab Position 2 ganz oder teilweise durch Fluoratome ersetzt sein können, substituiert sein kann,

durch eine $C_{1-5}$-Alkyl-, Phenyl-, Imidazolyl-, $C_{3-7}$-Cycloalkyl-, $C_{1-3}$-Alkoxy-$C_{1-3}$-alkoxy-, Phenyl-$C_{1-3}$-alkoxy-, Carboxy-$C_{1-3}$-alkyl-, $C_{1-3}$-Alkoxycarbonyl-$C_{1-3}$-alkyl-, Carboxy-, $C_{1-3}$-Alkoxycarbonyl-, Aminocarbonyl-, $C_{1-3}$-Alkylaminocarbonyl-, Di-($C_{1-3}$-Alkyl)-aminocarbonyl-, Phenyl-$C_{1-3}$-alkylaminocarbonyl-, N- ($C_{1-3}$-Alkyl)-phenyl-$C_{1-3}$-alkylaminocarbonyl-, Piperazinocarbonyl-, N-($C_{1-3}$-Alkyl)-piperazinocarbonyl-, Nitro-, Amino-, $C_{1-3}$-Alkylamino-, Di-($C_{1-3}$-alkyl)-amino-, Pyrrolidino-, Piperidino-, Morpholino-, $C_{2-4}$-Alkanoylamino-, N-($C_{1-3}$-Alkyl)-$C_{2-4}$-alkanoylamino-, Benzoylamino- oder N- ($C_{1-3}$-Alkyl) -benzoylaminogruppe,

durch eine N-($C_{1-3}$-Alkyl)-$C_{2-4}$-alkanoylaminogruppe, die im Alkylteil zusätzlich durch eine Carboxy- oder $C_{1-3}$-Alkoxycarbonylgruppe substituiert ist,

durch eine $C_{1-3}$-Alkylaminocarbonyl- oder Di-($C_{1-3}$-Alkyl)-aminocarbonylgruppe, in denen ein Alkylteil zusätzlich durch eine Di-($C_{1-3}$-Alkyl)-aminogruppe substituiert ist, oder

durch eine N-($C_{1-3}$-Alkyl)-$C_{1-3}$-alkylsulfonylamino- oder N-($C_{1-3}$-Alkyl)-phenylsulfonylaminogruppe, in denen der Alkylteil zusätzlich durch eine Cyano-, Carboxy-, $C_{1-3}$-Alkoxycarbonyl-, $C_{1-3}$-Alkylamino-, Di- ($C_{1-3}$-Alkyl)-amino-, Aminocarbonyl-, $C_{1-3}$-Alkylaminocarbonyl-, Di-($C_{1-3}$-Alkyl)-aminocarbonyl-, Piperidinocarbonyl- oder 2-[Di-($C_{1-3}$-Alkylamino)]-ethylaminocarbonylgruppe substituiert sein kann, substituiert sind,

eine durch eine $C_{1-3}$-Alkylgruppe substituierte Phenyl- oder Thienylgruppe, in der der Alkylteil durch eine Hydroxy-, $C_{1-3}$-Alkoxy-, Carboxy-, $C_{1-3}$-Alkoxy-carbonyl-, Amino-, $C_{1-5}$-Alkylamino-, Di-($C_{1-5}$-Alkyl)-amino-, $C_{2-4}$-Alkanoylamino-, N-($C_{1-3}$-Alkyl)-$C_{2-4}$-alkanoylamino-, Pyrrolidino-, Dehydropyrrolidino-, Piperidino-, Dehydropiperidino-, 3-Hydroxypiperidino-, 4-Hydroxypiperidino-, Hexamethylenimino-, Morpholino-, Thiomorpholino-, Piperazino-, 4-($C_{1-3}$-Alkyl)-piperazino-, 4-Phenyl-piperazino-, 4-($C_{2-4}$-Alkanoyl)-piperazino-, 4-Benzoyl-piperazino- oder Imidazolylgruppe substituiert ist,

wobei die vorstehend erwähnten gesättigten Cycloalkyleniminoringe, $C_{1-5}$-Alkylamino- oder Di-($C_{1-5}$-Alkyl)-aminogruppen zusätzlich durch eine oder zwei $C_{1-5}$-Alkylgruppen, durch eine $C_{3-7}$-Cycloalkyl-, Hydroxy-, $C_{1-3}$-Alkoxy-, Carboxy-, $C_{1-3}$-Alkoxycarbonyl-, Aminocarbonyl-, $C_{1-3}$-Alkylaminocarbonyloder Di-($C_{1-3}$-Alkyl)-aminocarbonylgruppe, durch eine im Phenylkern gegebenenfalls durch Fluor-, Chlor-, Brom- oder Jodatome, durch $C_{1-3}$-Alkyl- oder Cyanogruppen mono- oder disubstituierte Phenyl-$C_{1-3}$-alkyl- oder Phenylgruppe, wobei die Substituenten gleich oder verschieden sein können, substituiert sein können

oder eine zu dem Stickstoffatom benachbarte Methylengruppe in den vorstehend erwähnten Cycloalkyleniminoringe durch eine Carbonyl- oder Sulfonylgruppe ersetzt sein kann, und die vorstehend erwähnten monosubstituierten Phenylgruppen zusätzlich durch ein Fluor-, Chlor- oder Bromatom, eine Methyl-, Amino-, $C_{1-3}$-Alkylamino- oder Di- ($C_{1-3}$-Alkyl)-aminogruppe substituiert sein können, oder

an einen der vorstehend erwähnten unsubstituierten Cycloalkyleniminoringe über zwei benachbarte Kohlenstoffatome ein gegebenenfalls durch eine oder zwei $C_{1-3}$-Alkoxygruppen substituierter Phenylring ankondensiert sein kann,

wobei die bei der Definition der vorstehend erwähnten Resten erwähnten Carboxygruppen zusätzlich durch eine in-vivo in eine Carboxygruppe überführbare Gruppe ersetzt sein und
die bei der Definition der vorstehend erwähnten Resten erwähnten Amino- und Iminogruppen zusätzlich durch einen in vivo abspaltbaren Rest substituiert sein können, bedeuten,
deren Isomere und deren Salze.

**2.** Substituierte Indolinone der allgemeinen Formel I gemäß Anspruch 1, in denen
X ein Sauerstoffatom,
$R_1$ ein Wasserstoffatom oder eine $C_{1-4}$-Alkoxy-carbonylgruppe,
$R_2$ eine Carboxy-, $C_{1-4}$-Alkoxy-carbonyl- oder Aminocarbonylgruppe, in der der Aminoteil durch eine oder zwei $C_{1-3}$-Alkylgruppen substituiert sein kann und die Substituenten gleich oder verschieden sein können,
$R_3$ eine gegebenenfalls durch ein Fluor-, Chlor- oder Bromatom, durch eine Methyl-, Cyano- oder Aminomethylgruppe substituierte Phenylgruppe,
$R_4$ ein Wasserstofatom oder eine Methylgruppe und
$R_5$ ein Wasserstofatom,
eine gegebenenfalls durch eine Carboxy- oder $C_{1-3}$-Alkoxy-carbonylgruppe substituierte $C_{1-5}$-Alkylgruppe oder eine Benzylgruppe,
eine gegebenenfalls durch eine Methylgruppe substitierte $C_{3-7}$-Cycloalkylgruppe,
eine-gegebenenfalls durch eine Methylgruppe substituierte Indanyl-, Pyridyl-, Oxazolyl-, Thiazolyl- oder Imidazolylgruppe, an die jeweils zusätzlich über zwei benachbarte Kohlenstoffatome ein Phenylring ankondensiert sein kann,
eine gegebenenfalls durch ein Fluor-, Chlor- oder Bromatom, durch eine Methoxy-, Carboxy-, $C_{1-3}$-Alkyloxycarbonyl-, Nitrooder Aminosulfonylgruppe substituierte Methylphenylgruppe oder eine Dimethoxyphenylgruppe,
eine über ein Kohlenstoffatom verknüpfte Pyrrolidinyl- oder Piperidinylgruppe, die jeweils am Stickstoffatom durch eine $C_{1-3}$-Alkylgruppe substituiert sind,
eine Phenylgruppe, die

durch eine Trifluormethoxygruppe, durch ein ein Fluor-, Chlor-, Brom- oder Jodatom,

durch eine $C_{1-3}$-Alkoxygruppe, die in 2- oder 3-Stellung durch eine Amino-, $C_{1-3}$-Alkylamino-, Di-($C_{1-3}$-Alkyl)-amino-, Phenyl-$C_{1-3}$-alkylamino-, N- ($C_{1-3}$-Alkyl)-phenyl-$C_{1-3}$-alkylamino-, Pyrrolidino- oder Piperidinogruppe substituiert sein kann,

durch eine Phenyl-$C_{1-3}$-alkylamino-$C_{1-3}$-alkylgruppe, die im Phenylkern durch ein Fluor-, Chlor-, Brom- oder Jodatom, durch eine $C_{1-5}$-Alkyl-, $C_{1-3}$-Alkoxy- oder Trifluormethylgruppe substituiert sein kann und zusätzlich am Aminstickstoffatom durch eine $C_{1-3}$-Alkylgruppe, in der die Wasserstoffatome ab Position 2 ganz oder teilweise durch Fluoratome ersetzt sein können, substituiert sein kann

durch eine $C_{1-5}$-Alkyl-, Phenyl-, Imidazolyl-, $C_{3-7}$-Cycloalkyl-, $C_{1-3}$-Alkoxy-$C_{1-3}$-alkoxy-, Phenyl-$C_{1-3}$-alkoxy-, Carboxy-$C_{1-3}$-alkyl-, $C_{1-3}$-Alkoxycarbonyl-$C_{1-3}$-alkyl-, Carboxy-, $C_{1-3}$-Alkoxycarbonyl-, Aminocarbonyl-, $C_{1-3}$-Alkylaminocarbonyl-, Di- ($C_{1-3}$-Alkyl)-aminocarbonyl-, Phenyl-$C_{1-3}$-alkylaminocarbonyl-, N- ($C_{1-3}$-Alkyl)-phenyl-$C_{1-3}$-alkylaminocarbonyl-, Piperazinocarbonyl-, N-($C_{1-3}$-Alkyl)-piperazinocarbonyl-, Nitro-, Amino-, $C_{1-3}$-Alkylamino-, Di-($C_{1-3}$-alkyl)-amino-, Pyrrolidino-, Piperidino-, Morpholino-, $C_{2-4}$-Alkanoylamino-, N-($C_{1-3}$-Alkyl)-$C_{2-4}$-alkanoylamino-, Benzoylamino- oder N- ($C_{1-3}$-Alkyl)-benzoylaminogruppe,

durch eine N-($C_{1-3}$-Alkyl)-$C_{2-4}$-alkanoylaminogruppe, die im Alkylteil zusätzlich durch eine Carboxy- oder $C_{1-3}$-Alkoxycarbonylgruppe substituiert ist,

durch eine $C_{1-3}$-Alkylaminocarbonyl- oder Di-($C_{1-3}$-Alkyl)-aminocarbonylgruppe, in denen ein Alkylteil zusätzlich durch eine Di-($C_{1-3}$-Alkyl)-aminogruppe substituiert ist, oder

durch eine N-($C_{1-3}$-Alkyl)-$C_{1-3}$-alkylsulfonylamino- oder N-($C_{1-3}$-Alkyl)-phenylsulfonylaminogruppe, in denen der Alkylteil zusätzlich durch eine Cyano-, Carboxy-, $C_{1-3}$-Alkoxycarbonyl-, $C_{1-3}$-Alkylamino-, Di-($C_{1-3}$-Alkyl)-amino-, Aminocarbonyl-, $C_{1-3}$-Alkylaminocarbonyl-, Di-($C_{1-3}$-Alkyl)-aminocarbonyl-, Piperidinocarbonyl- oder 2-[Di-($C_{1-3}$-Alkylamino)]-ethylaminocarbonylgruppe substituiert sein kann, substituiert ist,

eine gegebenenfalls durch eine $C_{1-3}$-Alkylgruppe substituierte Phenylgruppe, in der der Alkylteil durch eine Hydroxy-, $C_{1-3}$-Alkoxy-, Carboxy-, $C_{1-3}$-Alkoxy-carbonyl-, Amino-, $C_{1-5}$-Alkylamino-, Di-($C_{1-5}$-Alkyl)-amino-, $C_{2-4}$-Alkanoylamino-, N-($C_{1-3}$-Alkyl)-$C_{2-4}$-alkanoylamino-, Pyrrolidino-, Dehydropyrrolidino-, Piperidino-, Dehydropiperidino-, 3-Hydroxypiperidino-, 4-Hydroxypiperidino-, Hexamethylenimino-, Morpholino-, Thiomorpholino-, Piperazino-, 4-($C_{1-3}$-Alkyl)-piperazino-, 4-Phenyl-piperazino-, 4-($C_{2-4}$-Alkanoyl)-piperazino-, 4-Benzoyl-piperazino- oder Imidazolylgruppe substituiert ist,

wobei die vorstehend erwähnten gesättigten Cycloalkyleniminoringe, $C_{1-5}$-Alkylamino- oder Di-($C_{1-5}$-Alkyl)-aminogruppen zusätzlich durch eine oder zwei $C_{1-5}$-Alkylgruppen, durch eine $C_{3-7}$-Cycloalkyl-, Hydroxy-, $C_{1-3}$-Alkoxy-, Carboxy-, $C_{1-3}$-Alkoxycarbonyl-, Aminocarbonyl-, $C_{1-3}$-Alkylaminocarbonyl- oder Di-($C_{1-3}$-Alkyl)-aminocarbonylgruppe, durch eine im Phenylkern gegebenenfalls durch Fluor-, Chlor-, Brom- oder Jodatome, durch $C_{1-3}$-

Alkyl- oder Cyanogruppen mono- oder disubstituierte Phenyl-$C_{1-3}$-alkyl- oder Phenylgruppe, wobei die Substituenten gleich oder verschieden sein können, substituiert sein können

oder eine zu dem Stickstoffatom benachbarte Methylengruppe in den vorstehend erwähnten Cycloalkyleniminoringe durch eine Carbonyl- oder Sulfonylgruppe ersetzt sein kann, und die vorstehend erwähnten monosubstituierten Phenylgruppen zusätzlich durch ein Fluor-, Chlor- oder Bromatom, eine Methyl-, Amino-, $C_{1-3}$-Alkylamino- oder Di-($C_{1-3}$-Alkyl)-aminogruppe substituiert sein können, oder

an einen der vorstehend erwähnten unsubstituierten Cycloalkyleniminoringe über zwei benachbarte Kohlenstoffatome ein gegebenenfalls durch eine oder zwei $C_{1-3}$-Alkoxygruppen substituierter Phenylring ankondensiert sein kann,

bedeuten, deren Isomere und deren Salze.

**3.** Substituierte Indolinone der allgemeinen Formel I gemäß Anspruch 1, in denen
X ein Sauerstoffatom,
$R_1$ ein Wasserstoffatom,
$R_2$ eine Carboxy-, $C_{1-4}$-Alkoxycarbonyl- oder Aminocarbonylgruppe, in der der Aminoteil durch eine oder zwei $C_{1-3}$-Alkylgruppen substituiert sein kann und die Substituenten gleich oder verschieden sein können,
$R_3$ eine gegebenenfalls durch eine Methylgruppe substituierte Phenylgruppe,
$R_4$ ein Wasserstoffatom oder eine Methylgruppe und
$R_5$ ein Wasserstoffatom,
eine $C_{1-3}$-Alkylgruppe, eine Benylgruppe oder eine durch eine Carboxy- oder $C_{1-3}$-Alkoxycarbonylgruppe substituierte Methyloder Ethylgruppe,
eine gegebenenfalls durch eine Methylgruppe substitierte $C_{3-7}$-Cycloalkylgruppe,
eine gegebenenfalls durch eine Methylgruppe substituierte Indanyl-, Pyridyl-, Oxazolyl-, Thiazolyl- oder Imidazolylgruppe, an die jeweils zusätzlich über zwei benachbarte Kohlenstoffatome ein Phenylring ankondensiert sein kann,
eine gegebenenfalls durch ein Fluor-, Chlor- oder Bromatom, durch eine Methoxy-, Carboxy-, $C_{1-3}$-Alkyloxycarbonyl-, Nitrooder Aminosulfonylgruppe substituierte Methylphenylgruppe oder eine Dimethoxyphenylgrruppe,
eine 3-Pyrrolidinyl- oder 4-Piperidinylgruppe, die jeweils am Stickstoffatom durch eine $C_{1-3}$-Alkylgruppe substituiert sind,
eine Phenylgruppe, die

durch eine Trifluormethoxy-, Benzyloxy-, Cyano- oder Nitrogruppe, durch ein ein Fluor-, Chlor- oder Bromatom,

durch eine $C_{1-3}$-Alkoxygruppe, wobei die Ethoxy- und n-Propoxygruppe jeweils endständig durch eine Dimethylamino-, Diethylamino-, N-Ethyl-methylamino-, N-Benzyl-methylamino- oder Piperidinogruppe substituiert sein kann,

durch eine Phenyl-$C_{1-3}$-alkylamino-$C_{1-3}$-alkylgruppe, die im Phenylkern durch ein Fluor-, Chlor-, Brom- oder Jodatom, durch eine Methyl-, Methoxy- oder Trifluormethylgruppe und zusätzlich am Aminstickstoffatom durch eine $C_{1-5}$-Alkyl- oder 2,2,2-Trifluorethylgruppe substituiert sein kann,

durch eine $C_{1-4}$-Alkyl-, Phenyl-, Imidazolyl-, Cyclohexyl-, Methoxymethyl-, Carboxymethyl-, $C_{1-3}$-Alkoxycarbonyl-methyl-, Carboxy-, $C_{1-3}$-Alkoxycarbonyl-, Aminocarbonyl-, $C_{1-3}$-Alkylaminocarbonyl-, Di-($C_{1-3}$-Alkyl)-aminocarbonyl-, Phenyl-$C_{1-3}$-alkylaminocarbonyl-, N- ($C_{1-3}$-Alkyl)-phenyl-$C_{1-3}$-alkylaminocarbonyl-, Piperazinocarbonyl-, N-($C_{1-3}$-Alkyl)-piperazinocarbonyl-, Amino-, $C_{1-3}$-Alkylamino-, Di- ($C_{1-3}$-alkyl)-amino-, Pyrrolidino-, Piperidino-, Morpholino-, $C_{2-4}$-Alkanoylamino-, N-($C_{1-3}$-Alkyl)-$C_{2-4}$-alkanoylamino-, Benzoylamino- oder N- ($C_{1-3}$-Alkyl)-benzoylaminogruppe,

durch eine N- ($C_{1-3}$-Alkyl)-$C_{2-4}$-alkanoylaminogruppe, die im Alkylteil zusätzlich durch eine Carboxy- oder $C_{1-3}$-Alkoxycarbonylgruppe substituiert ist,

durch eine $C_{1-3}$-Alkylaminocarbonyl- oder Di- ($C_{1-3}$-Alkyl)-aminocarbonylgruppe, in denen ein Alkylteil zusätzlich durch eine Di-($C_{1-3}$-Alkyl)-aminogruppe substituiert ist, oder

durch eine N-($C_{1-3}$-Alkyl)-$C_{1-3}$-alkylsulfonylamino- oder N-($C_{1-3}$-Alkyl)-phenylsulfonylaminogruppe, in denen der Alkylteil zusätzlich durch eine Cyano-, Carboxy-, $C_{1-3}$-Alkoxycarbonyl-, $C_{1-3}$-Alkylamino-, Di-($C_{1-3}$-Alkyl)-amino-, Aminocarbonyl-, $C_{1-3}$-Alkylaminocarbonyl-, Di-($C_{1-3}$-Alkyl)-aminocarbonyl-, Piperidinocarbonyl- oder 2-[Di-($C_{1-3}$-Alkylamino)]-ethylaminocarbonylgruppe substituiert sein kann, substituiert ist,

eine gegebenenfalls durch eine $C_{1-3}$-Alkylgruppe substituierte Phenylgruppe, in der die Alkylgruppe durch eine Hydroxy-, $C_{1-3}$-Alkoxy-, Carboxy-, $C_{1-3}$-Alkoxycarbonyl-, Amino-, $C_{1-5}$-Alkylamino-, Di- ($C_{1-5}$-Alkyl)-amino-, $C_{2-4}$-Alkanoylamino-, N-($C_{1-3}$-Alkyl) -$C_{2-4}$-alkanoylamino-, Pyrrolidino-, Dehydropyrrolidino-, Piperidino-, Dehydropiperidino-, 4-Hydroxypiperidino-, Hexamethylenimino-, Morpholino-, Thiomorpholino-, Piperazino-, 4-($C_{1-3}$-Alkyl)-piperazino-, 4-Phenyl-piperazino-, 4-($C_{2-4}$-Alkanoyl)-piperazino-, 4-Benzoyl-piperazino- oder Imidazolylgruppe substituiert ist,

wobei die vorstehend erwähnten gesättigten Cycloalkyleniminoringe zusätzlich durch eine Phenylgruppe oder durch eine oder zwei Methylgruppen,

die vorstehend erwähnten $C_{1-5}$-Alkylamino- und Di-($C_{1-5}$-Alkyl)-aminogruppen zusätzlich durch eine oder zwei $C_{1-3}$-Alkylgruppen, durch eine Cyclohexyl-, Hydroxy-, $C_{1-3}$-Alkoxy-, Carboxy-, $C_{1-3}$-Alkoxycarbonyl-, Aminocarbonyl-, $C_{1-3}$-Alkylaminocarbonyloder Di-($C_{1-3}$-Alkyl)-aminocarbonylgruppe, durch eine im Phenylkern gegebenenfalls durch ein Fluor-, Chlor-, Brom- oder Jodatom, durch eine Methyl- oder Cyanogruppe substituierte Phenyl-$C_{1-3}$-alkyl- oder Phenylgruppe substituiert sein kann, oder eine zu dem Stickstoffatom benachbarte Methylengruppe in den vorstehend erwähnten Cycloalkyleniminoringe durch eine Carbonyl- oder Sulfonylgruppe ersetzt sein kann, und die vorstehend erwähnten monosubstituierten Phenylgruppen zusätzlich durch ein Fluor-, Chlor- oder Bromatom, eine Methyl-, Amino-, $C_{1-3}$-Alkylamino- oder Di- ($C_{1-3}$-Alkyl)-aminogruppe substituiert sein können, oder

an einen der vorstehend erwähnten unsubstituierten Cycloalkyleniminoringe über zwei benachbarte Kohlenstoffatome ein gegebenenfalls durch eine oder zwei $C_{1-3}$-Alkoxygruppen substituierter Phenylring ankondensiert sein kann,

bedeuten, deren Isomere und deren Salze.

**4.** Substituierte Indolinone der allgemeinen Formel I gemäß Anspruch 1, in denen
X ein Sauerstoffatom,
$R_1$ ein Wasserstoffatom,
$R_2$ eine Carboxy- oder Aminocarbonylgruppe, in der der Aminoteil durch eine oder zwei $C_{1-3}$-Alkylgruppen substituiert sein kann und die Substituenten gleich oder verschieden sein können,
$R_3$ eine gegebenenfalls durch eine Methylgruppe substituierte Phenylgruppe,
$R_4$ ein Wasserstoffatom und
$R_5$ ein Wasserstoffatom,
eine 3-Pyrrolidinyl- oder 4-Piperidinylgruppe, die jeweils am Stickstoffatom durch eine $C_{1-3}$-Alkylgruppe substituiert sind,
eine Phenylgruppe, die

durch eine $C_{1-3}$-Alkoxygruppe, wobei die Ethoxy- und n-Propoxygruppe jeweils endständig durch eine Dimethylamino-, Diethylamino-, N-Ethyl-methylamino-, N-Benzyl-methylamino- oder Piperidinogruppe substituiert sein kann,

durch eine Phenyl-$C_{1-3}$-alkylamino-$C_{1-3}$-alkylgruppe, die im Phenylkern durch ein Fluor-, Chlor-, Brom- oder Jodatom, durch eine Methyl-, Methoxy- oder Trifluormethylgruppe und zusätzlich am Aminstickstoffatom durch eine $C_{1-5}$-Alkyl- oder 2,2,2-Trifluorethylgruppe substituiert sein kann, substituiert ist,

eine gegebenenfalls durch eine $C_{1-3}$-Alkylgruppe substituierte Phenylgruppe, in der die Alkylgruppe durch eine Hydroxy-, $C_{1-3}$-Alkoxy-, Carboxy-, $C_{1-3}$-Alkoxycarbonyl-, Amino-, $C_{1-5}$-Alkylamino-, Di- ($C_{1-5}$-Alkyl)-amino-, $C_{2-4}$-Alkanoylamino-, N-($C_{1-3}$-Alkyl)-$C_{2-4}$-alkanoylamino-, Pyrrolidino-, Dehydropyrrolidino-, Piperidino-, Dehydropiperidino-, 4-Hydroxypiperidino-, Hexamethylenimino-, Morpholino-, Thiomorpholino-, Piperazino-, 4-($C_{1-3}$-Alkyl)-piperazino-, 4-Phenyl-piperazino-, 4-($C_{2-4}$-Alkanoyl)-piperazino-, 4-Benzoyl-piperazino- oder Imidazolylgruppe substituiert ist,

wobei die vorstehend erwähnten gesättigten Cycloalkyleniminoringe zusätzlich durch eine Phenylgruppe oder durch eine oder zwei Methylgruppen,

die vorstehend erwähnten $C_{1-5}$-Alkylamino- und Di-($C_{1-5}$-Alkyl)-aminogruppen zusätzlich durch eine oder zwei $C_{1-3}$-Alkylgruppen, durch eine Cyclohexyl-, Hydroxy-, $C_{1-3}$-Alkoxy-, Carboxy-, $C_{1-3}$-Alkoxycarbonyl-, Aminocarbonyl-, $C_{1-3}$-Alkylaminocarbonyloder Di-($C_{1-3}$-Alkyl)-aminocarbonylgruppe, durch eine im Phenylkern gegebenenfalls durch ein Fluor-, Chlor-, Brom- oder Jodatom, durch eine Methyl- oder Cyanogruppe substituierte Phenyl-$C_{1-3}$-alkyl- oder Phenylgruppe substituiert sein kann, oder eine zu dem Stickstoffatom benachbarte Methylengruppe in den vorstehend erwähnten Cycloalkyleniminoringe durch eine Carbonyl- oder Sulfonylgruppe ersetzt sein kann, und die vorstehend erwähnten monosubstituierten Phenylgruppen zusätzlich durch ein Fluor-, Chlor- oder Bromatom, eine Methyl-, Amino-, $C_{1-3}$-Alkylamino- oder Di- ($C_{1-3}$-Alkyl) -aminogruppe substituiert sein können, oder

an einen der vorstehend erwähnten unsubstituierten Cycloalkyleniminoringe über zwei benachbarte Kohlenstoffatome ein gegebenenfalls durch eine oder zwei $C_{1-3}$-Alkoxygruppen substituierter Phenylring ankondensiert sein kann,

bedeuten, deren Isomere und deren Salze.

**5.** Substituierte Indolinone der allgemeinen Formel I nach mindestens einem der Ansprüch 1 bis 4, in denen der Rest $R_2$ in 5-Stellung steht.

**6.** Folgende substituierte Indolinone der allgemeinen Formel I gemäß Anspruch 1:

(a) 3-Z-[1-(4-Aminomethyl-phenylamino)-1-phenyl-methylen]-5-amido-2-indolinon,

(b) 3-Z-[1-Phenylamino)-1-phenyl-methylen]-5-amido-2-indolinon,

(c) 3-Z-[1-(4-Brom-phenylamino)-1-phenyl-methylen]-5-amido-2-indolinon,

(d) 3-Z-[1-(4-Dimethylamino-methyl)-phenylamino)-1-phenyl-methylen] -5-amido-2-indolinon,

(e) 3-Z-[1-(4-Pyrrolidinomethyl-phenylamino)-1-phenyl-methylen] -5-amido-2-indolinon,

(f) 3-Z-[1-(4-Piperidinomethyl-phenylamino)-1-phenyl-methylen]-5-amido-2-indolinon,

(g) 3-Z-[1-(4-Hexamethyleniminomethyl-phenylamino)-1-phenyl-methylen] -5-amido-2-indolinon,

(h) 3-Z-[1-(4-(4-Benzyl-piperidino)-methyl)-phenylamino)-1-phenyl-methylen] -5-amido-2-indolinon,

(i) 3-Z-[1-(4-(N-Butyl-aminomethyl)-phenylamino)-1-phenyl-methylen] -5-amido-2-indolinon,

(j) 3-Z-[1-(4-(N-(Phenyl-methyl)-aminomethyl)-phenylamino)-1-phenyl-methylen]-5-amido-2-indolinon,

(k) 3-Z-[1-(4-(N-Methyl-N-benzyl-amino-methyl)-phenylamino)-1-phenyl-methylen]-5-amido-2-indolinon,

(1) 3-Z-[1-(4-Piperidino-methyl-phenylamino)-1-phenyl-methylen]-5-dimethylcarbamoyl-2-indolinon,

(m) 3-Z-[1-(4-Piperidino-methyl-phenylamino)-1-phenyl-methylen]-5-diethylcarbamoyl-2-indolinon,

(n) 3-Z-[1-(4-(3-Diethylamino-propoxy)-phenylamino)-1-phenylmethylen]-5-amido-2-indolinon

und deren Salze.

**7.** Physiologisch verträgliche Salze der Verbindungen gemäß den Ansprüchen 1 bis 6.

**8.** Arzneimittel, enthaltend eine Verbindung nach mindestens einem der Ansprüche 1 bis 6 oder ein Salz gemäß Anspruch 7 neben gegebenenfalls einem oder mehreren inerten Trägerstoffen und/oder Verdünnungsmitteln.

**9.** Verwendung einer Verbindung nach mindestens einem der Ansprüche 1 bis 6 oder ein Salz gemäß Anspruch 7 zur Herstellung eines Arzneimittels, welches zur Behandlung von exzessiven oder anomalen Zellproliferationen geeignet ist.

**10.** Verfahren zur Herstellung eines Arzneimittels gemäß Anspruch 8, **dadurch gekennzeichnet, daß** auf nichtchemischem Wege eine Verbindung nach mindestens einem der Ansprüche 1 bis 6 oder ein Salz gemäß Anspruch 7 in einen oder mehrere inerte Trägerstoffe und/oder Verdünnungsmittel eingearbeitet wird.

**11.** Verfahren zur Herstellung der Verbindungen gemäß den Ansprüchen 1 bis 7, **dadurch gekennzeichnet, daß**

a. eine Verbindung der allgemeinen Formel

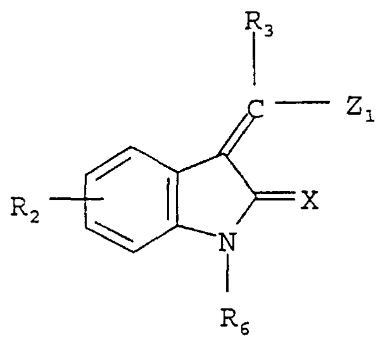

(II),

in der
X, $R_2$ und $R_3$ wie in den Ansprüchen 1 bis 6 erwähnt definiert sind,
$R_6$ ein Wasserstoffatom, eine Schutzgruppe für das Stickstoffatom der Lactamgruppe oder eine Bindung an eine Festphase und
$Z_1$ ein Halogenatom, eine Hydroxy-, Alkoxy- oder Aralkoxygruppe bedeuten,
mit einem Amin der allgemeinen Formel

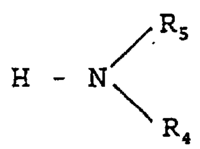

(III),

in der
$R_4$ und $R_5$ wie in den Ansprüchen 1 bis 6 erwähnt definiert sind, umgesetzt und erforderlichenfalls anschließend eine verwendete Schutzgruppe für das Stickstoffatom der Lactamgruppe oder eine so erhaltene Verbindung von einer Festphase abgespalten wird oder

b. zur Herstellung einer Verbindung der allgemeinen Formel I, die eine Aminomethylgruppe enthält und X ein Sauerstoffatom darstellt, eine Verbindung der allgemeinen Formel

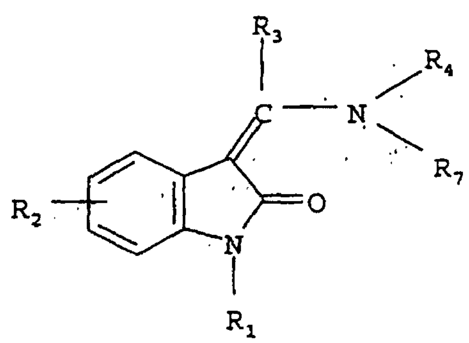

(IV),

in der
$R_1$ bis $R_4$ wie in den Ansprüchen 1 bis 6 erwähnt definiert sind und
$R_7$ mit der Maßgabe die für $R_5$ in den Ansprüchen 1 bis 6 erwähnten Bedeutungen aufweist, daß $R_5$ eine Cyanogruppe enthält, reduziert wird und
gewünschtenfalls anschließend eine so erhaltene Verbindung der allgemeinen Formel I, die eine Alkoxycarbonylgruppe enthält, mittels Hydrolyse in eine entsprechende Carboxyverbindung übergeführt wird oder
eine so erhaltene Verbindung der allgemeinen Formel I, die eine Amino- oder Alkylaminogruppe enthält, mittels Alkylierung oder reduktiver Alkylierung in eine entsprechende Alkylamino- oder Dialkylaminoverbindung übergeführt wird oder
eine so erhaltene Verbindung der allgemeinen Formel I, die eine Amino- oder Alkylaminogruppe enthält, mittels Acylierung in eine entsprechende Acylverbindung übergeführt wird oder
eine so erhaltene Verbindung der allgemeinen Formel I, die eine Carboxygruppe enthält, mittels Veresterung oder Amidierung in eine entsprechende Ester- oder Aminocarbonylverbindung übergeführt wird oder
erforderlichenfalls ein während den Umsetzungen zum Schutze von reaktiven Gruppen verwendeter Schutzrest abgespalten wird oder
gewünschtenfalls anschließend eine so erhaltene Verbindung der allgemeinen Formel I in ihre Stereoisomere aufgetrennt wird oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

**1.** Substituted indolinones of general formula

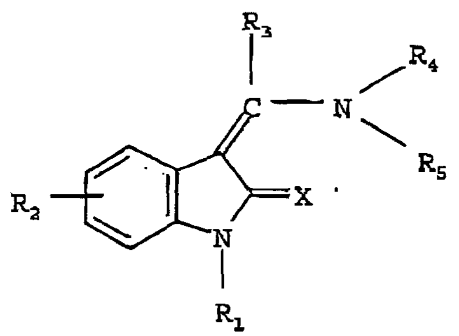

(I),

wherein
X denotes an oxygen or sulphur atom,
$R_1$ denotes a hydrogen atom, a $C_{1-4}$-alkoxy-carbonyl or $C_{2-4}$-alkanoyl group,
$R_2$ denotes a carboxy-, $C_{1-4}$-alkoxy-carbonyl or aminocarbonyl group wherein the amino moiety may be substituted by one or two $C_{1-3}$-alkyl groups and the substitutents may be identical or different,
$R_3$ denotes a phenyl or naphthyl group which may be substituted by fluorine, chlorine or bromine atoms, by $C_{1-3}$-alkyl, $C_{1-3}$-alkoxy, cyano, trifluoromethyl, nitro, amino, $C_{1-3}$-alkylamino, di-($C_{1-3}$-alkyl)-amino, $C_{2-4}$-alkanoyl-amino, N-($C_{1-3}$-alkyl)-$C_{2-4}$-alkanoylamino, $C_{1-3}$-alkylsulphonylamino, amino-$C_{1-3}$-alkyl, $C_{1-3}$-alkylamino-$C_{1-3}$-alkyl, di-($C_{1-3}$-alkyl)-amino-$C_{1-3}$-alkyl, N- ($C_{2-4}$-alkanoyl)-amino-$C_{1-3}$-alkyl or N- ($C_{2-4}$-alkanoyl)-$C_{1-3}$-alkylamino-$C_{1-3}$-alkyl groups and the substituents may be identical or different,
$R_4$ denotes a hydrogen atom or a $C_{1-3}$-alkyl group and
$R_5$ denotes a hydrogen atom,
a $C_{1-5}$-alkyl group optionally substituted by a phenyl, carboxy or $C_{1-3}$-alkoxy-carbonyl group,
a $C_{3-7}$-cycloalkyl group optionally substituted by a $C_{1-3}$-alkyl group,
an indanyl group optionally substituted by a $C_{1-3}$-alkyl group,
a 5-membered heteroaryl group which contains an imino group optionally substituted by a $C_{1-3}$-alkyl group, an oxygen or sulphur atom or an imino group optionally substituted by a $C_{1-3}$-alkyl group and an oxygen; sulphur or nitrogen atom or two nitrogen atoms or a 6-membered heteroaryl group which contains 1 to 3 nitrogen atoms, whilst additionally a 1,3-butadienylene bridge may be attached via two adjacent carbon atoms or via one carbon atom and an adjacent imino group of the abovementioned 5- and 6-membered heteroaryl groups and the carbon skeleton of the abovementioned mono- and bicyclic rings may be mono or disubstituted by fluorine, chlorine, bromine or iodine atoms, by $C_{1-5}$-alkyl or cyano groups and the substituents may be identical or different,
a pyrrolidinyl or piperidinyl group linked via a carbon atom, which may be substituted at the nitrogen atom by a $C_{1-3}$-alkyl group,
a phenyl group optionally disubstituted by fluorine, chlorine, bromine or iodine atoms, by $C_{1-5}$-alkyl, $C_{1-3}$-alkoxy, carboxy, $C_{1-3}$-alkoxycarbonyl, aminosulphonyl, nitro or cyano groups, whilst the substituents may be identical or

different,
a phenyl, pyridyl, pyrimidyl or thienyl group each of which is substituted
by a trifluoromethoxy group, by a fluorine, chlorine, bromine or iodine atom, by a $C_{1-3}$-alkoxy group which may be substituted in the 2- or 3-position by an amino, $C_{1-3}$-alkylamino, di- ($C_{1-3}$-alkyl) amino, phenyl-$C_{1-3}$-alkylamino, N-($C_{1-3}$-alkyl)-phenyl-$C_{1-3}$-alkylamino, pyrrolidino or piperidino group,
by a phenyl-$C_{1-3}$-alkylamino-$C_{1-3}$-alkyl group which may be mono- or disubstituted in the phenyl nucleus by a trifluoromethyl group, by fluorine, chlorine, bromine or iodine atoms, by $C_{1-5}$-alkyl or $C_{1-3}$-alkoxy groups, whilst the substituents may be identical or different, and additionally may be replaced at the amine nitrogen atom by a $C_{1-3}$-alkyl group wherein the hydrogen atoms from position 2 may be wholly or partially replaced by fluorine atoms,
by a $C_{1-5}$-alkyl, phenyl, imidazolyl, $C_{3-7}$-cycloalkyl, $C_{1-3}$-alkoxy-$C_{1-3}$-alkoxy, phenyl-$C_{1-3}$-alkoxy, carboxy-$C_{1-3}$-alkyl, $C_{1-3}$-alkoxycarbonyl-$C_{1-3}$-alkyl, carboxy, $C_{1-3}$-alkoxycarbonyl, aminocarbonyl, $C_{1-3}$-alkylaminocarbonyl, di-($C_{1-3}$-alkyl)-aminocarbonyl, phenyl-$C_{1-3}$-alkylaminocarbonyl, N-($C_{1-3}$-alkyl)-phenyl-$C_{1-3}$-alkylaminocarbonyl, piperazinocarbonyl, N- ($C_{1-3}$-alkyl)- piperazinocarbonyl, nitro, amino, $C_{1-3}$-alkylamino, di-($C_{1-3}$-alkyl)-amino, pyrrolidino, piperidino, morpholino, $C_{2-4}$-alkanoyl-amino, N-($C_{1-3}$-alkyl)-$C_{2-4}$-alkanoylamino, benzoylamino or N-($C_{1-3}$-alkyl)-benzoylamino group,
by an N- ($C_{1-3}$-alkyl) -$C_{2-4}$-alkanoylamino group which is additionally substituted in the alkyl moiety by a carboxy or $C_{1-3}$-alkoxycarbonyl group,
by a $C_{1-3}$-alkylaminocarbonyl or di-($C_{1-3}$-alkyl)-aminocarbonyl group wherein an alkyl moiety is additionally substituted by as di-($C_{1-3}$-alkyl)-amino group, or
by an N-($C_{1-3}$-alkyl)-$C_{1-3}$-alkylsulphonylamino or N-($C_{1-3}$-alkyl)-phenylsulphonylamino group wherein the
alkyl moiety may additionally be substituted by a cyano, carboxy, $C_{1-3}$-alkoxycarbonyl, $C_{1-3}$-alkylamino, di-($C_{1-3}$-alkyl)-amino group, aminocarbonyl,
$C_{1-3}$-alkylaminocarbonyl, di-($C_{1-3}$-alkyl)-aminocarbonyl, piperidinocarbonyl or 2-[di-($C_{1-3}$-alkylamino)]-ethylaminocarbonyl group,
a phenyl or thienyl group substituted by a $C_{1-3}$-alkyl group wherein the alkyl moiety is substituted by a hydroxy, $C_{1-3}$-alkoxy, carboxy, $C_{1-3}$-alkoxy-carbonyl, amino, $C_{1-5}$-alkylamino, di-($C_{1-5}$-alkyl)-amino, $C_{2-4}$-alkanoylamino, N-($C_{1-3}$-alkyl)-$C_{2-4}$-alkanoylamino, pyrrolidino, dehydropyrrolidino, piperidino, dehydropiperidino, 3-hydroxypiperidino, 4-hydroxypiperidino, hexamethyleneimino, morpholino, thiomorpholino, piperazino, 4-($C_{1-3}$-alkyl)-piperazino, 4-phenyl-piperazino, 4-($C_{2-4}$-alkanoyl)-piperazino, 4-benzoyl-piperazino or imidazolyl group,
whilst the abovementioned saturated cycloalkyleneimino rings, $C_{1-5}$-alkylamino or di-($C_{1-5}$-alkyl)-amino groups may additionally be substituted by one or two $C_{1-5}$-alkyl groups; by a $C_{3-7}$-cycloalkyl, hydroxy, $C_{1-3}$-alkoxy, carboxy, $C_{1-3}$-alkoxycarbonyl, aminocarbonyl, $C_{1-3}$-alkylaminocarbonyl or di-($C_{1-3}$-alkyl)-aminocarbonyl group, by a phenyl-$C_{1-3}$-alkyl or phenyl group optionally mono- or disubstituted in the phenyl nucleus by fluorine, chlorine, bromine or iodine atoms or by $C_{1-3}$-alkyl or cyano groups, whilst the substituents may be identical or different,
or a methylene group adjacent to the nitrogen atom in. the abovementioned cycloalkyleneimino rings may be replaced by a carbonyl or sulphonyl group, and the abovementioned monosubstituted phenyl groups may additionally be substituted by a fluorine, chlorine or bromine atom or by a methyl, amino, $C_{1-3}$-alkylamino or di-($C_{1-3}$-alkyl)-amino group, or
a phenyl ring optionally substituted by one or two $C_{1-3}$-alkoxy groups may be fused to one of the abovementioned unsubstituted cycloalkyleneimino rings via two adjacent carbon atoms,
whilst the carboxy groups mentioned in the definition of the groups above may additionally be replaced by a group which can be converted *in vivo* into a carboxy group and
the amino and imino groups mentioned in the definition of the groups above may additionally be replaced by a group which can be cleaved *in vivo*,
the isomers thereof and the salts thereof.

**2.** Substituted indolinones of general formula I according to claim 1, wherein
X denotes an oxygen atom,
$R_1$ denotes a hydrogen atom or a $C_{1-4}$-alkoxycarbonyl group,
$R_2$ denotes a carboxy, $C_{1-4}$-alkoxycarbonyl or aminocarbonyl group wherein the amino moiety may be substituted by one or two $C_{1-3}$-alkyl groups and the subatituents may be identical or different,
$R_3$ denotes a phenyl group optionally substituted by a fluorine, chlorine or bromine atom, by a methyl, cyano or aminomethyl group,
$R_4$ denotes a hydrogen atom or a methyl group and
$R_5$ denotes a hydrogen atom,
a $C_{1-5}$-alkyl group optionally substituted by a carboxy or $C_{1-3}$-alkoxycarbonyl group, or a benzyl group,
a $C_{3-7}$-cycloalkyl group optionally substituted by a methyl group,
an indanyl, pyridyl, oxazolyl, thiazolyl or imidazolyl group optionally substituted by a methyl group, to which a

phenyl ring may additionally be fused via two adjacent carbon atoms,
a methylphenyl group optionally substituted by a fluorine, chlorine or bromine atom, or by a methoxy, carboxy, $C_{1-3}$-alkyloxycarbonyl, nitro or aminosulphonyl group,.or a dimethoxyphenyl group,
a pyrrolidinyl or piperidinyl group linked via a carbon atom, which may be substituted at the nitrogen atom by a $C_{1-3}$-alkyl group,
a phenyl group which is substituted
by a trifluoromethoxy group, by a fluorine, chlorine, bromine or iodine atom,
by a $C_{1-3}$-alkoxy group which may be substituted in the 2- or 3-position by an amino, $C_{1-3}$-alkylamino, di-($C_{1-3}$-alkyl)amino, phenyl-$C_{1-3}$-alkylamino, N-($C_{1-3}$-alkyl)-phenyl-$C_{1-3}$-alkylamino, pyrrolidino or piperidino group,
by a phenyl-$C_{1-3}$-alkylamino-$C_{1-3}$-alkyl group which may be [substituted] in the phenyl nucleus by a fluorine, chlorine, bromine or iodine atom, by a $C_{1-5}$-alkyl, $C_{1-3}$-alkoxy or trifluoromethyl group and additionally at the amine nitrogen atom by a $C_{1-3}$-alkyl group wherein the hydrogen atoms from position 2 may be wholly or partially replaced by fluorine atoms,

by a $C_{1-5}$-alkyl, phenyl, imidazolyl, $C_{3-7}$-cycloalkyl, $C_{1-3}$-alkoxy-$C_{1-3}$-alkoxy, phenyl-$C_{1-3}$-alkoxy, carboxy-$C_{1-3}$-alkyl, $C_{1-3}$-alkoxycarbonyl-$C_{1-3}$-alkyl, carboxy, $C_{1-3}$-alkoxycarbonyl, aminocarbonyl, $C_{1-3}$-alkylaminocarbonyl, di-($C_{1-3}$-alkyl)-aminocarbonyl, phenyl-$C_{1-3}$-alkylaminocarbonyl, N- ($C_{1-3}$-alkyl)-phenyl-$C_{1-3}$-alkylaminocarbonyl, piperazinocarbonyl, N-($C_{1-3}$-alkyl)-piperazinocarbonyl, nitro, amino, $C_{1-3}$-alkylamino, di-($C_{1-3}$alkyl)-amino, pyrrolidino, piperidino, morpholino,

$C_{2-4}$-alkanoyl-amino, N-($C_{1-3}$-alkyl)-$C_{2-4}$-alkanoylamino, benzoylamino or N-($C_{1-3}$-alkyl)-benzoylamino group, by an N-($C_{1-3}$-alkyl)-$C_{2-4}$-alkanoylamino group which is additionally substituted in the alkyl moiety by a carboxy or $C_{1-3}$-alkoxycarbonyl group,

by a $C_{1-3}$-alkylaminocarbonyl or di-($C_{1-3}$-alkyl)-aminocarbonyl group wherein an alkyl moiety is additionally substituted by a di-($C_{1-3}$-alkyl)-amino group, or

by an N-($C_{1-3}$-alkyl)-$C_{1-3}$-alkylsulphonylamino or N-($C_{1-3}$-alkyl)-phenylsulphonylamino group wherein the alkyl moiety may additionally be substituted by a cyano, carboxy, $C_{1-3}$-alkoxycarbonyl, $C_{1-3}$-alkylamino, di-($C_{1-3}$-alkyl)-amino group, aminocarbonyl, $C_{1-3}$-alkylaminocarbonyl, di-($C_{1-3}$-alkyl)-aminocarbonyl, piperidinocarbonyl or 2-[di-($C_{1-3}$-alkylamino)]-ethylaminocarbonyl group,
a phenyl group optionally substituted by a $C_{1-3}$-alkyl group wherein the alkyl moiety is substituted by a hydroxy, $C_{1-3}$-alkoxy, carboxy, $C_{1-3}$-alkoxy-carbonyl, amino, $C_{1-5}$-alkylamino, di-($C_{1-5}$-alkyl)-amino, $C_{2-4}$-alkanoylamino, N-($C_{1-3}$-alkyl)-$C_{2-4}$-alkanoylamino, pyrrolidino, dehydropyrrolidino, piperidino, dehydropiperidino, 3-hydroxypiperidino, 4-hydroxypiperidino, hexamethyleneimino, morpholino, thiomorpholino, piperazino, 4-($C_{1-3}$-alkyl)-piperazino, 4-phenyl-piperazino, 4-($C_{2-4}$-alkanoyl)-piperazino, 4-benzoyl-piperazino or imidazolyl group,

whilst the abovementioned saturated cycloalkyleneimino rings, $C_{1-5}$-alkylamino or di-($C_{1-5}$-alkyl)-amino groups may additionally be substituted by one or two $C_{1-5}$-alkyl groups, by a $C_{3-7}$-cycloalkyl, hydroxy, $C_{1-3}$-alkoxy, carboxy, $C_{1-3}$-alkoxycarbonyl, aminocarbonyl, $C_{1-3}$-alkylaminocarbonyl or di-($C_{1-3}$-alkyl)-aminocarbonyl group, by a phenyl-$C_{1-3}$-alkyl or phenyl group optionally mono- or disubstituted in the phenyl nucleus by fluorine, chlorine, bromine or iodine atoms or by $C_{1-3}$-alkyl or cyano groups, whilst the subetituents may be identical or different,

or a methylene group adjacent to the nitrogen atom in the abovementioned cycloalkyleneimino rings may be replaced by a carbonyl or. sulphonyl group, and the abovementioned monosubstituted phenyl groups may additionally be substituted by a fluorine, chlorine or bromine atom or by a methyl, amino, $C_{1-3}$-alkylamino or di-($C_{1-3}$-alkyl)-amino group, or

a phenyl ring optionally substituted by one or two $C_{1-3}$-alkoxy group may be fused to one of the abovementioned unsubstituted cycloalkyleneimino rings via two adjacent carbon atoms,
the isomers and the salts thereof.

**3.** Substituted indolinones of general formula I according to claim 1, wherein
X denotes an oxygen atom,
$R_1$ denotes a hydrogen atom,
$R_2$ denotes a carboxy, $C_{1-4}$-alkoxycarbonyl or aminocarbonyl. group wherein the amino moiety may be substituted by one or two $C_{1-3}$-alkyl groups and the substituents may be identical or different,
$R_3$ denotes a phenyl group optionally substituted by a methyl group,
$R_4$ denotes a hydrogen atom or a methyl group and
$R_5$ denotes a hydrogen atom,
a $C_{1-3}$-alkyl group, a benzyl group or a methyl or ethyl group substituted by a carboxy or $C_{1-3}$-alkoxycarbonyl group,
a $C_{3-7}$-cycloalkyl group optionally substituted by a methyl group,
an indanyl, pyridyl, oxazolyl, thiazolyl or imidazolyl group optionally substituted by a methyl group, to which a phenyl ring may additionally be fused via two adjacent carbon atoms,
a methylphenyl group optionally substituted by a fluorine, chlorine or bromine atom, or by a methoxy, carboxy,

$C_{1-3}$-alkyloxycarbonyl, nitro or aminosulphonyl group, or a dimethoxyphenyl group,
a 3-pyrrolidinyl or 4-piperidinyl group which may be substituted at the nitrogen atom by a $C_{1-3}$-alkyl group,
a phenyl group which is substituted
by a trifluoromethoxy, benzyloxy, cyano or nitro group, by a fluorine, chlorine of bromine atom,
by a $C_{1-3}$-alkoxy group, whilst the ethoxy and n-propoxy groups may each be terminally substituted by a dimethylamino, diethylamino, N-ethyl-methylamino, N-benzylmethylamino or piperidino group,

by a phenyl-$C_{1-3}$-alkylamino-$C_{1-3}$-alkyl group which may be substituted in the phenyl nucleus by a fluorine, chlorine, bromine or iodine atom, by a methyl, methoxy or trifluoromethyl group and additionally at the amine nitrogen atom by a $C_{1-5}$-alkyl or 2,2,2-trifluoroethyl group,

by a $C_{1-4}$-alkyl, phenyl, imidazolyl, cyclohexyl, methoxymethyl, carboxymethyl, $C_{1-3}$-alkoxycarbonyl-methyl, carboxy, $C_{1-3}$-alkoxycarbonyl, aminocarbonyl, $C_{1-3}$-alkylaminocarbonyl, di-($C_{1-3}$-alkyl)-aminocarbonyl, phenyl-$C_{1-3}$-alkylaminocarbonyl, N-($C_{1-3}$-alkyl)-phenyl-$C_{1-3}$-alkylaminocarbonyl, piperazinocarbonyl, N-($C_{1-3}$-alkyl)-piperazinocarbonyl, amino, $C_{1-3}$-alkylamino, di-($C_{1-3}$-alkyl)-amino, pyrrolidino, piperidino, morpholino, $C_{2-4}$-alkanoylamino, N-($C_{1-3}$-alkyl)-$C_{2-4}$-alkanoylamino, benzoylamino or N-($C_{1-3}$-alkyl)-benzoylamino group,
by an N-($C_{1-3}$-alkyl)-$C_{2-4}$-alkanoylamino group which is additionally substituted in the alkyl moiety by a carboxy or $C_{1-3}$-alkoxycarbonyl group,

by a $C_{1-3}$-alkylaminocarbonyl or di-($C_{1-3}$-alkyl)-aminocarbonyl group wherein an alkyl moiety is additionally substituted by a di-($C_{1-3}$-alkyl)-amino group, or

by an N-($C_{1-3}$-alkyl)-$C_{1-3}$-alkylsulphonylamino or N-($C_{1-3}$-alkyl)-phenylsulphonylamino group wherein the alkyl moiety may additionally be substituted by a cyano, carboxy, $C_{1-3}$-alkoxycarbonyl, $C_{1-3}$-alkylamino, di- ($C_{1-3}$-alkyl) -amino group, aminocarbonyl, $C_{1-3}$-alkylaminocarbonyl, di-($C_{1-3}$-alkyl)-aminocarbonyl, piperidinocarbonyl or 2-[di-($C_{1-3}$-alkylamino)]-ethylaminocarbonyl group,
a phenyl group optionally substituted by a $C_{1-3}$-alkyl group wherein the alkyl moiety is substituted by a hydroxy, $C_{1-3}$-alkoxy, carboxy, $C_{1-3}$-alkoxy-carbonyl, amino, $C_{1-5}$-alkylamino, di-($C_{1-5}$-alkyl)-amino, $C_{2-4}$-alkanoylamino, N-($C_{1-3}$-alkyl)-$C_{2-4}$-alkanoylamino, pyrrolidino, dehydropyrrolidino, piperidino, dehydropiperidino, 4-hydroxypiperidino, hexamethyleneimino, morpholino, thiomorpholino, piperazino, 4-($C_{1-3}$-alkyl)-piperazino, 4-phenyl-piperazino, 4- ($C_{2-4}$-alkanoyl)-piperazino, 4-benzoyl-piperazino or imidazolyl group,

whilst the abovementioned saturated cycloalkyleneimino rings may additionally be substituted by a phenyl group or by one or two methyl groups,
the abovementioned $C_{1-5}$-alkylamino and di-($C_{1-5}$-alkyl)-amino groups may additionally be substituted by one or two $C_{1-3}$-alkyl groups, by a cyclohexyl, hydroxy, $C_{1-3}$-alkoxy, carboxy, $C_{1-3}$-alkoxycarbonyl, aminocarbonyl, $C_{1-3}$-alkylaminocarbonyl or di-($C_{1-3}$-alkyl)-aminocarbonyl group, by a phenyl-$C_{1-3}$-alkyl or phenyl group optionally substituted in the phenyl nucleus by a fluorine, chlorine, bromine or iodine atom or by a methyl or cyano group,

or a methylene group adjacent to the nitrogen atom in the abovementioned cycloalkyleneimino rings may be replaced by a carbonyl or sulphonyl group, and the abovementioned monosubstituted phenyl groups may additionally be substituted by a fluorine, chlorine or bromine atom or by a methyl, amino, $C_{1-3}$-alkylamino or di-($C_{1-3}$-alkyl)-amino group, or

a phenyl ring optionally substituted by one or two $C_{1-3}$-alkoxy groups may be fused to one of the abovementioned unsubstituted cycloalkyleneimino rings via two adjacent carbon atoms,
the isomers and salts thereof.

**4.** Substituted indolinones of general formula I according to claim 1, wherein
X denotes an oxygen atom,
$R_1$ denotes a hydrogen atom,
$R_2$ denotes a carboxy or aminocarbonyl group wherein the amino moiety may be substituted by one or two $C_{1-3}$-alkyl groups and the substituents may be identical or different,
$R_3$ denotes a phenyl group optionally substituted by a methyl group,
$R_4$ denotes a hydrogen atom and
$R_5$ denotes a hydrogen atom,
a 3-pyrrolidinyl or 4-piperidinyl group which may be substituted at the nitrogen atom by a $C_{1-3}$-alkyl group,
a phenyl group which is substituted
by a $C_{1-3}$-alkoxy group, whilst the ethoxy and n-propoxy groups may each be terminally substituted by a dimethylamino, diethylamino, N-ethyl-methylamino, N-benzylmethylamino or piperidino group,
by a phenyl-$C_{1-3}$-alkylamino-$C_{1-3}$-alkyl group which may be substituted in the phenyl nucleus by a fluorine, chlorine, bromine or iodine atom, by a methyl, methoxy or trifluoromethyl group and additionally at the amine nitrogen atom by a $C_{1-5}$-alkyl or 2,2,2-trifluoroethyl group,
a phenyl group optionally substituted by a $C_{1-3}$-alkyl group wherein the alkyl moiety is substituted by a hydroxy, $C_{1-3}$-alkoxy, carboxy, $C_{1-3}$-alkoxy-carbonyl, amino, $C_{1-5}$-alkylamino, di-($C_{1-5}$-alkyl)-amino, $C_{2-4}$-alkanoylamino,

N-($C_{1-3}$-alkyl)-$C_{2-4}$-alkanoylamino, pyrrolidino, dehydropyrrolidino, piperidino, dehydropiperidino, 4-hydroxypiperidino, hexamethyleneimino, morpholino, thiomorpholino, piperazino, 4-($C_{1-3}$-alkyl)-piperazino, 4-phenyl-piperazino, 4-($C_{2-4}$-alkanoyl)-piperazino, 4-benzoyl-piperazino or imidazolyl group,

whilst the abovementioned saturated cycloalkyleneimino rings may additionally be substituted by a phenyl group or by one or two methyl groups,
the abovementioned $C_{1-5}$-alkylamino and di-($C_{1-5}$-alkyl)-amino groups may additionally be substituted by one or two $C_{1-3}$-alkyl groups, by a cyclohexyl, hydroxy, $C_{1-3}$-alkoxy, carboxy, $C_{1-3}$-alkoxycarbonyl, aminocarbonyl, $C_{1-3}$-alkylaminocarbonyl or di- ($C_{1-3}$-alkyl) -aminocarbonyl group, by a phenyl-$C_{1-3}$-alkyl or phenyl group optionally substituted in the phenyl nucleus by a fluorine, chlorine, bromine or iodine atom or by a methyl or cyano group,

or a methylene group adjacent to the nitrogen atom in the abovementioned cycloalkyleneimino rings may be replaced by a carbonyl or sulphonyl group, and the abovementioned monosubstituted phenyl groups may additionally be substituted by a fluorine, chlorine or bromine atom or by a methyl, amino, $C_{1-3}$-alkylamino or di-($C_{1-3}$-alkyl)-amino group, or

a phenyl ring optionally substituted by one or two $C_{1-3}$-alkoxy groups may be fused to one of the abovementioned unsubstituted cycloalkyleneimino rings via two adjacent carbon atoms,
the isomers and salts thereof.

**5.** Substituted indolinones of general formula I according to at least one of claims 1 to 4, wherein the group $R_2$ is in the 5 position.

**6.** The following substituted indolinones of general formula I according to claim 1:

(a) 3-Z- [1-(4-aminomethyl-phenylamino)-1-phenyl-methylene]-5-amido-2-indolinone,

(b) 3-Z- (1-phenylamino)-1-phenyl-methylene)-5-amido-2-indolinone,

(c) 3-Z-[1-(4-bromophenylamino)-1-phenyl-methylene]-5-amido-2-indolinone,

(d) 3-Z-[1-(4-dimethylamino-methyl)-phenylamino)-1-phenylmethylene]-5-amido-2-indolinone,

(e) 3-Z-[1-(4-pyrrolidinomethyl-phenylamino) -1-phenylmethylene]-5-amido-2-indolinone,

(f) 3-Z-[1-(4-piperidinomethyl-phenylamino),-1-phenylmethylene]-5-amido-2-indolinone,

(g) 3-Z-[1-(4-hexamethyleneiminomethyl-phenylamino)-1-phenyl-methylene]-5-amido-2-indolinone,

(h) 3-Z-[1-(4-(4-benzyl-piperidino)-methyl)-phenylamino)-1-phenyl-methylene]-5-amido-2-indolinone,

(i) 3-Z-[1-(4-(N-butyl-aminomethyl)-phenylamino)-1-phenylmethylene] -5-amido-2-indolinone,

(j) 3-Z-[1-(4- (N-(phenyl-methyl)-aminomethyl)-phenylamino)-1-phenyl-methylene]-5-amido-2-indolinone,

(k) 3-Z- [1-(4-(N-methyl-N-benzyl-amino-methyl)-phenylamino)-1-phenyl-methylene]-5-amido-2-indolinone,

(1) 3-Z- [1-(4-piperidino-methyl-phenylamino)-1-phenyl-methylene]-5-dimethylcarbamoyl-2-indolinorie,

(m) 3-Z-[1-(4-piperidino-methyl-phenylamino)-1-phenyl-methylene]-5-diethylcarbamoyl-2-indolinone,

(n) 3-Z-[1-(4-(3-diethylamino-propoxy)-phenylamino)-1-phenyl-methylene]-5-amido-2-indolinone

and the salts thereof.

**7.** Physiologically acceptable salts of the compounds according to claims 1 to 6.

**8.** Pharmaceutical compositions containing a compound according to at least one of claims 1 to 6 or a salt according to claim 7 optionally together with one or more inert carriers and/or diluents.

**9.** Use of a compound according to at least one of claims 1 to 6 or a salt according to claim 7 for preparing a pharmaceutical composition which is suitable for treating excessive or abnormal cell proliferation.

**10.** Process for preparing a pharmaceutical composition according to claim 8, **characterised in that** a compound according to at least one of claims 1 to 6 or a salt according to claim 7 is incorporated in one or more inert carriers and/or diluents by a non-chemical method.

**11.** Process for preparing the compounds according to claims 1 to 7, **characterised in that**

a. a compound of general formula

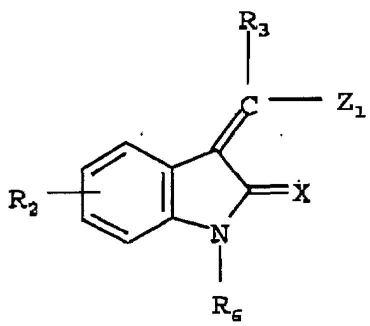

(II),

wherein
X, $R_2$ and $R_3$ are defined as mentioned in claims 1 to 6,
$R_6$ denotes a hydrogen atom, a protecting group for the nitrogen atom of the lactam group or a bond to a solid phase and
$Z_1$ denotes a halogen atom, a hydroxy, alkoxy or aralkoxy group,
is reacted with an amine of general formula

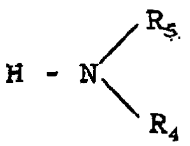

(III),

wherein
$R_4$ and $R_5$ are defined as in claims 1 to 6,
and subsequently, if necessary, any protecting group used for the nitrogen atom of the lactam group is cleaved or a compound thus obtained is cleaved from a solid phase or

b. in order to prepare a compound of general formula I which contains an aminomethyl group and where X denotes an oxygen atom, a compound of general formula

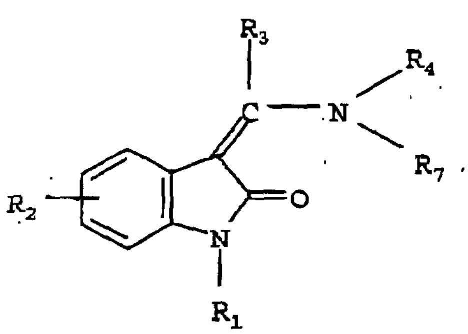

(IV),

wherein

$R_1$ to $R_4$ are defined as in claims 1 to 6 and

$R_7$ has the meanings given for $R_5$ in claims 1 to 6, with the proviso that $R_5$ contains a cyano group, is reduced and

subsequently if desired a compound of general formula I thus obtained which contains an alkoxycarbonyl group is converted by hydrolysis into a corresponding carboxy compound or

a compound of general formula I thus obtained which contains an amino or alkylamino group is converted by alkylation or reductive alkylation into a corresponding alkylamino or dialkylamino compound or

a compound of general formula I thus obtained which contains an amino or alkylamino group is converted by acylation into a corresponding acyl compound or

a compound of general formula I thus obtained which contains a carboxy group is converted by esterification or amidation into a corresponding ester or aminocarbonyl compound or

if necessary a protecting group used during the reactions to protect reactive groups is cleaved or

subsequently if desired a compound of general formula I thus obtained is resolved into the stereoisomers thereof or

a compound of general formula I thus obtained is converted into the salts thereof, particularly, for pharmaceutical use, into the physiologically acceptable salts thereof with an inorganic or organic acid or base.

## Revendications

**1.** Indolinones substituées de formule générale

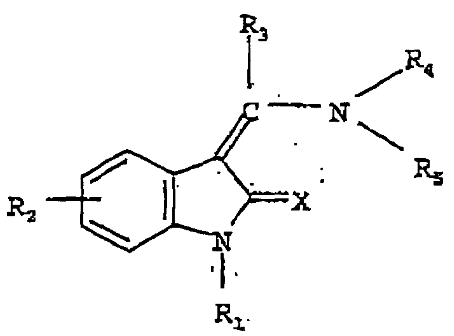

(I)

dans laquelle

X représente un atome d'oxygène ou de soufre,

$R_1$ représente un atome d'hydrogène, un reste (alcoxy en $C_1$ à $C_4$)-carbonyle ou alcanoyle en $C_2$ à $C_4$,

$R_2$ représente un reste carboxyle, (alcoxy en $C_1$ à $C_4$)-carbonyle ou aminocarbonyle dans lequel le groupe amino est éventuellement substitué par un ou deux restes alkyle en $C_1$ à $C_3$, les substituants pouvant être identiques ou différents,

$R_3$ représente un reste phényle ou naphtyle qui est éventuellement substitué par des atomes de fluor, de chlore ou de brome ou par des restes alkyle en $C_1$ à $C_3$, alcoxy en $C_1$ à $C_3$, cyano, trifluorométhyle, nitro, amino, (alkyle en $C_1$ à $C_3$)-amino, di-(alkyle en $C_1$ à $C_3$)-amino, (alcanoyle en $C_2$ à $C_4$)-amino, N-(alkyle en $C_1$ à $C_3$)-(alcanoyle en $C_2$ à $C_4$)-amino, (alkyle en $C_1$ à $C_3$)-sulfonylamino, amino-(alkyle en $C_1$ à $C_3$), (alkyle en $C_1$ à $C_3$)-amino-(alkyle en $C_1$ à $C_3$), di-(alkyle en $C_1$ à $C_3$)-amino-(alkyle en $C_1$ à $C_3$), N-(alcanoyle en $C_2$ à $C_4$)-amino-(alkyle en $C_1$ à $C_3$) ou N-(alcanoyle en $C_2$ à $C_4$)-(alkyle en $C_1$ à $C_3$)-amino-(alkyle en $C_1$ à $C_3$), les substituants pouvant être identiques ou différents,

$R_4$ représente un atome d'hydrogène ou un reste alkyle en $C_1$ à $C_3$, et

$R_5$ représente un atome d'hydrogène,

un reste alkyle en $C_1$ à $C_5$ qui est éventuellement substitué par un reste phényle, carboxyle ou (alcoxy en $C_1$ à $C_3$)-carbonyle,
un reste cycloalkyle en $C_3$ à $C_7$ qui est éventuellement substitué par un reste alkyle en $C_1$ à $C_3$,
un reste indanyle qui est éventuellement substitué par un reste alkyle en $C_1$ à $C_3$, un reste hétéroaryle à 5 maillons qui comprend un reste imino qui est éventuellement substitué par un reste alkyle en $C_1$ à $C_3$, un atome de soufre ou d'oxygène ou un reste imino qui est éventuellement, substitué par un reste alkyle en $C_1$ à $C_3$, et qui comprend un atome d'oxygène, de soufre ou d'azote ou deux atomes d'azote, ou bien représente un reste hétéroaryle à 6 maillons qui comprend 1 à 3 atomes d'azote, où il est possible de relier en plus, par l'intermédiaire de deux atomes de carbone vicinaux ou par l'intermédiaire d'un atome de carbone et un reste imino vicinal appartenant aux restes hétéroaryle à 5 ou à 6 maillons susmentionnés, un pont butadiényle en position 1,3, et où le squelette carboné appartenant aux cycles monocycliques ou bicycliques susmentionnés est éventuellement mono- ou disubstitué par des atomes de fluor, de chlore, de brome ou d'iode ou par des restes alkyle en $C_1$ à $C_5$ ou cyano, les substituants pouvant être identiques ou différents,
un reste pyrrolidinyle ou pipéridinyle relié par un atome de carbone, qui est éventuellement substitué, à chaque fois au niveau de l'atome d'azote, par un reste alkyle en $C_1$ à $C_3$,
un reste phényle qui est éventuellement disubstitué par des atomes de fluor, de chlore, de brome ou d'iode ou par des restes alkyle en $C_1$ à $C_5$, alcoxy en $C_1$ à $C_3$, carboxyle, (alcoxy en $C_1$ à $C_3$)-carbonyle, aminosulfonyle, nitro ou cyano, les substituants pouvant être identiques ou différents,
un reste phényle, pyridyle, pyrimidyle ou thiényle, chacun étant substitué

par un reste trifluorométhoxy, par un atome de fluor, de chlore, de brome ou d'iode,

par un reste alcoxy en $C_1$ à $C_3$ qui est éventuellement substitué, en position 2 ou 3, par un reste amino, (alkyle en $C_1$ à $C_3$)-amino, di-(alkyle en $C_1$ à $C_3$)-amino, phényl-(alkyle en $C_1$ à $C_3$)-amino, N-(alkyle en $C_1$ à $C_3$)-phényl(alkyle en $C_1$ à $C_3$)-amino, pyrrolidino ou pipéridino,

par un reste phényl-(alkyle en $C_1$ à $C_3$)-amino-(alkyle en $C_1$ à $C_3$) qui est éventuellement mono- ou disubstitué, au niveau du noyau phényle, par un reste trifluorométhyle, par des atomes de fluor, de chlore, de brome ou d'iode, par des restes alkyle en $C_1$ à $C_5$ ou alcoxy en $C_1$ à $C_3$, les substituants pouvant être identiques ou différents, et, qui peut être en plus substitué au niveau de l'atome d'azote de l'amine, par un reste alkyle en $C_1$ à $C_3$ dans lequel les atomes d'hydrogène, à partir de la position 2, peuvent être, complètement ou partiellement, remplacés par des atomes de fluor,

par un reste alkyle en $C_1$ à $C_5$, phényle; imidazolyle, cycloalkyle en $C_3$ à $C_7$, (alcoxy en $C_1$ à $C_3$)-(alcoxy en $C_1$ à $C_3$), phényl-(alcoxy en $C_1$ à $C_3$), carboxy-(alkyle en $C_1$ à $C_3$), (alcoxy en $C_1$ à $C_3$)-carbonyl-(alkyle en $C_1$ à $C_3$), carboxyle, (alcoxy en $C_1$ à $C_3$)-carbonyle, aminocarbonyle, (alkyle en $C_1$ à $C_3$)-aminocarbonyle, di-(alkyle en $C_1$ à $C_3$)-aminocarbonyle, phényl(alkyle en $C_1$ à $C_3$)-aminocarbonyle, N-(alkyle en $C_1$ à $C_3$)-phényl-(alkyle en $C_1$ à $C_3$)-aminocarbonyle, pipérazinocarbonyle, N-(alkyle en $C_1$ à $C_3$)-pipérazinocarbonyle, nitro, amino, (alkyle en $C_1$ à $C_3$)-amino, di-(alkyle en $C_1$ à $C_3$)-amino, pyrrolidino, pipéridino, morpholino, (alcanoyle en $C_2$ à $C_4$)-amino, N-(alkyle en $C_1$ à $C_3$)-(alcanoyle en $C_2$ à $C_4$)-amino, benzoylamino ou N-(alkyle en $C_1$ à $C_3$)-benzoylamino,

par un reste N-(alkyle en $C_1$ à $C_3$)-(alcanoyle en $C_2$ à $C_4$)-amino qui est en plus substitué, au niveau de son groupe alkyle, par un reste carboxyle ou (alcoxy en $C_1$ à $C_3$)-carbonyle,

par un reste (alkyle en $C_1$ à $C_3$)-aminocarbonyle ou di-(alkyle en $C_1$ à $C_3$)-aminocarbonyle dans lesquels un groupe alkyle est en plus substitué par un reste di-(alkyle en $C_1$ à $C_3$)-amino, ou

par un reste N-(alkyle en $C_1$ à $C_3$)-(alkyle en $C_1$ à $C_3$)-sulfonylamino ou N-(alkyle en $C_1$ à $C_3$)-phénylsulfonylamino dans lesquels le groupe alkyle peut être en plus substitué par un reste cyano, carboxyle, (alcoxy en $C_1$ à $C_3$)-carbonyle, (alkyle en $C_1$ à $C_3$)-amino, di-(alkyle en $C_1$ à $C_3$)-amino, aminocarbonyle, (alkyle en $C_1$ à $C_3$)-aminocarbonyle, di-(alkyle en $C_1$ à $C_3$)-aminocarbonyle, pipéridinocarbonyle ou 2-[di-((alkyle en $C_1$ à $C_3$)-amino)]-éthylaminocarbonyle,

un reste phényle ou thiényle qui est substitué par un reste alkyle en $C_1$ à $C_3$ et dans lequel le groupe alkyle est substitué par un reste hydroxy, alcoxy en $C_1$ à $C_3$, carboxyle, (alcoxy en $C_1$ à $C_3$)-carbonyle, amino, (alkyle en $C_1$ à $C_5$)-amino, di-(alkyle en $C_1$ à $C_5$)-amino, (alcanoyle en $C_2$ à $C_4$)-amino, N-(alkyle en $C_1$ à $C_3$)-(alcanoyle en $C_2$ à $C_4$)-amino, pyrrolidino, déhydropyrrolidino, pipéridino, déhydropipéridino, 3-hydroxypipéridino, 4-hydroxypipéridino, hexaméthylène-imino, morpholino, thiomorpholino, pipérazino, 4-(alkyle en $C_1$ à $C_3$)-pipérazino, 4-phénylpipérazino, 4-(alcanoyle en $C_2$ à $C_4$)-pipérazino, 4-benzoylpipérazino ou imidazolyle,

où les restes (alkyle en $C_1$ à $C_5$)-amino, di-(alkyle en $C_1$ à $C_5$)-amino ou les cycles cycloalkylène-imino saturés susmentionnés peuvent être en plus substitués par un ou deux restes alkyle en $C_1$ à $C_5$, par un reste cycloalkyle en $C_3$ à $C_7$, hydroxy, alcoxy en $C_1$ à $C_3$, carboxyle, (alcoxy en $C_1$ à $C_3$)-carbonyle, aminocarbonyle, (alkyle en $C_1$ à $C_3$)-aminocarbonyle ou di-(alkyle en $C_1$ à $C_3$)-aminocarbonyle, par un reste phényl-(alkyle en $C_1$ à $C_3$) ou phényle qui est éventuellement mono- ou disubstitué au niveau de son noyau phényle, par des atomes de fluor, de chlore, de brome ou d'iode, par des restes alkyle en $C_1$ à $C_3$ ou cyano, les substituants pouvant être identiques ou différents,

ou bien où un reste méthylène qui, dans les cycles cycloalkylène-imino susmentionnés, est voisin de l'atome d'azote, est éventuellement remplacé par un reste carbonylé ou sulfonyle et où les restes phényle monosubstitués susmentionnés peuvent être en plus substitués par un atome de fluor, de chlore ou de brome, un reste méthyle, amino, (alkyle en $C_1$ à $C_3$)-amino ou di-(alkyle en $C_1$ à $C_3$)-amino, ou bien

où un cycle phényle qui est éventuellement substitué par un ou par deux restes alcoxy en $C_1$ à $C_3$, peut être relié par condensation et par l'intermédiaire de deux atomes de carbone vicinaux, à un des cycles cycloalkylène-imino non substitués susmentionnés,

où les restes carboxyle que l'on a mentionnés dans la définition des restes susmentionnés, sont en outre remplacés par un reste apte à une transformation in vivo en un reste carboxyle, et

où les restes amino et imino que l'on a mentionnés dans la définition des restes susmentionnés, peuvent être en outre substitués par un reste apte à une dissociation in vivo,

leurs isomères et leurs sels.

**2.** Indolinones substituées de formule générale 1 selon la revendication 1, dans lesquelles

X représente un atome d'oxygène,

$R_1$ représente un atome d'hydrogène ou un reste (alcoxy en $C_1$ à $C_4$)-carbonyle,

$R_2$ représente un reste carboxyle, (alcoxy en $C_1$ à $C_4$)-carbonyle ou aminocarbonyle dans lequel le groupe amino est éventuellement substitué par un ou deux restes alkyle en $C_1$ à $C_3$, les substituants pouvant être identiques ou différents,

$R_3$ représente un reste phényle qui est éventuellement substitué par un atome de fluor, de chlore ou de brome ou par un reste méthyle, cyano ou aminométhyle,

$R_4$ représente un atome d'hydrogène ou un reste méthyle, et

$R_5$ représente un arôme d'hydrogène,

un reste benzyle ou un reste alkyle en $C_1$ à $C_5$ qui est éventuellement substitué par un reste carboxyle ou (alcoxy en $C_1$ à $C_3$)-carbonyle,

un reste cycloalkyle en $C_3$ à $C_7$ qui est éventuellement substitué par un reste méthyle,

un reste indanyle, pyridyle, oxazolyle, thiazolyle ou imidazolyle, éventuellement substitué par un reste méthyle, qui peut être en outre relié, par condensation et par l'intermédiaire de deux atomes de carbone vicinaux, à un cycle phényle,

un reste diméthoxyphényle ou méthylphényle qui est éventuellement substitué par un atome de fluor, de chlore ou de brome ou par des restes méthoxy, carboxyle, (alkyle en $C_1$ à $C_3$)-oxycarbonylé, nitro ou aminosulfonyle,

un reste pyrrolidinyle ou pipéridinyle relié par un atome de carbone qui est substitué, à chaque fois au niveau de l'atome d'azote, par un reste alkyle en $C_1$ à $C_3$,

un reste phényle qui est substitué

par un reste trifluorométhoxy, par un atome de fluor, de chlore, de brome ou d'iode,

par un reste alcoxy en $C_1$ à $C_3$ qui peut être substitué, en position 2 ou 3, par un reste amino, (alkyle en $C_1$ à $C_3$)-amino, di-(alkyle en $C_1$ à $C_3$)-amino, phényl-(alkyle en $C_1$ à $C_3$)-amino, N-(alkyle en $C_1$ à $C_3$)-phényl-(alkyle en $C_1$ à $C_3$)-amino, pyrrolidino ou pipéridino,

par un reste phényl-(alkyle en $C_1$ à $C_3$)-amino-(alkyle en $C_1$ à $C_3$) qui peut être substitué, au niveau de son noyau phényle, par un atome de fluor, de chlore, de brome ou d'iode, par un reste alkyle en $C_1$ à $C_5$, alcoxy en $C_1$ à $C_3$ ou trifluorométhyle, et, qui, en outre, peut être substitué au niveau de l'atome d'azote de l'amine, par un reste alkyle en $C_1$ à $C_3$ dans lequel les atomes d'hydrogène, à partir de la position 2, peuvent être, complètement ou partiellement, remplacés par des atomes de fluor,

par un reste alkyle en $C_1$ à $C_5$, phényle, imidazolyle, cycloalkyle en $C_3$ à $C_7$, (alcoxy en $C_1$ à $C_3$)-(alcoxy en $C_1$ à $C_3$), phényl-(alcoxy en $C_1$ à $C_3$), carboxy-(alkyle en $C_1$ à $C_3$), (alcoxy en $C_1$ à $C_3$)-carbonyl-(alkyle en $C_1$ à $C_3$), carboxyle, (alcoxy en $C_1$ à $C_3$)-carbonyle, aminocarbonyle, (alkyle en $C_1$ à $C_3$)-aminocarbonyle, di-(alkyle en $C_1$ à $C_3$)-aminocarbonyle, phényl(alkyle en $C_1$ à $C_3$)-aminocarbonyle, N-(alkyle en $C_1$ à $C_3$)-phényl-(alkyle en $C_1$ à $C_3$)-aminocarbonyle, pipérazinocarbonyle, N-(alkyle en $C_1$ à $C_3$)-pipérazinocarbonyle, nitro, amino, (alkyle en $C_1$ à $C_3$)-amino, di-(alkyle en $C_1$ à $C_3$)-amino, pyrrolidino; pipéridino, morpholino, (alcanoyle en $C_2$ à $C_4$)-amino, N-(alkyle en $C_1$ à $C_3$)-(alcanoyle en $C_2$ à $C_4$)-amino, benzoylamino ou N-(alkyle en $C_1$ à $C_3$)-benzoylamino,

par un reste N-(alkyle en $C_1$ à $C_3$)-(alcanoyle en $C_2$ à $C_4$)-amino qui est en plus substitué, au niveau de son

groupe alkyle, par un reste carboxyle ou (alcoxy en $C_1$ à $C_3$)-carbonyle,

par un reste (alkyle en $C_1$ à $C_3$)-aminocarbonyle ou di-(alkyle en $C_1$ à $C_3$)-aminocarbonyle dans lesquels un groupe alkyle est en plus substitué par un reste di-(alkyle en $C_1$ à $C_3$)-amino, ou

par un reste N-(alkyle en $C_1$ à $C_3$)-(alkyle en $C_1$ à $C_3$)-sulfonylamino ou N-(alkyle en $C_1$ à $C_3$)-phénylsulfonylamino dans lesquels le groupe alkyle peut être en plus substitué par un reste cyano, carboxyle, (alcoxy en $C_1$ à $C_3$)-carbonyle, (alkyle en C1 à $C_3$)-amino, di-(alkyle en $C_1$ à $C_3$)-amino, aminocarbonyle, (alkyle en $C_1$ à $C_3$)-aminocarbonyle, di-(alkyle en $C_1$ à $C_3$)-aminocarbonyle, pipéridinocarbonyle ou 2-[di-((alkyle en $C_1$ à $C_3$)-amino)]-éthylaminocarbonyle,

un reste phényle qui est éventuellement substitué par un reste alkyle en $C_1$ à $C_3$ et dans lequel le groupe alkyle est substitué par un reste hydroxy, alcoxy en $C_1$ à $C_3$, carboxyle, (alcoxy en $C_1$ à $C_3$)-carbonyle, amino, (alkyle en $C_1$ à $C_5$)-amino, di-(alkyle en $C_1$ à $C_5$)-amino, (alcanoyle en $C_2$ à $C_4$)-amino, N-(alkyle en $C_1$ à $C_3$)-(alcanoyle en $C_2$ à $C_4$)-amino, pyrrolidino, déhydropyrrolidino, pipéridino, déhydropipéridino, 3-hydroxypipéridino, 4-hydroxypipéridino, hexaméthylène-imino, morpholino, thiomorpholino, pipérazino, 4-(alkyle en $C_1$ à $C_3$)-pipérazino, 4-phénylpipérazino, 4-(alcanoyle en $C_2$ à $C_4$)-pipérazino, 4-benzoylpipérazino ou imidazolyle,

où les restes (alkyle en $C_1$ à $C_5$)-amino, di-(alkyle en $C_1$ à $C_5$)-amino ou les cycles cycloalkylène-imino saturés susmentionnés peuvent être en plus substitués par un ou deux restes alkyle en $C_1$ à $C_5$, par un reste cycloalkyle en $C_3$ à $C_7$, hydroxy, alcoxy en $C_1$ à $C_3$, carboxyle, (alcoxy en $C_1$ à $C_3$)-carbonyle, aminocarbonyle, (alkyle en $C_1$ à $C_3$)-aminocarbonyle ou di-(alkyle en $C_1$ à $C_3$)-aminocarbonyle, par un reste phényl-(alkyle en $C_1$ à $C_3$) ou phényle éventuellement mono- ou disubstitué au niveau de son noyau phényle, par des atomes de fluor, de chlore, de brome ou d'iode, par des restes alkyle en $C_1$ à $C_3$ ou cyano, les substituants pouvant être identiques ou différents,

ou bien où un reste méthylène qui, dans les cycles cycloalkylène-imino susmentionnés, est voisin de l'atome d'azote, peut être remplacé par un reste carbonyle ou sulfonyle et où les restes phényle monosubstitués susmentionnés peuvent être en plus substitués par un atome de fluor, de chlore ou de brome, un reste méthyle, amino, (alkyle en $C_1$ à $C_3$)-amino ou di-(alkyle en $C_1$ à $C_3$)-amino, ou bien

où un cycle phényle qui est éventuellement substitué par un ou par deux restes alcoxy en $C_1$ à $C_3$, est relié par condensation et par l'intermédiaire de deux atomes de carbone vicinaux, à un des cycles cycloalkylène-imino non substitués susmentionnés, leurs isomères et leurs sels.

**3.** Indolinones substituées de formule générale I selon la revendication 1, dans lesquelles

X représente un atome d'oxygène,

$R_1$ représente un atome d'hydrogène,

$R_2$ représente un reste carboxyle, (alcoxy en $C_1$ à $C_4$)-carbonyle ou aminocarbonyle dans lequel le groupe amino peut être substitué par un ou deux restes alkyle en $C_1$ à $C_3$, les substituants pouvant être identiques ou différents,

$R_3$ représente un reste phényle éventuellement substitué par un reste méthyle,

$R_4$ représente un atome d'hydrogène ou un reste méthyle, et

$R_5$ représente un atome d'hydrogène,

un reste alkyle en $C_1$ à $C_3$, un reste benzyle ou un reste méthyle ou éthyle qui est substitué par un reste carboxyle ou (alcoxy en $C_1$ à $C_3$)-carbonyle,

un reste cycloalkyle en $C_3$ à $C_7$ qui est éventuellement substitué par un reste méthyle,

un reste indanyle, pyridyle, oxazolyle, thiazolyle ou imidazolyle qui est éventuellement substitué par un reste méthyle et qui peut être en plus relié, a chaque fois par condensation et par l'intermédiaire de deux atomes de carbone vicinaux, à un cycle phényle,

un reste diméthoxyphényle ou méthylphényle qui est éventuellement substitué par un atome de fluor, de chlore ou de brome ou par un reste méthoxy, carboxyle, (alkyle en $C_1$ à $C_3$)-oxycarbonyle, nitro ou aminosulfonyle,

un reste 3-pyrrolidinyle ou 4-pipéridinyle qui est substitué, à chaque fois au niveau de l'atome d'azote, par un reste allyle en $C_1$ à $C_3$,

un reste phényle qui est substitué

par un reste trifluorométhoxy, benzyloxy, cyano ou nitro, par un atome de fluor, de chlore ou de brome,

par un reste alcoxy en $C_1$ à $C_3$, où le reste éthoxy ou n-propoxy peut être substitué, à chaque fois en position

terminale, par un reste diméthylamino, diéthylamino, N-éthylméthylamino, N-benzylméthylamino ou pipéridino,

par un reste phényl-(alkyle en $C_1$ à $C_3$)-amino-(alkyle en $C_1$ à $C_3$) qui peut être substitué, au niveau de son noyau phényle, par un atome de fluor, de chlore, de brome ou d'iode, par un reste méthyle, méthoxy ou trifluorométhyle, et, qui peut être en plus substitué au niveau de l'atome d'azote de l'amine, par un reste alkyle en $C_1$ à $C_5$ ou 2,2,2-trifluoroéthyle,

par un reste alkyle en $C_1$ à $C_4$, phényle, imidazolyle, cyclohexyle; méthoxyméthyle, carboxyméthyle, (alcoxy en $C_1$ à $C_3$)-carbonylméthyle, carboxyle, (alcoxy en $C_1$ à $C_3$)-carbonyle, aminocarbonyle, (alkyle en $C_1$ à $C_3$)-aminocarbonyle, di-(alkyle en $C_1$ à $C_3$)-aminocarbonyle, phényl-(alkyle en $C_1$ à $C_3$)-aminocarbonyle, N-(alkyle en $C_1$ à $C_3$)-phényl-(alkyle en $C_1$ à $C_3$)-aminocarbonyle, pipérazinocarbonyle, N-(alkyle en $C_1$ à $C_3$)-pipérazinocarbonyle, amino, (alkyle en $C_1$ à $C_3$)-amino, di-(alkyle en $C_1$ à $C_3$)-amino, pyrrolidino, pipéridino, morpholino, (alcanoyle en $C_2$ à $C_4$)-amino, N-(alkyle en $C_1$ à $C_3$)-(alcanoyle en $C_2$ à $C_4$)-amino, benzoylamino ou N-(alkyle en $C_1$ à $C_3$)-benzoylamino,

par un reste N-(alkyle en $C_1$ à $C_3$)-(alcanoyle en $C_2$ à $C_4$)-amino qui est en plus substitué, au niveau de son groupe alkyle, par un reste carboxyle ou (alcoxy en $C_1$ à $C_3$)-carbonyle,

par un reste (alkyle en $C_1$ à $C_3$)-aminocarbonyle ou di-(alkyle en $C_1$ à $C_3$)-aminocarbonyle dans lesquels un groupe alkyle est en plus substitué par un reste di-(alkyle en $C_1$ à $C_3$)-amino, ou

par un reste N-(alkyle en $C_1$ à $C_3$)-(alkyle en $C_1$ à $C_3$)-sulfonylamino ou N-(alkyle en $C_1$ à $C_3$)-phénylsulfonylamino dans lesquels le groupe alkyle peut être en plus substitué par un reste cyano, carboxyle, (alcoxy en $C_1$ à $C_3$)-carbonyle, (alkyle en $C_1$ à $C_3$)-amino, di-(alkyle en $C_1$ à $C_3$)-amino, aminocarbonyle, (alkyle en $C_1$ à $C_3$)-aminocarbonyle, di-(alkyle en $C_1$ à $C_3$)-aminocarbonyle, pipéridinocarbonyle ou 2-[di-((alkyle en $C_1$ à $C_3$)-amino)]-éthylaminocarbonyle,

un reste phényle qui est éventuellement substitué par un reste alkyle en $C_1$ à $C_3$ et dans lequel le reste alkyle est substitué par un reste hydroxy, alcoxy en $C_1$ à $C_3$, carboxyle, (alcoxy en $C_1$ à $C_3$)-carbonyle, amino, (alkyle en $C_1$ à $C_5$)-amino, di-(alkyle en $C_1$ à $C_5$)-amino, (alcanoyle en $C_2$ à $C_4$)-amino, N-(alkyle en $C_1$ à $C_3$)-(alcanoyle en $C_2$ à $C_4$)-amino, pyrrolidino, déhydropyrrolidino, pipéridino, déhydropipéridino, 4-hydroxypipéridino, hexaméthylène-imino, morpholino, thiomorpholino, pipérazino, 4-(alkyle en $C_1$ à $C_3$)-pipérazino, 4-phénylpipérazino, 4-(alcanoyle en $C_2$ à $C_4$)-pipérazino, 4-benzoylpipérazino ou imidazolyle,

où les cycles cycloalkylène-imino saturés susmentionnés peuvent être en plus substitués par un reste phényle ou par un ou deux restes méthyle,

où les restes (alkyle en $C_1$ à $C_5$)-amino et di-(alkyle en $C_1$ à $C_5$)-amino susmentionnés peuvent être en plus substitués par un ou deux restes alkyle en $C_1$ à $C_3$, par un reste cyclohexyle, hydroxy, alcoxy en $C_1$ à $C_3$, carboxyle, (alcoxy en $C_1$ à $C_3$)-carbonyle, aminocarbonyle, (alkyle en $C_1$ à $C_3$)-aminocarbonyle ou di-(alkyle en $C_1$ à $C_3$)-aminocarbonyle, par un reste phényl-(alkyle en $C_1$ à $C_3$) ou phényle qui est éventuellement substitué, au niveau de son noyau phényle, par un atome de fluor, de chlore, de brome ou d'iode, par un reste méthyle ou cyano,

ou bien où un reste méthylène qui, dans les cycles cycloalkylène-imino susmentionnés, est voisin de l'atome d'azote, peut être remplacé par un reste carbonyle ou sulfonyle et où les restes phényle monosubstitués susmentionnés peuvent être en plus substitués par un atome de fluor, de chlore ou de brome, un reste méthyle, amino, (alkyle en $C_1$ à $C_3$)-amino ou di-(alkyle en $C_1$ à $C_3$)-amino, ou bien

où un cycle phényle qui est éventuellement substitué par un ou par deux restes alcoxy en $C_1$ à $C_3$, peut être relié par condensation et par l'intermédiaire de deux atomes de carbone vicinaux, à un des cycles cycloalkylène-imino non substitués susmentionnés,

leurs isomères et leurs sels.

**4.** Indolinones substituées de formule générale I selon la revendication 1, dans lesquelles

X représente un atome d'oxygène,

$R_1$ représente un atome d'hydrogène,

$R_2$ représente un reste carboxyle ou aminocarbonyle dans lequel le groupe amino peut être substitué par un ou deux restes alkyle en $C_1$ à $C_3$, les substituants pouvant être identiques ou différents,

$R_3$ représente un reste phényle éventuellement substitué par un reste méthyle,

$R_4$ représente un atome d'hydrogène et

$R_5$ représente un atome d'hydrogène,

un reste 3-pyrrolidinyle ou 4-pipéndinyle qui est substitué, à chaque fois au niveau de l'atome d'azote, par un reste alkyle en $C_1$ à $C_3$,
un reste phényle qui est substitué
par un reste alcoxy en $C_1$ à $C_3$, où le reste éthoxy et n-propoxy peuvent être substitués, à chaque fois en position terminale, par un reste diméthylamino, diéthylamino, N-éthylméthylamino, N-benzylméthylamino on pipéridino,
par un reste phényl-(alkyle en $C_1$ à $C_3$)-amino-(alkyle en $C_1$ à $C_3$) qui peut être substitué, au niveau de son noyau phényle, par un atome de fluor, de chlore, de brome ou d'iode, par un reste méthyle, méthoxy ou trifluorométhyle, et, qui peut être en plus substitué au niveau de l'atome d'azote de l'amine, par un reste alkyle en $C_1$ à $C_5$ ou 2,2,2-trifluoroéthyle,
un reste phényle qui est éventuellement substitué par un reste alkyle en $C_1$ à $C_3$ et dans lequel le groupe alkyle est substitué par un reste hydroxy, alcoxy en $C_1$ à $C_3$, carboxyle, (alcoxy en $C_1$ à $C_3$)-carbonyle, amino, (alkyle en $C_1$ à $C_5$)-amino, di-(alkyle en $C_1$ à $C_5$)-amino, (alcanoyle en $C_2$ à $C_4$)-amino, N-(alkyle en $C_1$ à $C_3$)-(alcanoyle en $C_2$ à $C_4$)-amino, pyrrolidino, déhydropyrrolidino, pipéridino, déhydropipéridino, 4-hydroxypipéridino, hexaméthylène-imino, morpholino, thiomorpholino, pipérazino, 4-(alkyle en $C_1$ à $C_3$)-pipérazino, 4-phénylpipérazino, 4-(alcanoyle en $C_2$ à $C_4$)-pipérazino, 4-benzoylpipérazino ou imidazolyle,
où les cycles cycloalkylène-imino saturés susmentionnés peuvent être en plus substitués par un reste phényle ou par un ou deux restes méthyle,
où les restes (alkyle en $C_1$ à $C_5$)-amino et di-(alkyle en $C_1$ à $C_5$)-amino peuvent être en plus substitués par un ou deux restes alkyle en $C_1$ à $C_3$, par un reste cyclohexyle, hydroxy, alcoxy en $C_1$ à $C_3$, carboxyle, (alcoxy en $C_1$ à $C_3$)-carbonyle, aminocarbonyle, (alkyle en $C_1$ à $C_3$)-aminocarbonyle ou di-(alkyle en $C_1$ à $C_3$)-aminocarbonyle, par un reste phényl-(alkyle en $C_1$ à $C_3$) ou phényle qui est éventuellement substitué, au niveau de son noyau phényle, par un atome de fluor, de chlore, de brome ou d'iode, par un reste méthyle ou cyano,
ou bien où un reste méthylène qui, dans les cycles cycloalkylène-imino susmentionnés, est voisin de l'atome d'azote, peut être remplacé par un reste carbonyle ou sulfonyle, et où les restes phényle monosubstitués susmentionnés peuvent être en plus substitués par un atome de fluor, de chlore ou de brome, un reste méthyle, amino, (alkyle en $C_1$ à $C_3$)-amino ou di-(alkyle en $C_1$ à $C_3$)-amino, ou bien
où un cycle phényle qui est éventuellement substitué par un ou par deux restes alcoxy en $C_1$ à $C_3$, peut être relié par condensation et par l'intermédiaire de deux atomes de carbone vicinaux, à un des cycles cycloalkylène-imino non substitués susmentionnés,
leurs isomères et leurs sels.

**5.** Indolinones substituées de formule générale I selon au moins une des revendications 1 à 4, dans lesquelles le reste $R_2$ est situé dans la position 5.

**6.** Les indolinones substituées de formule générale I, selon la revendication 1, suivantes:

(a) la 3-Z-[1-(4-aminométhyl-phénylamino)-1-phényl-méthylène]-5-amido-2-indolinone,

(b) la 3-Z-[1-phénylamino)-1-phényl-méthylène]-5-amido-2-indolinone,

(c) la 3-Z-[1-(4-brome-phénylamino)-1-phényl-méthylène]-5-amido-2-indolinone,

(d) la 3-Z-[1-(4-diméthylamino-méthyl)-phénylamino)-1-phényl-méthylène]-5-amido-2-indolinone,

(e) la 3-Z-[1-(4-pyrrolidinométhyl-phénylamino)-1-phényl-méthylène]-5-amido-2-indolinone,

(f) la 3-Z-[1-(4-pipéridinométhyl-phénylamino)-1-phényl-méthylène]-5-amido-2-indolinone,

(g) la 3-Z-[1-(4-hexaméthylène-iminométhyl-phénylamino)-1-phénylméthylène]-5-amido-2-indolinone,

(h) la 3-Z-[1-(4-(4-benzyl-pipéridino)-méthyl)-phénylamino)-1-phénylméthylène]-5-amido-2-indolinone,

(i) la 3-Z-[1-(4-(N-butyl-aminométhyl)-phénylamino)-1-phényl-méthylène]-5-amido-2-indolinone,

(j) la 3-Z-[1-(4-(N-(phényl-méthyl)-aminométhyl)-phénylamino)-1-phénylméthylène]-5-amido-2-indolinone,

(k) la 3-Z-[1-(4-(N-méthyl-N-benzyl-amino-méthyl)-phénylamino)-1-phénylméthylène]-5-amido-2-indolinone,

(l) la 3-Z-[1-(4-pipéridino-méthyl-phénylamino)-1-phényl-miéthylène]-5-diméthylcarbamoyl-2-indolinone,

(m) la 3-Z-[1-(4-pipéridino-méthyl-phénylamino)-1-phényl-méthylène]-5-diéthylcarbamoyl-2-indolinone,

(n) la 3-Z-[1-(4-(3-diéthylamino-propoxy)-phénylamino)-1-phényl-méthylène]-5-amido-2-indolinone,

et leurs sels.

**7.** Les sels dérivés des composés selon les revendications 1 à 6 qui sont physiologiquement acceptables.

**8.** Médicaments contenant, outre éventuellement un ou plusieurs supports inertes et/ou des diluants, un composé selon au moins une des revendications 1 à 6 ou un sel selon la revendication 7.

**9.** Utilisation d'un composé selon au moins une des revendications 1 à 6 ou d'un sel selon la revendication 7, pour la préparation d'un médicament approprié au traitement des proliférations cellulaires, excessives ou anormales.

**10.** Procédé de préparation d'un médicament selon la revendication 8, **caractérisé en ce que** l'on incorpore, par voie non chimique, dans un ou plusieurs supports inertes et/ou diluants, un composé selon au moins une des revendications 1 à 6 ou un sel selon la revendication 7.

**11.** Procédé de préparation des composés selon les revendications 1 à 7, **caractérisé en ce que**

a. l'on fait réagir un composé de formule générale

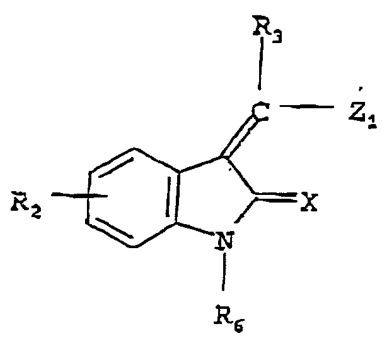

(II)

dans laquelle
X, $R_2$ et $R_3$ ont la signification mentionnée dans les revendications 1 à 6,
$R_6$ représente un atome d'hydrogène, un reste protecteur pour l'atome d'azote appartenant au reste lactame, ou bien une liaison à une phase solide, et
$Z_1$ représente un atome d'halogène, un reste hydroxy, alcoxy ou aralcoxy,
avec une amine de formule générale

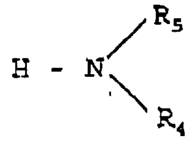

(III)

dans laquelle
$R_4$ et $R_5$ ont la signification mentionnée dans les revendications 1 à 6,
et puis, si nécessaire, on élimine un reste protecteur mis en oeuvre pour l'atome d'azote appartenant au reste lactame ou on détache le composé ainsi obtenu de la phase solide,

ou

b. afin de préparer un composé de formule générale I qui comprend un reste aminométhyle et dans laquelle X représente un atome d'oxygène, on réduit un composé de formule générale

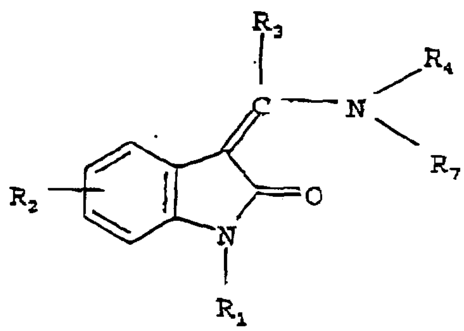

(IV)

dans laquelle
$R_1$ à $R_4$ ont la signification mentionnée dans les revendications 1 à 6, et
$R_7$ a les significations de $R_5$, telles que mentionnées dans les revendications 1 à 6, $R_5$ contenant un reste cyano, et
on transforme, au moyen d'une hydrolyse, le composé de formule générale I que l'on a ainsi obtenu et qui comprend un reste alcoxycarbonyle, en un composé carboxyle correspondant, ou
on transforme, au moyen d'une alkylation on d'une alkylation réductive, du composé de formule générale I que l'on a ainsi obtenu et qui comprend un reste amino ou alkylamino, en un composé alkylamino ou dialkylamino correspondant, ou
on transforme, au moyen d'une acylation, du composé de formule générale I que l'on a ainsi obtenu et qui comprend un reste amino ou alkylamino, en un composé acyle correspondant, ou
on transforme, au moyen d'une estérification ou d'une amidation, du composé de formule générale I que l'on a ainsi obtenu et qui comprend un reste carboxyle, en un composé ester ou aminocarbonyle correspondant, ou
on procède, si nécessaire, à une élimination d'un reste protecteur que l'on a utilisé, pendant les réactions, pour la protection des restes réactifs, ou'
on réalise ensuite éventuellement une séparation du composé de formule générale I que l'on a ainsi obtenu, en ses stéréoisomères, ou
on transforme, à l'aide d'une base ou d'un acide organique ou minéral, le composé de formule générale I que l'on a ainsi obtenu, en ses sels, en particulier et en vue de son utilisation pharmaceutique, en ses sels physiologiquement acceptables.